# EUROPÄISCHE PATENTSCHRIFT

(11) **EP 1 730 131 B1**
(45) Veröffentlichungstag und Bekanntmachung des Hinweises auf die Patenterteilung: **09.05.2012**
(21) Anmeldenummer: 05715979.0
(22) Anmeldetag: 11.03.2005
(51) Int. Cl.: C07D 309/10, A61K 31/351

(54) **GLUCOPYRANOSYL-SUBSTITUIERTE BENZOL-DERIVATE, DIESE VERBINDUNGEN ENTHALTENDE ARZNEIMITTEL, DEREN VERWENDUNG UND VERFAHREN ZU IHRER HERSTELLUNG**
GLUCOPYRANOSYL-SUBSTITUTED BENZOL DERIVATIVES, DRUGS CONTAINING SAID COMPOUNDS, THE USE THEREOF AND METHOD FOR THE PRODUCTION THEREOF
DERIVES DU BENZOL SUBSTITUES PAR UN GLUCOPYRANOSYLE,, MEDICAMENTS RENFERMANT CES COMPOSES, LEUR UTILISATION ET LEUR PROCEDE DE PRODUCTION

(30) Priorität: 16.03.2004 DE 102004012676; 18.08.2004 DE 102004040168; 16.12.2004 DE 102004061145; 09.02.2005 EP 05002628
(43) Veröffentlichungstag der Anmeldung: 13.12.2006
(60) Teilanmeldung: 10179228.1
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: HIMMELSBACH, Frank, 88441 Mittelbiberach (DE); ECKHARDT, Matthias, 88400 Biberach (DE); EICKELMANN, Peter, 88441 Mittelbiberach (DE); BARSOUMIAN, Edward Leon, Toyonaka, Osaka 560-0005 (JP); THOMAS, Leo, 88400 Biberach (DE)
(74) Vertreter: Hammann, Heinz
(86) Internationale Anmeldenummer: PCT/EP2005/002618
(87) Internationale Veröffentlichungsnummer: WO 2005/092877

(56) Entgegenhaltungen:
- WO-A-02/083066
- US-A1- 2003 114 390

## Beschreibung

Gegenstand der vorliegenden Erfindung sind Glucopyranosyl-substituierte Benzol-Derivate der allgemeinen Formel I 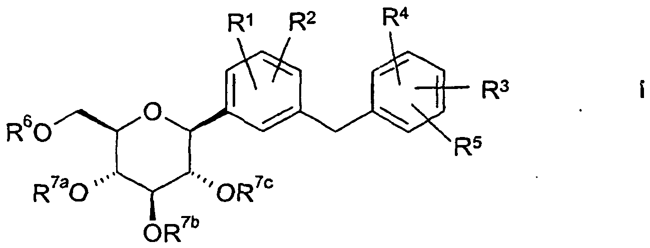 wobei die Reste R¹ bis R⁶ sowie R^{7a}, R^{7b}, R^{7c} nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Stoffwechselerkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf den natriumabhängigen Glucose-Cotransporter SGLT2 besitzen, zur Behandlung von Krankheiten, insbesondere von Diabetes vorgeschlagen.

Aus den internationalen Offenlegungsschriften WO 98/31697, WO 01/27128, WO 02/083066, WO 03/099836, WO 2004/063209, WO 2004/080990, WO 2004/013118, WO 2004/052902, WO 2004/052903 und der US-Anmeldung US 2003/0114390 sind Glucopyranosyl-substituierte Aromaten sowie deren Herstellung und deren mögliche Aktivität als SGLT2-Inhibitoren bekannt.

### Aufgabe der Erfindung

Der vorliegenden Erfindung liegt die Aufgabe zugrunde, neue Pyranosyl-substituierte Benzol-Derivate aufzuzeigen, insbesondere solche, die eine Aktivität bezüglich des natriumabhängigen Glucose-Cotransporters SGLT, insbesondere SGLT2 besitzen. Eine weitere Aufgabe der vorliegenden Erfindung besteht im Aufzeigen von Pyranosylsubstituierten Benzol-Derivate, die *in vitro* und/oder *in vivo* im Vergleich mit bekannten, strukturähnlichen Verbindungen eine erhöhte Hemmwirkung bezüglich des natriumabhängigen Glucose-Cotransporters SGLT2 besitzen und/oder verbesserte pharmakologische oder pharmakokinetische Eigenschaften aufweisen.

Ferner ist es eine Aufgabe der vorliegenden Erfindung, neue Arzneimittel bereit zu stellen, welche zur Prophylaxe und/oder Behandlung von Stoffwechselerkrankungen, insbesondere von Diabetes geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind Glucopyranosyl-substituierte Benzol-Derivate der allgemeinen Formel I 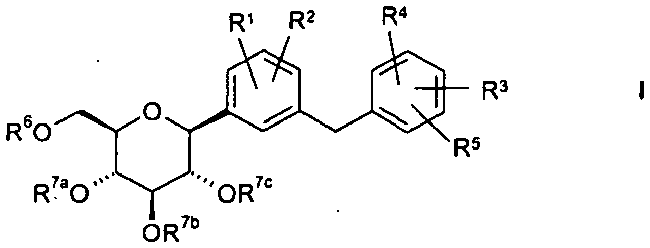 in der
- R¹: ausgewählt ist aus den Bedeutungen Wasserstoff, Fluor, Chlor, Brom, lod, C₁₋₄-Alkyl, C₂₋₈-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₈-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cycloalkenyloxy, Hydroxy, Amino, Nitro oder Cyan, wobei in den C₅₋₆-Cycloalkylgruppen eine Methylengruppe durch O ersetzt sein kann
- R²: Wasserstoff, Fluor, Chlor, Brom, Hydroxy, C₁₋₄-Alkyl, C₁₋₄-Alkoxy, Cyan oder Nitro, wobei die Alkyl- oder Alkoxygruppe ein oder mehrfach mit Fluor substituiert sein kann, und
- R³: ausgewählt ist aus den Bedeutungen Tri-(C₁₋₄-alkyl)silyl-C₁₋₁₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsuffinyl und C₅₋₇-Cycloalkenylsulfonyl,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N}, vorzugsweise O, CO, S, SO₂ oder NR^{N}, ganz besonders bevorzugt durch O oder CO ersetzt sein können,
- R⁴, R⁵: unabhängig voneinander Wasserstoff, Fluor, Chlor, Brom, lod, Cyan, Nitro, C₁₋₃-Alkyl, C₁₋₃-Alkoxy, durch 1 bis 3 Fluoratome substituiertes Methyl- oder Methoxy,
- R^{N}: H, C₁₋₄-Alkyl, C₁₋₄-Alkylcarbonyl oder C₁₋₄-Alkylsulfonyl,
- L1: unabhängig voneinander ausgewählt aus der Gruppe bestehend aus Hydroxy, Cyan-, Nitro-, C₃₋₇-Cycloalkyl, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁₋₃-alkyl)-aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkyl-sulfinyl-, Arylsulfinyl-, Heteroarylsulfinyl-, C₁₋₄-Arylsulfonyl- und Heteroarylsulfonyl-; und
- L2: unabhängig voneinander ausgewählt aus der Gruppe bestehend aus Fluor, Chlor, Brom, lod, C₁₋₃-Alkyl, Difluormethyl, Trifluormethyl, C₁₋₃-Alkoxy, Difluormethoxy, Trifluormethoxy und Cyan; und
- R⁶: Wasserstoff, (C₁₋₆-Alkyl)oxycarbonyl oder C₁₋₆-Alkylcarbonyl bedeutet,
- R^{7a}, R^{7b}, R^{7c}: Wasserstoff bedeuten,
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinyl- oder Tetrazolylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L2 substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Die erfindungsgemäßen Verbindungen der allgemeinen Formel I und ihre physiologisch verträglichen Salze weisen wertvolle pharmakologische Eigenschaften auf, insbesondere eine Hemmwirkung auf den natriumabhängigen Glucose-Cotransporter SGLT, insbesondere SGLT2. Ferner können erfindunsgemäße Verbindungen eine Hemmwirkung auf den natriumabhängigen Glucose-Cotransporter SGLT1 aufweisen. Verglichen mit einer möglichen Hemmwirkung auf SGLT1 hemmen die erfindungsgemäßen Verbindungen vorzugsweise selektiv SGLT2.

Gegenstand der vorliegenden Erfindung sind auch die physiologisch verträglichen Salze der erfindungsgemäßen Verbindungen mit anorganischen oder organischen Säuren.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ein weiterer Gegenstand dieser Erfindung ist die Verwendung mindestens einer erfindungsgemäßen Verbindung oder eines seiner physiologisch verträglichen Salze zur Herstellung eines Arzneimittels, das zur Behandlung von Stoffwechselerkrankungen ausgewählt aus der Gruppe bestehend aus Diabetes mellitus Typ 1 und Typ 2, diabetische Komplikationen, metabolische Azidose oder Ketose, reaktiver Hypoglykämie, Hyperinsulinämie, Glukosestoffwechselstörung, Insulinresistenz, Metabotischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet ist

Ein weiterer Gegenstand dieser Erfindung ist die Verwendung mindestens einer erfindungsgemäßen Verbindung oder eines seiner physiologisch verträglichen Salze zur Herstellung eines Arzneimittels zur Inhibition des natriumabhängigen Glucose-Cotransporters SGLT, insbesondere SGLT2.

Ein weiterer Gegenstand dieser Erfindung ist die Verwendung mindestens einer erfindungsgemäßen Verbindung oder eines seiner physiologisch verträglichen Salze zur Herstellung eines Arzneimittels zum Verhindern der Degeneration von pankreatischen beta-Zellen und/oder zum Verbessern und/oder Wiederherstellen der Funktionalität von pankreatischen beta-Zellen.

Ein weiterer Gegenstand dieser Erfindung ist die Verwendung mindestens einer erfindungsgemäßen Verbindung oder eines seiner physiologisch verträglichen Salze zur Herstellung von Diuretika und/oder Antihypertensiva.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung oder eines seiner physiologisch verträglichen Salze in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, dadurch gekennzeichnet, dass
a) zur Herstellung von Verbindungen der allgemeinen Formel I, die wie vor- und nachstehend definiert ist,
   eine Verbindung der allgemeinen Formel II 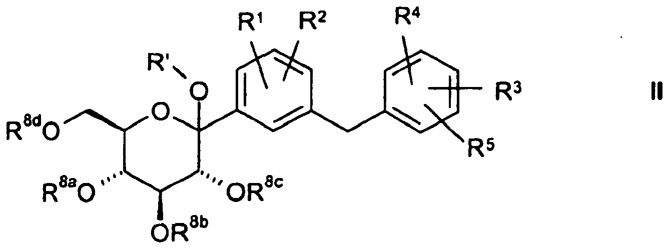 in der
   - R': H, C₁₋₄-Alkyl, (C₁₋₁₈-Alkyl)carbonyl, (C₁₋₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
   - R^{8a}, R^{8b}, R^{8c}, R^{8d}: unabhängig voneinander ausgewählt sind aus der Gruppe Wasserstoff, (C₁₋₈-Alkyl)carbonyl, (C₁₋₁₈-Alkyl)oxycarbonyl, Arylcarbonyl und Aryl-(C₁-₃-alkyl)-carbonyl, oder eine Benzyl-Gruppe oder eine R^{a}R^{b}R^{c}Si-Gruppe oder eine Ketal-oder Acetalgruppe, insbesondere eine Alkyliden- oder Arylalkyliden- Ketal- oder Acetalgruppe bedeuten, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring, insbesondere einen 2,3-Dimethyl-2,3-di(C₁₋₃-alkoxy)-1,4-dioxan-Ring, bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
   - R^{a}, R^{b}, R^{c}: unabhängig voneinander C₁₋₄-Alkyl, Aryl oder Aryl-C₁₋₃-alkyl, worin die Aryl- oder Alkylgruppen ein- oder mehrfach mit Halogen substituiert sein können;
   wobei unter den bei der Definition der vorstehend genannten Reste erwähnten Arylgruppen Phenyl- oder Naphthylgruppen, vorzugsweise Phenylgruppen zu verstehen sind;
   und in der die Reste R¹ bis R⁵ wie vor- und nachstehend definiert sind;
   mit einem Reduktionsmittel in Gegenwart einer Lewis- oder Brønsted-Säure umgesetzt wird, wobei die eventuell vorhandenen Schutzgruppen gleichzeitig oder nachträglich abgespalten werden; oder
b) zur Herstellung von Verbindungen der allgemeinen Formel I, in der R⁶ Wasserstoff bedeutet,
   eine Verbindung der allgemeinen Formel III 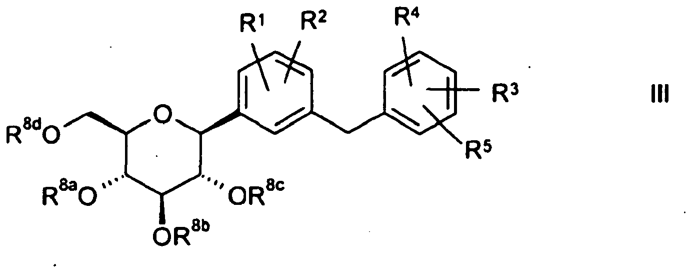
in der R^{8a}, R^{8b}, R^{8c}, R^{8d} sowie R¹ bis R⁵ wie zuvor und nachstehend definiert sind, jedoch mindestens einer der Reste R^{8a}, R^{8b}, R^{8c}, R^{8d} nicht Wasserstoff bedeutet, hydrolysiert wird, und
gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre Salze, insbesondere für die pharmazeutische Anwendung in ihre physiologisch verträglichen Salze, überführt wird.

Ein weiterer Gegenstand dieser Erfindung ist ein Verfahren zur Herstellung von Verbindungen der allgemeinen Formel II 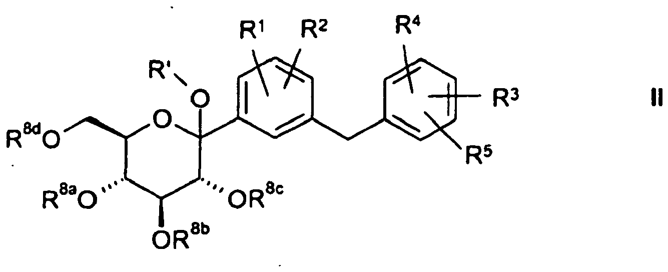 in der
- R': H, C₁₋₄-Alkyl, (C₁₋₁₈-Alkyl)carbonyl, (C₁₋₁₈-Alkyl)oxycarbonyl, Arylcarbonyl und Aryl-(C₁₋₃-alkyl)-carbonyl bedeutet, worin die Alkyl- oder Arylgruppen ein- oder mehrfach mit Halogen substituiert sein können;
- R^{8a} R^{8b}, R^{8c}, R^{8d}: unabhängig voneinander ausgewählt sind aus der Gruppe Wasserstoff, (C₁₋₁₈-Alkyl)carbonyl, (C₁₋₁₈-Alkyl)oxycarbonyl, Arylcarbonyl und Aryl-(C₁₋₃-alkyl)-carbonyl, oder eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder mit zwei Sauerstoffatomen des Pyranose-Rings einen substituierten 2,3-Oxydioxan-Ring, insbesondere einen 2,3-Dimethyl-2,3-di(C₁₋₃-alkoxy)-1,4-dioxan-Ring, bilden können, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)aminoGruppe substituiert sein können; und
- R^{a}, R^{b}, R^{c}: unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵, R⁶ die zuvor und nachstehend angegebenen Bedeutungen besitzen,
bei dem eine Organometall-Verbindung (V), die durch Halogen-Metall-Austausch oder durch Insertion eines Metalls in die Kohlenstoff-Halogen-Bindung einer Halogen-Benzylbenzol-Verbindung der allgemeinen Formel IV 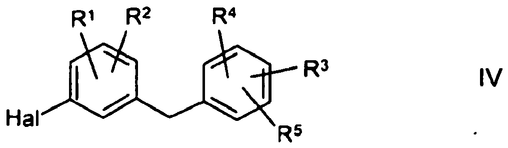 in der Hal Cl, Br und I bedeuten und R¹ bis R⁵ wie zuvor und nachstehend definiert sind, und gegebenenfalls anschließender Ummetallierung erhältlich ist, an ein Gluconolacton der allgemeinen Formel VI 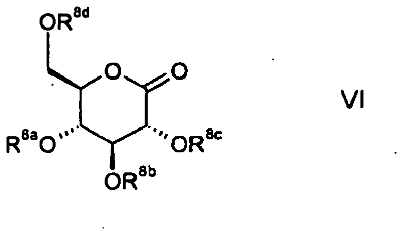 in der R^{8a}, R^{8b}, R^{8c}, R^{8d} wie zuvor und nachstehend definiert sind,
addiert wird, und
anschließend das erhaltene Addukt, vorzugsweise in situ, mit Wasser oder einem Alkohol R'-OH, wobei R¹ gegebenenfalls substituiertes C₁₋₄-Alkyl bedeutet, in Gegenwart einer Säure, wie beispielsweise Methansulfonsäure, Schwefelsäure, Salzsäure, Essigsäure oder Ammoniumchlorid, umgesetzt wird und optional das in der Umsetzung mit Wasser erhaltene Produkt, in dem R' H bedeutet, in einer nachfolgenden Reaktion mit einem Acylierungsmittel, wie beispielsweise des entsprechenden Säurechlorids oder -anhydrids, in das Produkt der Formel II überführt wird, worin R' (C₁₋₁₈-Alkyl)carbonyl, (C₁₋₁₈-Alkyl)oxycarbonyl, Arylcarbonyl oder Aryl-(C₁₋₃-alkyl)-carbonyl, das wie angegeben substituiert sein kann, bedeutet.

Die aufgezeigten Zwischenprodukte, insbesondere der Formel IV und der Formel II, stellen ebenfalls Gegenstände dieser Erfindung dar.

Ein weiterer Gegenstand dieser Erfindung sind Glucopyranosyl-substituierte Benzol-Derivate der allgemeinen Formel I 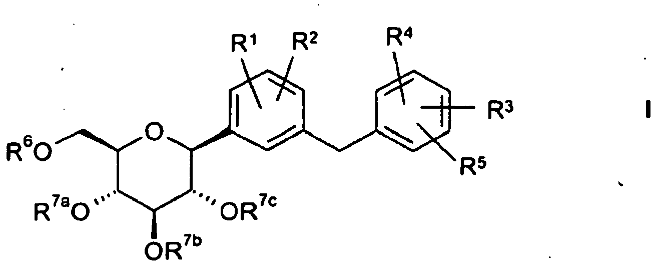 in der R¹ bis R⁵ wie zuvor und nachfolgend definiert definiert sind und
- R⁶, R^{7a}, R^{7b}, R^{7c}: unabhängig voneinander eine Bedeutung ausgewählt aus der Gruppe (C₁₋₈-Alkyl)carbonyl, (C₁₋₈-Alkyl)oxycarbonyl, Arylcarbonyl und Aryl-(C₁₋₃-alkyl)-carbonyl besitzen,
- L2: unabhängig voneinander ausgewählt sind aus der Gruppe bestehend aus Fluor, Chlor, Brom, lod, C₁₋₃-Alkyl, Difluormethyl, Trifluormethyl, C₁₋₃-Alkoxy, Difluormethoxy, Trifluormethoxy und Cyan; und
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

Ein weiterer Gegenstand dieser Erfindung ist ein Arzneimittel, das eine erfindungsgemäße Verbindung oder ein physiologisch verträgliches Salz solch einer Verbindung sowie mindestens einen Wirkstoff ausgewählt aus der Gruppe der Antidiabetika neben gegebenenfalls einem oder mehreren inerten Trägerstoffen und/oder Verdünnungsmitteln aufweist.

Ein weiterer Gegenstand dieser Erfindung ist ein Arzneimittel, das eine erfindungsgemäße Verbindung oder ein physiologisch verträgliches Salz solch einer Verbindung sowie mindestens einen Wirkstoff ausgewählt aus der Gruppe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer, Fibrate, Nikotinsäure und deren Derivate, PPAR-alpha Agonisten, PPAR-delta Agonisten, ACAT Inhibitoren oder Cholesterolresorptionsinhibitoren, gallensäurebindende Substanzen, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder ß3-Agonisten und Agonisten des 5HT2c Rezeptors neben gegebenenfalls einem oder mehreren inerten Trägerstoffen und/oder Verdünnungsmitteln aufweist.

Ein weiterer Gegenstand dieser Erfindung ist ein Arzneimittel, das eine erfindungsgemäße Verbindung oder ein physiologisch verträgliches Salz solch einer Verbindung sowie mindestens einen Wirkstoff ausgewählt aus der Gruppe der Medikamente zur Beeinflussung des Bluthochdrucks, des chronischen Herzversagens oder der Atherosklerose wie z.B. A-II Antagonisten oder ACE Inhibitoren, ECE-Inhibitoren, Diuretika, ß-Blocker, Ca-Antagonisten, zentral wirksamen Antihypertensiva, Antagonisten des alpha-2-adrenergen Rezeptors, Inhibitoren der neutralen Endopeptidase, und Thrombozytenaggregationshemmer oder Kombinationen daraus neben gegebenenfalls einem oder mehreren inerten Trägerstoffen und/oder Verdünnungsmitteln aufweist.

Ein weiterer Gegenstand dieser Erfindung ist ein Arzneimittel, das eine erfindungsgemäße Verbindung oder ein physiologisch verträgliches Salz solch einer Verbindung sowie mindestens einen Wirkstoff ausgewählt aus der Gruppe Harnsäuresynthese Inhibitoren und Urikosurika neben gegebenenfalls einem oder mehreren inerten Trägerstoffen und/oder Verdünnungsmitteln aufweist.

Ein weiterer Gegenstand dieser Erfindung ist ein Arzneimittel, das eine erfindungsgemäße Verbindung oder ein physiologisch verträgliches Salz solch einer Verbindung sowie mindestens einen Wirkstoff ausgewählt aus der Gruppe GABA-Rezeptor-Antagonisten, Na-Kanal-Blockern, Topiramat, Protein-Kinase C Inhibitoren, advanced glycation endproduct Inhibitoren und Aldose Reduktase Inhibitoren neben gegebenenfalls einem oder mehreren inerten Trägerstoffen und/oder Verdünnungsmitteln aufweist.

### Detailierte Beschreibung der Erfindung

Sofern nicht anders angegeben besitzen die Gruppen, Reste und Substituenten, insbesondere R¹ bis R⁵, L1, L2, R^{N}, R⁶, R^{7a}, R^{7b}, R^{7c}, R^{8a}, R^{8b}, R^{8c}, R^{8d} die zuvor und nachfolgend angegebenen Bedeutungen.

Kommen Reste, Substituenten oder Gruppen in einer Verbindung mehrfach vor, so können diese eine gleiche oder verschiedene Bedeutungen aufweisen.

Nachfolgend werden bevorzugte Bedeutungen einzelner Gruppen und Substituenten der erfindungsgemäßen Verbindungen angegeben.

Der Rest R³ steht vorzugsweise in meta- oder para Position zur -CH₂-Brücke, so dass Verbindungen gemäß der folgenden Formeln I.1 und 1.2, insbesondere der Formel 1.2, bevorzugt sind:

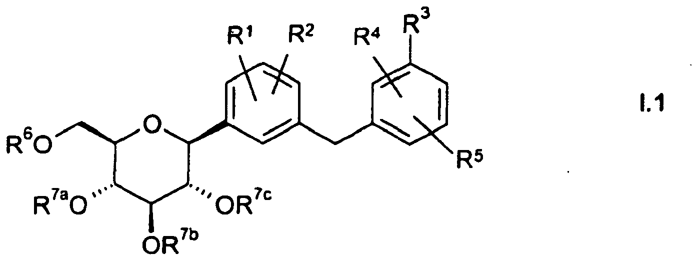

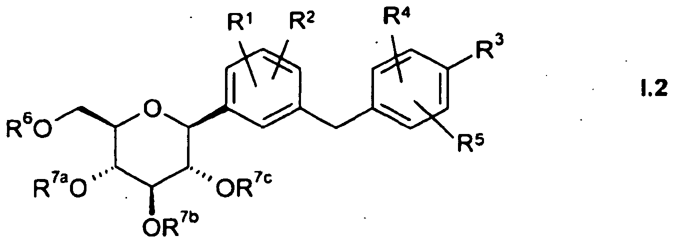

Die in den Gruppen L1 vorkommende Bezeichnung Aryl bedeutet vorzugsweise Phenyl.

Die in den Gruppen L1 vorkommende Bezeichnung Heteroaryl bedeutet vorzugsweise Pyridinyl, Pyrimidinyl, Pyridazinyl, Pyrazinyl, Triazinyl, Imidazolyl, Pyrazolyl, Triazolyl, Tetrazolyl, Oxazolyl, Oxadiazolyl, Thiazolyl oder Thiadiazolyl.

Besonders bevorzugt bedeutet die Gruppe R³ Tri-(C₁₋₄-alkyl)silyl-C₁₋₆-alkyl, C₂₋₆-Alkin-1-yl, C₂₋₆ -Alken-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl, C₅₋₇-Cycloalkenylsulfonyl,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N}, vorzugsweise O, CO, S, SO₂ oder NR^{N}, ganz besonders bevorzugt durch O oder CO, ersetzt sein können.

Ganz besonders bevorzugt bedeutet die Gruppe R³ Tri-(C₁₋₄-alkyl)silyl-C₁₋₆-alkyl, C₂₋₆-Alkin-1-yl, C₂₋₆-Alken-1-yl, C₃₋₇-Cycloalkyloxy, C₅₋₇-Cycloalkenyloxy, C₃₋₇-Cycloalkylsulfanyl, C₅₋₇-Cycloalkenylsulfanyl, wobei die vorstehend genannten Alkinyl- und Alkenylgruppen ein- oder mehrfach mit Fluor oder einfach mit Chlor oder dem Rest L1 substituiert sein können, und wobei in den Cycloalkyl- und Cycloalkenylgruppen ein oder zwei Methylengruppen unabhängig voneinander durch O, CO, S, SO₂ oder NR^{N}, insbesondere O oder CO, ersetzt sein können.

Beispiele ganz besonders bevorzugter Bedeutungen der Gruppe R³ sind Trimethylsilylethyl, Ethinyl, 1-Propin-1-yl, 1-Butin-1-yl, tert.-Butylethinyl, 2-Hydroxyprop-2-ylethinyl, 2-Methoxy-prop-2-ylethinyl, 3-Hydroxy-1-propin-1-yl, 3-Methoxy-1-propin-1-yl, Ethenyl, 1-Propenyl, 1-Butenyl, tert.-Butylethenyl, Cyclopropyloxy, Cyclobutyloxy, Cyclopentyloxy, Cyclohexyloxy, Tetrahydrofuranyloxy, Tetrahydrothiophenyloxy, 1,1-Dioxotetrahydrothiophenyloxy, Tetrahydropyranyloxy, Tetrahydrothiopyranyloxy, 1,1-Dioxotetrahydrothiopyranyloxy, Tetrahydrofuranonyloxy, Piperidinyloxy, Piperidinonyloxy, Pyrrolidin-3-yloxy, Pyrrolidinon-3-yloxy, Tetrahydrofuranyl-sulfanyl, Cyclopropylsulfanyl, Cyclobutylsulfanyl, Cyclopentylsulfanyl und Cyclohexylsulfanyl, wobei die -NH-Gruppe in einem Piperidinyl-, Piperidinonyl-, Pyrrolidinyl- oder Pyrrolidinonyl-Ring mit R^{N}, insbesondere C₁₋₃-Alkyl oder Acetyl, substituiert sein kann.

Ganz besonders bevorzugte Bedeutungen sind hierbei Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy. Hiervon besonders hervorzuhebende Beispiele sind Ethinyl, Trimethylsilylethyl, Cyclobutyloxy, Cyclopentyloxy, Cyclohexyloxy, Tetrahydrofuran-3-yloxy und Tetrahydropyran-4-yloxy.

Sind in den Resten oder Gruppen R¹ oder R³ Cycloalkyl- oder Cycloalkenyl-Ringe vorhanden, in denen zwei Methylengruppen durch O, S oder NR^{N} ersetzt sind oder durch S, NR^{N}_{,} CO, SO oder SO₂ ersetzt sind, so sind diese Methylengruppen vorzugsweise nicht unmittelbar miteinander verbunden. Sind jedoch zwei Methylengruppen durch O und CO oder durch NR^{N} und CO ersetzt, so können diese unmittelbar miteinander verbunden sein, so dass eine -O-CO- bzw. -NR^{N}-CO-Gruppe gebildet wird.

Bevorzugte Bedeutungen des Rests L1 sind ausgewählt aus der Gruppe bestehend aus Hydroxy-, Cyan-, C₃₋₆-Cycloalkyl, C₁₋₄-Alkylcarbonyl-, Aminocarbonyl-, C₁₋₄-Alkylaminocarbonyl-, Di-(C₁₋₃-alkyl)aminocarbonyl-, Pyrrolidin-1-ylcarbonyl-, Piperidin-1-ylcarbonyl-, Morpholin-4-ylcarbonyl, C₁₋₄-Alkoxycarbonyl-, C₁₋₄-Alkyloxy-, C₁₋₄-Alkylsulfanyl-, C₁₋₄-Alkylsulfinyl-, und C₁₋₄-Alkylsulfonyl-.

Besonders bevorzugte Bedeutungen des Rests L1 sind ausgewählt aus der Gruppe bestehend aus Hydroxy-, C₁₋₄-Alkyloxy- und C₁₋₄-Alkylsulfanyl-.

Bedeutet L1 Hydroxy, so ist die Hydroxygruppe nicht unmittelbar an ein C-Atom einer Doppel- oder Dreifachbindung geknüpft.

Bevorzugte Bedeutungen des Rests R¹ sind Wasserstoff, Fluor, Chlor, Brom, lod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₈-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cycloalkenyloxy, Hydroxy, Amino, Nitro oder Cyan, wobei in den C₅₋₆-Cycloalkylgruppen eine Methylengruppe durch O ersetzt sein kann.

Besonders bevorzugte Bedeutungen sind hierbei Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy, Cyclohexyloxy, insbesondere Methyl und Chlor.

Nachfolgend werden Bedeutungen weiterer Reste und Substituenten angegeben, die gemäß der allgemeinen Formel I, der Formeln I.1 und I.2 als auch gemäß der zuvor beschriebenen Ausführungsformen als bevorzugt anzusehen sind:

Bevorzugte Bedeutungen des Rests R² sind Wasserstoff, Fluor, Chlor, Brom, Methyl, Hydroxy, Methoxy, Ethoxy, Trifluormethoxy, Cyan, Nitro und durch 1 bis 3 Fluoratome substituiertes Methyl.

Besonders bevorzugte Bedeutungen des Rests R² sind Wasserstoff, Fluor, Hydroxy, Methoxy, Ethoxy und Methyl, insbesondere Wasserstoff und Methyl.

Bevorzugte Bedeutungen des Rests R⁴ sind Wasserstoff und Fluor, insbesondere Wasserstoff.

Bevorzugte Bedeutungen des Rests R⁵ sind Wasserstoff und Fluor, insbesondere Wasserstoff.

Der Rest R^{N} bedeutet vorzugsweise H, Methyl, Ethyl oder Acetyl.

Der Rest R⁶ bedeutet erfindungsgemäß besonders bevorzugt Wasserstoff, Methylcarbonyl, Methoxycarbonyl oder Ethoxycarbonyl, ganz besonders bevorzugt Wasserstoff oder Methoxycarbonyl.

Die Verbindungen der Formel I, in denen R⁶, R^{7a}, R^{7b} und R^{7c} eine erfindungsgemäße, von Wasserstoff verschiedene Bedeutung aufweisen, beispielsweise C₁₋₈-Alkylcarbonyl, eignen sich bevorzugt als Zwischenprodukte bei der Synthese von Verbindungen der Formel I in denen R^{7a}, R^{7b} und R^{7c} Wasserstoff bedeuten.

Besonders bevorzugte Verbindungen der allgemeinen Formel I sind ausgewählt aus der Gruppe der Formeln I.2a bis 1.2d, insbesondere 1.2c: 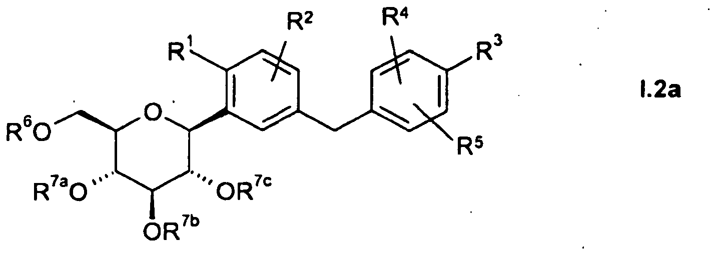 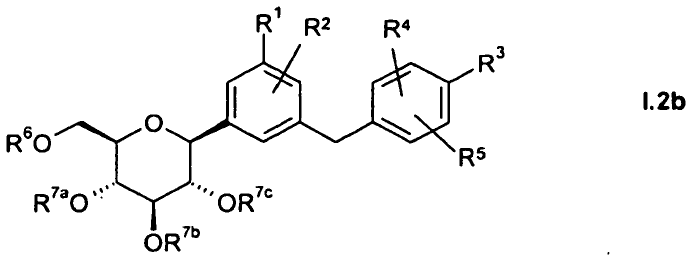 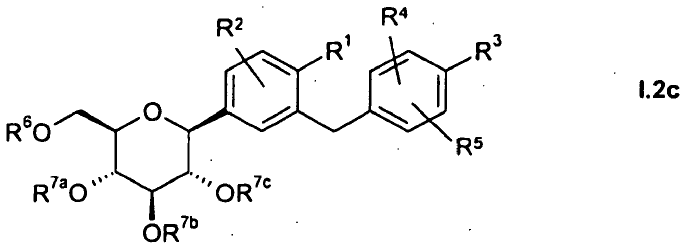 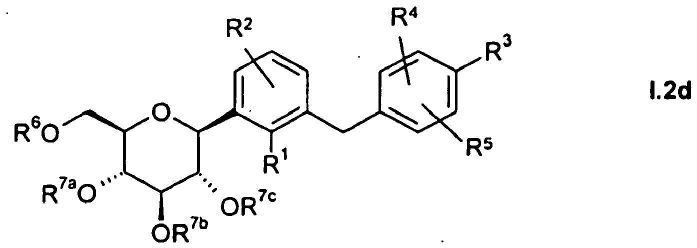 wobei die Reste R' bis R⁶ und R^{7a} , R^{7b}, R^{7c} eine der zuvor angegebenen, insbesondere eine der als bevorzugt angegebenen Bedeutungen aufweisen; insbesondere wobei
- R¹: Wasserstoff, Fluor, Chlor, Brom, lod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₆-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cyclo-alkenyloxy, Hydroxy, Amino, Nitro oder Cyan bedeutet, wobei in den C₅₋₆-Cycloalkylgruppen eine Methylengruppe durch O ersetzt sein kann; besonders bevorzugt Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy oder Cyclohexyloxy bedeutet; und
- R²: Wasserstoff, Fluor, Hydroxy, Methoxy, Ethoxy oder Methyl, insbesondere Wasserstoff oder Methyl bedeutet; und
- R³: ausgewählt ist aus der Gruppe bestehend aus Trimethylsilylethyl, Ethinyl, 1-Propin-1-yl, 1-Butin-1-yl, tert.-Butylethinyl, 2-Hydroxyprop-2-ylethinyl, 2-Methoxyprop-2-ylethinyl, 3-Hydroxy-1-propin-1-yl, 3-Methoxy-1-propin-1-yl, Ethenyl, 1-Propenyl, 1-Butenyl, tert.-Butylethenyl, Cyclopropyloxy, Cyclobutyloxy, Cyclopentyloxy, Cyclohexyloxy, Tetrahydrofuranyloxy, Tetrahydrothiophenyloxy, 1,1-Dioxotetrahydrothiophenyloxy, Tetrahydropyranyloxy, Tetrahydrothiopyranyloxy, 1,1-Dioxotetrahydrothiopyranyloxy, Tetrahydrofuranonyloxy, Piperidinyloxy, Piperidinonyloxy, Pyrrolidin-3-yloxy, Pyrrolidinon-3-yloxy, Tetrahydrofuranyl-sulfanyl, Cyclopropylsulfanyl, Cyclobutylsulfanyl, Cyclopentylsulfanyl und Cyclohexylsulfanyl, wobei die -NH-Gruppe in einem Piperidinyl-, Piperidinonyl-, Pyrrolidinyl- oder Pyrrolidinonyl-Ring mit R^{N}, insbesondere C₁₋₃-Alkyl oder Acetyl, substituiert sein kann; besonders bevorzugt ausgewählt aus Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy; und
- R⁴: Wasserstoff oder Fluor, insbesondere Wasserstoff bedeutet; und
- R⁵: Wasserstoff oder Fluor, insbesondere Wasserstoff bedeutet; und
- R⁶: Wasserstoff, (C₁₋₆-Alkyl)oxycarbonyl, oder (C₁₋₆-Alkyl)carbonyl, insbesondere Wasserstoff, Methylcarbonyl, Methoxycarbonyl oder Ethoxycarbonyl, ganz besonders bevorzugt Wasserstoff bedeutet; und
- R^{7a}, R^{7b}, R^{7c}: Wasserstoff;
einschließlich deren Tautomere, deren Stereoisomere, deren Gemische und deren Salze.

Gemäß einer Variante der zuvor angeführten Ausführungsformen sind diejenigen Verbindungen auch bevorzugt, in denen die Phenylgruppe, die den Substituenten R³ trägt, mindestens einen weiteren, von Wasserstoff verschiedenen Substituenten R⁴ und/oder R⁵ aufweist. Nach dieser Variante sind diejenigen Verbindungen besonders bevorzugt, die einen Substituenten R⁴ in der Bedeutung Fluor aufweisen.

Der Phenylrest, der den Substituenten R³ trägt, ist vorzugsweise maximal einfach fluoriert.

Die im nachfolgenden experimentellen Teil angegebenen Verbindungen der allgemeinen Formel I, sowie deren Derivate, in denen R⁶ eine erfindungsgemäße, von Wasserstoff verschiedene Bedeutung aufweist, insbesondere R⁶ Ethoxycarbonyl oder Methoxycarbonyl bedeutet, einschließlich deren Tautomere, deren Stereoisomere und deren Gemische, sind erfindungsgemäß bevorzugt.

Besonders bevorzugte Verbindungen der allgemeinen Formel I sind ausgewählt aus der Gruppe :
(1) 1-Chlor-2-(4-cyclopentyloxybenzyl)-4-(ß-D-glucopyranos-1-yl)-benzol
(2) 1-Chlor-4-(ß-D-glucopyranos-1-yl)-2-[4-(*(R)*-tetrahydrofuran-3-yloxy)-benzyl]-benzol
(3) 1-Chlor-4-(ß-D-glucopyranos-1-yl)-2-[4-(*(S)*-tetrahydrofuran-3-yloxy)-benzyl]-benzol
(4) 1-Chlor-4-(ß-D-glucopyranos-1-yl)-2-[4-(tetrahydrofuran-2-on-3-yloxy)-benzyl]-benzol
(5) 1-Chlor-4-(ß-D-glucopyranos-1-yl)-2-(4-cyclobutyloxy-benzyl)-benzol
(6) 1-Chlor-4-(ß-D-glucopyranos-1-yl)-2-(4-cyclohexyloxy-benzyl)-benzol
(7) 1-Chlor-4-(ß-D-glucopyranos-1-yl)-2-[4-(tetrahydropyran-4-yloxy)-benzyl]-benzol
(8) 1-Chlor-4-(ß-O-glucopyranos-1-yl)-2-[4-(1-acetyl-piperidin-4-yloxy)-benzyl]-benzol
(10) 1-(ß-D-Glucopyranos-1-yl)-4-methyl-3-[4-(tetrahydrofuran-3-yloxy)-benzyl]-benzol
(11) 1-(ß-D-Glucopyranos-1-yl)-4-methyl-3-[4-(2-trimethylsilyl-ethyl)-benzyl]-benzol
(12) 1-Chlor-4-(ß-D-glucopyranos-1-yl)-2-(4-ethinyl-benzyl)-benzol
(13) 1-Chlor-4-(ß-D-glucopyranos-1-yl)-2-[4-(piperidin-4-yloxy)-benzyl]-benzol
(14) 1-Fluor-4-(ß-D-glucopyranos-1-yl)-2-(4-ethinyl-benzyl)-benzol
(15) 1-(ß-D-Glucopyranos-1-yl)-3-(4-ethinyl-benzyl)-benzol
(17) 1-Methoxy-4-(ß-D-glucopyranos-1-yl)-2-(4-ethinyl-benzyl)-benzol
sowie deren Derivate, in denen R⁶ eine erfindungsgemäße, von Wasserstoff verschiedene Bedeutung aufweist, insbesondere R⁶ Ethoxycarbonyl oder Methoxycarbonyl bedeutet,
einschließlich deren Tautomere, deren Stereoisomere und deren Gemische.

In den erfindungsgemäßen Verfahren weisen die Reste R¹, R², R³. R⁴ und R⁵ vorzugsweise die zuvor als bevorzugt angegebenen Definitionen auf. Ferner bedeutet hierbei R' vorzugsweise H, C₁₋₃-Alkyl oder Benzyl, insbesondere H, Ethyl oder Methyl. Die Reste R^{8a}, R^{8b}, R^{8c} und R^{8d} bedeuten vorzugsweise unabhängig voneinander H, C₁₋₄-Alkylcarbonyl oder Benzyl, insbesondere H, Methylcarbonyl, Ethylcarbonyl oder Benzyl.

Als Zwischenprodukte oder Ausgangsstoffe in der Synthese der erfindungsgemäßen Verbindungen sind auch Verbindungen der allgemeinen Formel IV, insbesondere der allgemeinen Formel IV' 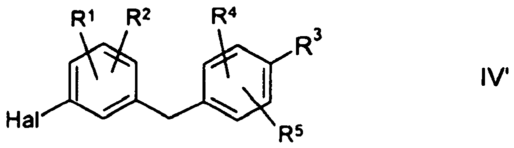 in der Hal Chlor, Brom oder lod bedeutet und die Reste R¹, R², R³, R⁴ und R⁵ wie zuvor definiert sind, Gegenstand dieser Erfindung. Besonders bevorzugt weisen hierbei die Reste R¹, R², R³, R⁴ und R⁵ die im Anschluss an die Formeln I.2a bis I.2d angegebenen Definitionen auf. Ganz besonders bevorzugt sind Verbindungen der allgemeinen Formel IV', in der Hal Chlor, Brom oder lod bedeutet und die Reste R¹, R², R⁴ und R⁵ die im Anschluss an die Formeln I.2a bis I.2d angegebenen Definitionen aufweisen und der Rest R³ Ethinyl oder C₃₋₆-1-Alkin-1-yl bedeutet, wobei die Ethinyl-Gruppe mit dem Rest -SiR₃ substituiert sein kann, wobei die Reste R unabhängig voneinander C₁₋₄-Alkyl, C₁₋₄-Alkoxy oder Aryl bedeuten, und wobei die C₃₋₆-1-Alkin-1-yl-Gruppe mit Hydroxy oder C₁₋₃-Alkoxy, insbesondere Hydroxy oder Methoxy, substituiert sein kann.

Als Zwischenprodukte oder Ausgangsstoffe in der Synthese der erfindungsgemäßen Verbindungen sind auch Verbindungen der allgemeinen Formel II, insbesondere der allgemeinen Formel II' 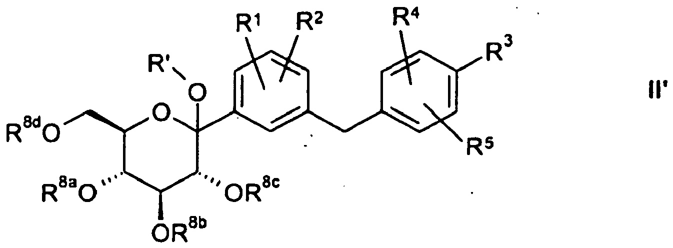 in der R', R^{8a}, R^{8b}, R^{8c}, R^{8d}, R¹, R², R³, R⁴ und R⁵ wie zuvor und nachstehend definiert sind; insbesondere in der R' H, C₁₋₃-Alkyl oder Benzyl, insbesondere H, Ethyl oder Methyl bedeutet; und die Reste R^{8a}, R^{8b}, R^{8c} und R^{8d} unabhängig voneinander H, C₁₋₄-Alkylcarbonyl oder Benzyl, insbesondere H, Methylcarbonyl, Ethylcarbonyl oder Benzyl bedeuten und die Reste R¹, R², R³, R⁴ und R⁵ wie zuvor definiert sind, Gegenstand dieser Erfindung. Besonders bevorzugt weisen hierbei die Reste R¹, R², R³, R⁴ und R⁵ die im Anschluss an die Formeln I.2a bis I.2d angegebenen Definitionen auf.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und I, insbesondere F, Cl und Br.

Die Bezeichnung C₁₋ₙ-Alkyl, wobei n einen Wert von 1 bis 18 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc..

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 1-Hexinyl, 2-Hexinyl, 3-Hexinyl, 4-Hexinyl-, 5-Hexinyl etc.. Sofern nicht anders angegeben sind Alkinyl-Gruppen über das C-Atom in Position 1 mit dem Rest des Moleküls verbunden. Daher sind Bezeichnungen wie 1-Propinyl, 2-Propinyl, 1-Butinyl, etc. gleichbedeutend mit den Bezeichnungen 1-Propin-1-yl, 2-Propin-1-yl, 1-Butin-1-yl, etc.. Dies gilt in anologer Anwendung auch für C₂₋ₙ-Alkenyl-Gruppen.

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, Worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclodecyl, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen.

Der Begriff C₅₋ₙ-Cycloalkenyl bezeichnet eine C₅₋ₙ-Cycloalkyl-Gruppe, die wie oben definiert ist und zusätzlich mindestens eine ungesättigte C=C-Doppelbindung hat.

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist.

Der Begriff Tri-(C-₁₋₄-alkyl)silyl umfasst Silyl-Gruppen, die gleiche oder zwei oder drei verschiedene Alkylgruppen aufweisen.

Der Begriff Di-(C₁₋₃-alkyl)amino umfasst Amino-Gruppen, die gleiche oder zwei verschiedene Alkylgruppen aufweisen.

Die vorstehend und nachfolgend verwendete Schreibweise, bei der in einer Phenyl-gruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

Die erfindungsgemäßen Glucose-Derivate der Formel II können aus D-Gluconolacton oder eines Derivats davon durch Addition der gewünschten Benzylbenzol-Verbindung in Form einer Organometallverbindung (Schema 1) aufgebaut werden.

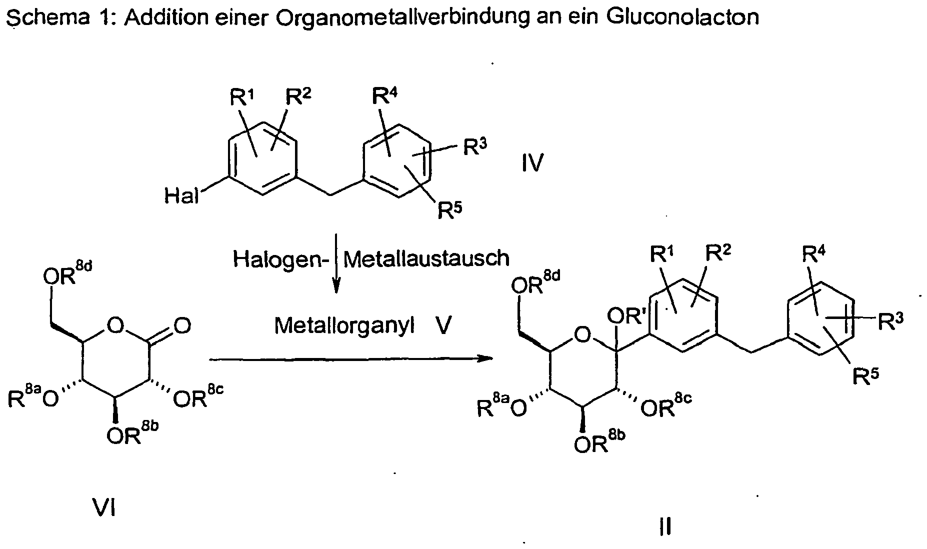

Die Reaktion gemäß Schema 1 wird vorzugsweise ausgehend von einer Halogen-Benzylbenzol-Verbindung der allgemeinen Formel IV, in der Hal Chlor-, Brom- oder lod bedeutet, durchgeführt. Ausgehend vom Halogenaromaten IV kann die entsprechende Organometallverbindung (V) entweder über einen so genannten Halogen-Metall-Austausch oder über eine Insertion des Metalls in die Kohlenstoff-Halogen-Bindung hergestellt werden. Der Halogen-Metallaustausch mit Brom- oder lod-substituierten Aromaten kann beispielweise mit einer Organolithiumverbindung wie z.B. n-, sec- oder tert-Butyllithium durchgeführt werden und liefert dabei den entsprechenden lithiierten Aromaten. Die analoge Magnesiumverbindung kann ebenfalls über einen Halogen-Metallaustausch mit einer geeigneten Grignard-Verbindung wie z.B. Isopropylmagnesiumbromid oder Diisopropylmagnesium generiert werden. Die Reaktionen werden vorzugsweise zwischen 0 und -100°C, besonders bevorzugt zwischen -10 und -80°C, in einem inerten Lösungsmittel oder Gemischen daraus, wie beispielsweise Diethylether, Tetrahydrofuran, Toluol, Hexan oder Methylenchlorid, durchgeführt. Die so erhaltenen Magnesium- bzw. Lithium-Verbindungen können gegebenenfalls mit Metallsalzen, wie z.B. Certrichlorid, zu weiteren zur Addition geeigneten Organometallverbindungen (V) ummetalliert werden. Alternativ kann die Organometallverbindung (V) auch durch Insertion eines Metalls in die Kohlenstoff-Halogen-Bindung des Halogenaromaten IV dargestellt werden. Hierzu eignen sich Metalle wie z.B. Lithium oder Magnesium. Die Addition der Organometallverbindung V an das Gluconolacton bzw. Derivaten davon der Formel VI erfolgt vorzugsweise bei Temperaturen zwischen 0 und -100°C, besonders bevorzugt bei -30 bis -80°C, in einem inerten Lösungsmittel oder Gemischen daraus unter Erhalt der Verbindung der Formel 11. Die Lithiierungs- und/oder Kupplungsreaktion kann zur Vermeidung tiefer Temperaturen auch in Mikroreaktoren und/oder Mikromischern durchgeführt werden; beispielsweise analog zu den in der WO 2004/076470 beschriebenen Verfahren.

Als Lösungsmittel eignen sich z.B. Diethylether, Toluol, Methylenchlorid, Hexan, Tetrahydrofuran oder Gemische daraus. Die Reaktionen können ohne weitere Hilfsmittel oder im Fall von reaktionsträgen Kupplungspartnern in Gegenwart von Lewis-Säuren wie z.B. BF₃*OEt₂ oder Me₃SiCl durchgeführt werden (siehe M. Schlosser, Organometallics in Synthesis, John Wiley & Sons, Chichester/New York/Brisbane/Toronto/Singapore, 1994). Hierbei bevorzugte Bedeutungen der Gruppen R^{8a}, R^{8b}, R^{8c} und R^{8d} sind Benzyl, substituiertes Benzyl, Trialkylsilyl, besonders bevorzugt Trimethylsilyl, Triisopropylsilyl, 4-Methoxybenzyl und Benzyl. Wenn zwei benachbarte Reste der Gruppe bestehend aus R^{8a}, R^{8b}, R^{8c} und R^{8d} miteinander verknüpft sind, sind diese beiden Reste bevorzugt Bestandteil eines Benzylidenacetals, 4-Methoxybenzylidenacetals, Isopropylketals oder stellen eine 2,3-Dimethoxy-butylengruppe dar, die über die 2 und 3-Position des Butans mit den benachbarten Sauerstoffatomen des Pyranoserings verknüpft ist. Der Rest R' bedeutet vorzugsweise Wasserstoff oder C₁₋₄-Alkyl, besonders bevorzugt Wasserstoff, Methyl oder Ethyl. Der Rest R' wird nach der Addition der metallorganischen Verbindung V oder eines Derivats davon an das Gluconolacton VI eingeführt. Dazu wird die Reaktionslösung mit einem Alkohol wie z.B. Methanol oder Ethanol oder Wasser in Gegenwart einer Säure wie z.B. Methansulfonsäure, Toluolsulfonsäure, Schwefelsäure oder Salzsäure behandelt.

Die Synthese von Halogenaromaten der Formel IV kann unter Anwendung von Standardtransformationen in der Organischen Chemie oder zumindest von aus der Fachliteratur bekannten Methoden in der organischen Synthese durchgeführt werden (siehe u.a. J. March, Advanced Organic Reactions, Reactions, Mechanisms, and Structure, 4. Edition, John Wiley & Sons, Chichester/New York/Brisbane/Toronto/Singapore, 1992 und darin zitierte Literatur). Die nachfolgend dargestellten Synthesestrategien sollen das stellvertretend demonstrieren.

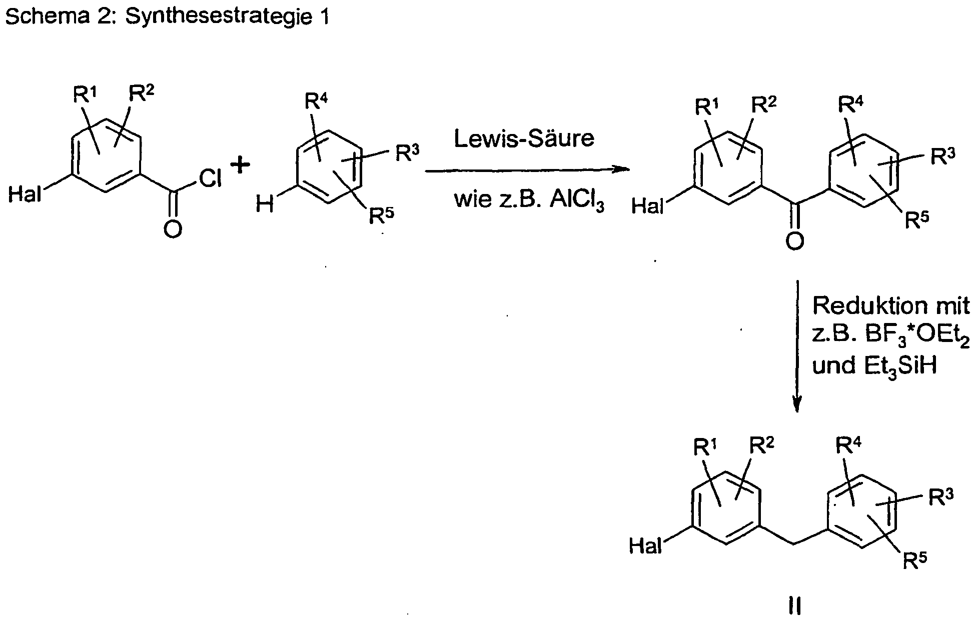

Synthesestrategie 1 (Schema 2) zeigt die Darstellung des Halogenaromaten der Formel II ausgehend von einem Benzoylchlorid und einem zweiten Aromaten, der mittels einer Friedel-Crafts-Acylierung in das Diphenylketonderivat überführt wird. Diese klassische Reaktion hat eine große Substratbreite und wird in Gegenwart eines Katalysators, der katalytisch oder stöchiometrisch eingesetzt wird, wie z.B. AlCl₃, FeCl₃, lod, Eisen, ZnCl₂, Schwefelsäure oder Trifluormethansulfonsäure durchgeführt. An Stelle des Carbonsäurechlorids können auch die Carbonsäure, ein Anhydrid oder Ester davon oder auch das entsprechende Benzonitril eingesetzt werden. Die Reaktionen werden bevorzugt in chlorierten Kohlenwasserstoffen wie z.B. Dichlormethan und 1,2-Dichlorethan bei Temperaturen von -30 bis 120°C, bevorzugt bei 30 bis 100°C durchgeführt. Aber auch lösungsmittelfreie Umsetzungen oder Umsetzungen in einem Mikrowellenofen sind möglich. In einem zweiten Reaktionsschritt wird das Diphenylketon zum Diphenylmethan reduziert. Diese Reaktion kann zweistufig über das entsprechende Diphenylmethanol oder einstufig ausgeführt werden. In der zweistufigen Variante wird das Keton mit einem Reduktionsmittel wie beispielsweise einem Metallhydrid wie z.B. NaBH₄, LiAlH₄ oder iBu₂AlH zum Alkohol reduziert. Der resultierende Alkohol lässt sich in Gegenwart einer Lewis-Säure wie beispielsweise BF₃*OEt₂, Trifluoressigsäure, InCl₃ oder AlCl₃ mit einem Reduktionsmittel wie z.B. Et₃SiH, NaBH₄, oder Ph₂SiClH zum gewünschten Diphenylmethan umformen. Der einstufige Prozess ausgehend vom Keton zum Diphenylmethan ist z.B. mit einem Silan wie z.B. Et₃SiH, einem Borhydrid wie z.B. NaBH₄ oder einem Aluminiumhydrid wie LiAlH₄ in Gegenwart einer Lewis-Säure wie beispielsweise BF₃*OEt₂, Tris(pentafluorphenyl)boran, Trifluoressigsäure, Aluminiumchlorid oder InCl₃ möglich. Die Reaktionen werden bevorzugt in Lösungsmitteln wie z.B. halogenierten Kohlenwasserstoffen wie Dichlormethan, Toluol oder Acetonitril bei Temperaturen von -30 bis 150°C, bevorzugt bei 20 bis 100°C durchgeführt. Reduktionen mit Wasserstoff in Gegenwart eines Übergangsmetallkatalysators wie z.B. Pd auf Kohle sind ebenfalls ein möglicher Syntheseweg. Reduktionen nach Wolff-Kishner oder Varianten davon sind auch möglich. Dabei wird das Keton mit Hydrazin oder einem Derivat davon, wie z.B. 1,2-Bis(tert-butyldimethylsilyl)hydrazin, zuerst in das Hydrazon überführt, welches unter stark basischen Reaktionsbedingungen und Erwärmen zum Diphenylmethan und Stickstoff zerfällt. Die Reaktion kann in einem Reaktionsschritt oder nach Isolierung des Hydrazons oder eines Derivats davon in zwei separaten Reaktionsschritten durchgeführt werden. Als Basen finden z.B. KOH, NaOH oder KOtBu in Lösungsmitteln wie z.B. Ethylenglycol, Toluol, DMSO, 2-(2-Butoxyethoxy)ethanol oder t-Butanol Anwendung; lösungsmittelfreie Umsetzungen sind ebenfalls möglich. Die Reaktionen können bei Temperaturen zwischen 20 bis 250°C, bevorzugt zwischen 80 bis 200°C durchgeführt werden. Eine Alternative zu den basischen Bedingungen der Wolff-Kishner-Reduktion ist die unter sauren Bedingungen verlaufende Clemmensen-Reduktion, die hier ebenfalls Anwendung finden kann.

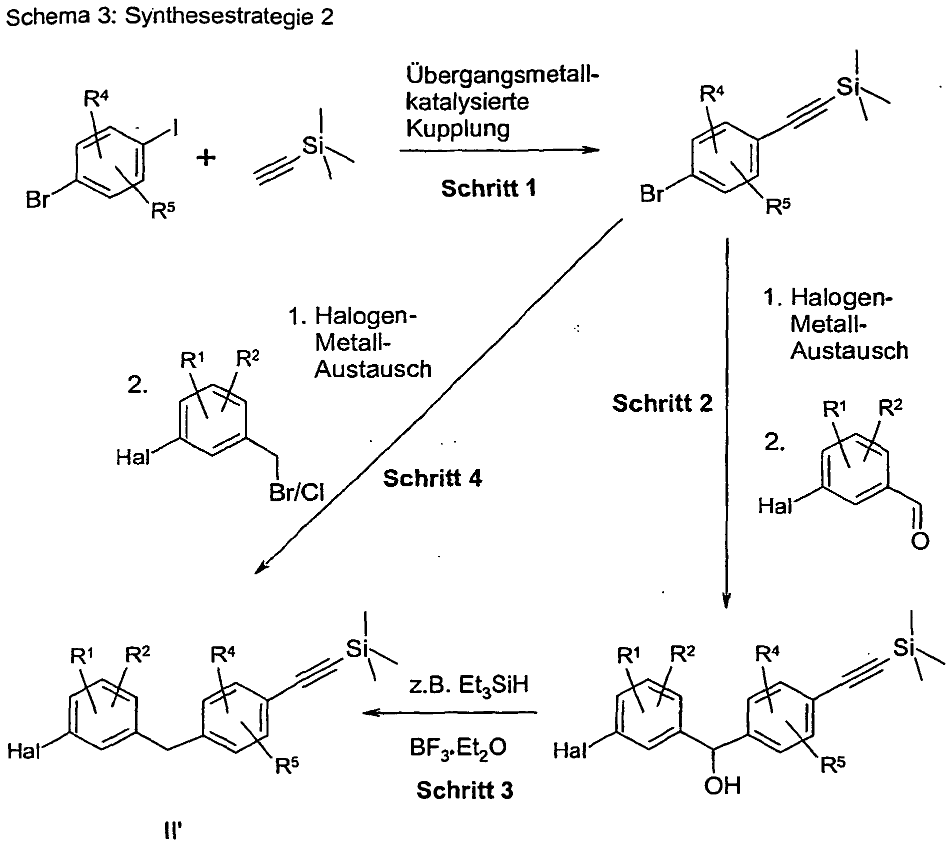

Die zweite Synthesestrategie (Schema 3) zeigt eine weitere Möglichkeit zum Aufbau des Halogen-Aromaten der Formel II' am Beispiel von einem Trimethylsilylacetylen-substituierten Diphenylmethan. Ausgehend von einem Aromaten, der zwei Reste aus der Gruppe lod, Brom, Chlor oder Sulfonat wie z.B. Trifluormethylsulfonat, trägt, wird über eine Übergangsmetall-katalysierte Monokupplung am reaktiveren Ende des Dihalogenaromaten, der lod-Kohlenstoff-Bindung, ein Alkinrest angehängt (Schritt 1). Als Katalysatoren werden beispielsweise elementares Palladium oder Nickel oder Salze oder Komplexe daraus eingesetzt. Die Reaktionen können mit dem Alkin selbst oder Metallacetyliden daraus durchgeführt werden. Wenn das Alkin selbst eingesetzt wird, kann die Kupplung in Gegenwart einer Base wie z.B. NEt₃ und eines Co-Katalysators wie z.B. eines Kupfersalzes wie Cul durchgeführt werden (Sonogashira-Kupplung). Die Reaktionen sind nicht auf Trimethylsilylacetylen beschränkt, sondern erlauben die Verwendung einer Vielzahl von terminalen Alkinen. Die Reaktion ist in allen ihren Variationen ausführlich in der Literatur dokumentiert (siehe P.J. Stang, F. Diederich, Metal-Catalyzed Cross-Coupling Reactions, Wiley-VCH, Weinheim, 1997 und Angew. Chem. Int. Ed. 2003, 42, 1566-1568 und dort zitierte Literatur). Die beiden weiteren Stufen zur Herstellung des Diphenylmethan-Derivats beinhalten die Umfunktionalisierung des Alkin-substituierten Aromaten zu einem metallierten (Mg-, Li-) Aromaten, der beispielsweise mittels eines Halogen-Metall-Austausches wie zuvor schon beschrieben, dargestellt werden kann (Schritt 2). Dieser metallierte Aromat, der direkt oder nach einer weiteren Ummetallierung eingesetzt werden kann, wird an ein Benzaldehyd-Derivat addiert. Dabei entsteht das im Schema abgebildete Diphenylmethanol. Alternativ kann auch ein Benzoesäurederivat wie z.B. ein Benzoesäureester, -anhydrid, -chlorid oder auch die Säure selbst oder das Benzonitril eingesetzt werden. Dabei entsteht statt dem Alkohol das entsprechende Keton, welches auch über die oben bereits dargestellte Friedel-Crafts-Acylierung zugänglich ist. Die weitere Umsetzung sowohl des Alkohols als auch des Ketons zum Diphenylmethan-Derivat wurde bereits oben beschrieben (Schritt 3). Der trimethylsilylethinylierte Halogenaromat kann aber auch direkt nach Ummetallierung in das gewünschte Produkt überführt werden (Schritt 4). Dazu setzt man den nach einem Halogen-Metallaustausch erhaltenen Lithium- oder Magnesium-Aromaten mit einem Benzylelektrophil wie z.B. einem Benzylbromid oder -chlorid um. Die Reaktion kann ohne oder besser in Gegenwart eines Übergangsmetallkatalysators, wie z.B. eines Kupfersalzes oder eines Palladiumkomplexes, durchgeführt werden (siehe z.B. Org. Lett. 2001, 3, 2871-2874 und dort zitierte Literatur). Der Lithium- oder Magnesiumaromat kann aber auch zuerst beispielsweise zu den entsprechenden Boronsäuren, Boronsäureestern, Stannanen, Silanen oder Zink-Verbindungen ummetalliert werden. Danach wird dann mittels eines Übergangsmetalles wie z.B. Palladium, Nickel, Rhodium, Kupfer oder Eisen der Benzylrest angeknüpft (siehe L. Brandsma, S.F. Vasilevsky, H.D. Verkruijsse, Application of Transition Metal Catalysts in Organic Synthesis, Springer-Verlag, Berlin/Heidelberg, 1998). Die Umsetzungen des Alkin-substituierten Aromaten zum Zwischenprodukt der Formel II' gemäß der Schritte 2 und 3 oder Schritt 4, die hier beispielhaft für R³ in der Bedeutung Ethinyl bzw. Trimethylsilylethinyl dargestellt sind, können in analoger Anwendung auch mit anderen R³-substituierten Aromaten durchgeführt werden.

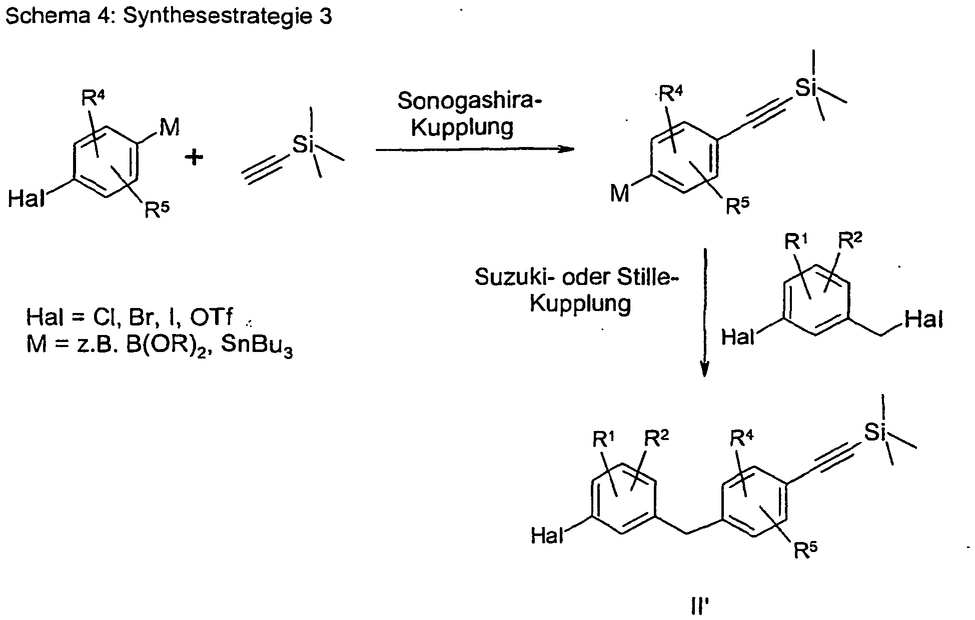

Synthesestrategie 3 (Schema 4) stellt eine Variante zu Synthesestrategie 2 vor, die ebenfalls anhand eines Trimethylsilylethinylaromaten II' verdeutlicht wird, jedoch nicht auf diesen beschränkt sein soll. Die Synthese startet mit einem Aromaten, der sowohl eine Gruppe Hal, die ein Halogenatom Chlor, Brom oder lod, oder eine Pseudohalogengruppe, wie z.B. Trifluormethansulfonat, bedeutet, als auch ein metallisches Zentrum M, wie z.B. ein B(OH)₂-, Si(OAlk)₃ oder SnBu₃-Rest trägt. Die beiden so "aktivierten" Zentren können nacheinander chemoselektiv ausgetauscht werden. Synthesestrategie 3 verdeutlicht das an einem Beispiel, in dem zuerst das Halogenatom Hal in einer Übergangsmetall-katalysierten Reaktion, wie z.B. der sogenannten Sonogashira-Kupplung, gegen einen Alkin-Substituenten ausgetauscht wird. Im zweiten Schritt wird das metallische Zentrum M in einer weiteren Übergangsmetall-katalysierten Kupplung gegen einen Benzylrest, der z.B. als Benzylhalogenid aktiviert ist, zum gewünschten Produkt ausgetauscht (siehe z.B. Tetrahedron Lett. 2003, 44, 9255-9258 und dort zitierte Literatur). Beide Schritte sind unter Einsatz von Übergangsmetallen, wie z.B. Palladium, Rhodium, Nickel, Kupfer oder Eisen, oder Komplexen dieser durchführbar. Beide Reaktionstypen sind in der Literatur ausführlich beschrieben. Das Vorgehen ist nicht auf das hier Geschilderte beschränkt, sondern kann auch unter Umkehrung der Reihenfolge der beiden Reaktionsschritte durchgeführt werden. Hiemach wird zuerst das metallische Zentrum M mit dem Benzylrest verknüpft und dann die Halogen- oder Pseudohalogengruppe Hal gegen das Alkin ausgetauscht.

Zur Herstellung von Verbindungen der allgemeinen Formel I wird gemäß dem erfindungsgemäßen Verfahren a) eine Verbindung der allgemeinen Formel II 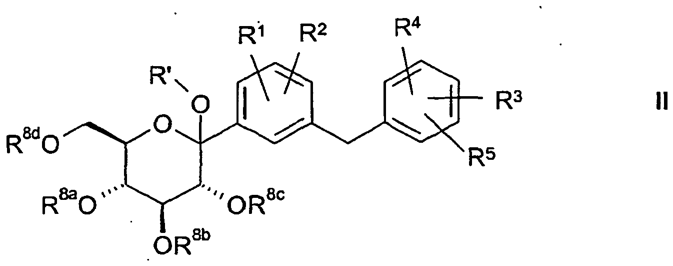 in der R', R¹ bis R⁵ wie zuvor definiert sind und
R^{8a}, R^{8b}, R^{8c}, R^{8d} wie zuvor definiert sind und beispielsweise unabhängig voneinander Acetyl, Pivaloyl, Benzoyl, tert-Butoxycarbonyl, Benzyloxycarbonyl, Trialkylsilyl, Benzyl oder substituiertes Benzyl bedeuten oder jeweils zwei benachbarte Reste R^{8a}, R^{8b}, R^{8c}, R^{8d} ein Benzylidenacetal oder Isopropylidenketal bilden oder eine 2,3-Dimethoxy-butylen-Gruppe, die über die Position 2 und 3 der Butylengruppe mit den Sauerstoffatomen des Pyranoserings verknüpft ist und mit diesen ein substituiertes Dioxan bildet,
die wie zuvor beschrieben erhältlich ist, mit einem Reduktionsmittel in Gegenwart einer Lewis- oder Brønsted-Säure umgesetzt.

Für die Umsetzung eignen sich als Reduktionsmittel beispielsweise Silane, wie Triethyl-, Tripropyl-, Trüsopropyl- oder Diphenylsilan, Natriumborhydrid, Natriumcyanoborhydrid, Zinkborhydrid, Boran, Lithiumaluminiumhydrid, Düsobutylaluminiumhydrid oder Samariumiodid. Die Reduktionen finden ohne oder in Gegenwart einer geeigneten Bronsted-Säure, wie z.B. Salzsäure, Toluolsulfonsäure, Trifluoressigsäure oder Essigsäure, oder Lewis-Säure, wie z.B. Bortrifluoridetherat, Trimethylsilyltriflat, Titantetrachlorid, Zinntetrachlorid, Scandiumtriflat oder Zinkiodid statt. In Abhängigkeit vom Reduktionsmittel und der Säure kann die Reaktion in einem Lösungsmittel, wie beispielsweise Methylenchlorid, Chloroform, Acetonitril, Toluol, Hexan, Diethylether, Tetrahydrofuran, Dioxan, Ethanol, Wasser oder Gemischen daraus bei Temperaturen zwischen -60°C und 120°C durchgeführt werden. Ein besonders geeignete Reagenzienkombination besteht beispielsweise aus Triethylsilan und Bortrifluorid-Etherat, die zweckmäßigerweise in Acetonitril oder Dichlormethan bei Temperaturen von -60°C und 60°C zum Einsatz kommt. Des Weiteren kann Wasserstoff in Gegenwart eines Übergangsmetallkatalysators, wie z.B. Palldium auf Kohle oder Raney-Nickel, in Lösungsmitteln wie Tetrahydrofuran, Ethylacetat, Methanol, Ethanol, Wasser oder Essigsäure, für die dargestellte Transformation angewendet werden.

Alternativ werden zur Herstellung von Verbindungen der allgemeinen Formel I gemäß dem erfindungsgemäßen Verfahren b) in einer Verbindung der allgemeinen Formel III 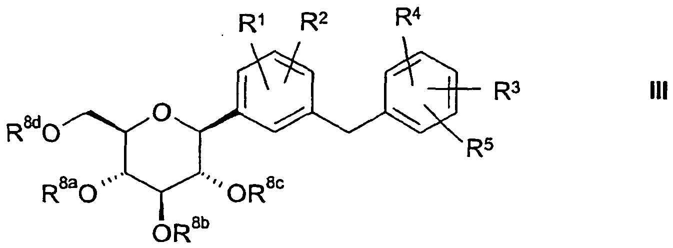 in der R¹ bis R⁵ wie zuvor definiert sind und
R^{8a} bis R^{8d} eine der zuvor definierten Schutzgruppen, wie z.B. eine Acyl-, Arylmethyl-, Acetal-, Ketal- oder Silylgruppe bedeuten, und die beispielsweise aus der Verbindung der Formel II wie zuvor beschrieben durch Reduktion erhältlich ist, die Schutzgruppen abgespalten.

Die Abspaltung eines verwendeten Acyl-Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C. Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran oder Methanol bei Temperaturen zwischen 0 und 50°C.

Die Abspaltung eines verwendeten Acetal- oder Ketal-Schutzrestes erfolgt beispielsweise hydrolytisch in einem wässrigen Lösungsmittel, z.B. in Wasser, IsopropanoI/Wasser, Essigsäure/Wasser, Tetrahydrofuran/Wasser oder Dioxan/Wasser, in Gegenwart einer Säure wie Trifluoressigsäure, Salzsäure oder Schwefelsäure oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Trimethylsilylrestes erfolgt beispielsweise in Wasser, einem wässrigen Lösemittelgemisch oder einem niederen Alkohol wie Methanol oder Ethanol in Gegenwart einer Base wie Lithiumhydroxid, Natriumhydroxid, Kaliumcarbonat oder Natriummethylat. In wässrigen oder alkoholischen Lösungsmitteln eignen sich ebenfalls Säuren, wie z.B. Salzsäure, Trifluoressigsäure oder Essigsäure. Zur Abspaltung in organischen Lösungsmitteln, wie beispielsweise Diethylether, Tetrahydrofuran oder Dichlormethan, eignen sich auch Fluoridreagenzien, wie z.B. Tetrabutylammoniumfluorid.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig, gegebenenfalls unter Zusatz einer Säure wie Salzsäure bei Temperaturen zwischen 0 und 100°C, vorzugsweise jedoch bei Raumtemperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Bei den vorstehend beschriebenen Umsetzungen können gegebenenfalls vorhandene reaktive Gruppen wie Ethinyl-, Hydroxy-, Amino-, Alkylamino- oder Iminogruppen während der Umsetzung durch übliche Schutzgruppen geschützt werden, welche nach der Umsetzung wieder wie u.a. oben beschrieben abgespalten werden.

Beispielsweise kommen als Schutzrest für eine Ethinylgruppe die Trimethylsilyl- oder Triisopropytgruppe in Betracht. Die 2-Hydroxisoprop-2-ylgruppe kann ebenfalls als Schutzgruppe Anwendung finden.

Beispielsweise kommen als Schutzrest für eine Hydroxygruppe die Trimethylsilyl-, Acetyl-, Trityl-, Benzyl- oder Tetrahydropyranylgruppe in Betracht.

Als Schutzreste für eine Amino-, Alkylamino- oder Iminogruppe kommen beispielsweise die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe in Betracht.

Ferner können die erhaltenen Verbindungen der allgemeinen Formel 1, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel 1 mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Weiterhin können die erhaltenen Verbindungen in Gemische, beispielsweise in 1:1 oder 1:2 Gemische mit Aminosäuren, insbesondere mit alpha-Aminosäuren wie Prolin oder Phenylalanin,- übergeführt werden, die besonders günstige Eigenschaften wie hohe Kristallinität aufweisen können.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren, insbesondere den in den WO 98/31697, WO 01/27128, WO 02/083066, WO 03/099836 und WO 2004/063209 beschriebenen Verfahren, kombiniert werden können.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf natriumabhängige Glucose-Cotransporter, vorzugsweise SGLT2.

Die biologischen Eigenschaften der neuen Verbindungen können wie folgt geprüft werden:

Die Fähigkeit der Substanzen die SGLT-2 Aktivität zu hemmen, kann in einem Versuchsaufbau gezeigt werden, in dem eine CHO-K1 Zelllinie (ATCC No. CCL 61) oder alternativ eine HEK293 Zeillinie (ATCC No. CRL-1573), die stabil mit einem Expressionsvektor pZeoSV (Invitrogen, EMBL accession number L36849) transfiziert ist, der die cDNA für die kodierende Sequenz des humanen Natrium Glucose Cotransporters 2 (Genbank Acc. No.NM_003041) enthält (CHO-hSGLT2 bzw. HEK-hSGLT2). Diese Zelllinien transportieren Natrium-abhängig ¹⁴C-markiertes alpha-Methyl-Glucopyranosid (¹⁴C-AMG, Amersham) in das Zellinnere.

Der SGLT-2 Assay wird wie folgt durchgeführt:
CHO-hSGLT2 Zellen werden in Ham's F12 Medium (BioWhittaker) mit 10% fötalem Kälberserum und 250 µg/ml Zeocin (Invitrogen), HEK293-hSGLT2 Zellen in DMEM Medium mit 10% fötalem Kälberserum und 250 µg/ml Zeocin (Invitrogen) kultiviert.

Die Zellen werden von den Kulturflaschen durch zweimaliges Waschen mit PBS und anschließende Behandlung mit Trypsin/EDTA abgelöst. Nach Zugabe von Zellkulturmedium werden die Zellen abzentrifugiert, in Kulturmedium resuspendiert und in einem Casy-cellcounter gezählt. Anschließend werden 40.000 Zellen pro Loch in eine weiße, Poly-D-Lysin beschichtete 96-Loch Platte ausgesät und über Nacht bei 37°C, 5% CO₂ inkubiert. Die Zellen werden zweimal mit 300 µl Assaypuffer (Hanks Balanced Salt Solution, 137 mM NaCl, 5,4 mM KCI, 2,8 mM CaCl₂, 1,2 mM MgSO₄ und 10 mM HEPES (pH7,4), 50µg/ml Gentamycin) gewaschen. In jedes Loch werden dann 250 µl Assaypuffer und 5 µl Testverbindung hinzugegeben und für weitere 15 Minuten im Brutschrank inkubiert. Als Negativkontrolle werden 5 µl 10% DMSO eingesetzt Durch Zugabe von 5 µl ¹⁴C-AMG (0.05 µCi) in jedes Loch wird die Reaktion gestartet. Nach einer 2 stündigen Inkubation bei 37°C, 5% CO₂ werden die Zellen wiederum mit 300 µl PBS (20°C) gewaschen und anschließend durch Zugabe von 25 µl 0.1 N NaOH lysiert (5 min. bei 37°C). Pro Loch werden 200 µl MicroScint20 (Packard) hinzugefügt und für weitere 20 min bei 37°C inkubiert. Nach dieser Inkubation wird die Radioaktivität des aufgenommenen ¹⁴C-AMG in einem Topcount (Packard) mittels eines ¹⁴C-Szintillationsprogramms gemessen.

Zur Bestimmung der Selektivität gegenüber dem humanen SGLT1 wird ein analoger Test aufgebaut, in dem die cDNA für hSGLT1 (Genbank Acc. No. NM000343) statt der hSGLT2 cDNA in CHO-K1 bzw. HEK293 Zellen exprimiert wird.

Die erfindungsgemäßen Verbindungen der allgemeinen Formel I können beispielsweise EC50-Werte unter 1000 nM, insbesondere unter 200 nM, besonders bevorzugt unter 50 nM aufweisen.

Im Hinblick auf die Fähigkeit, die SGLT Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der SGLT Aktivität, insbesondere der SGLT-2 Aktivität beeinflusst werden können. Daher sind erfindungsgemäße Verbindungen insbesondere zur Prophylaxe oder Behandlung von Krankheiten, insbesondere Stoffwechselerkrankungen, oder Zuständen wie Diabetes mellitus Typ 1 und Typ 2, diabetische Komplikationen (wie z.B. Retinopathie, Nephropathie oder Neuropathien, diabetischer Fuß, Ulcus, Makroangiopathien), metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme. Hyperurikämie geeignet. Darüber hinaus sind diese Substanzen geeignet, die beta-Zelldegeneration wie z.B. Apoptose oder Nekrose von pankreatischen beta-Zellen zu verhindern. Die Substanzen sind weiter geeignet, die Funktionalität von pankreatischen Zellen zu verbessern oder wiederherzustellen, daneben die Anzahl und Größe von pankreatischen beta-Zellen zu erhöhen. Die erfindungsgemäßen Verbindungen sind ebenfalls als Diuretika oder Antihypertensiva einsetzbar und zur Prophylaxe und Behandlung des akuten Nierenversagens geeignet.

Ganz besonders sind die erfindungsgemäßen Verbindungen, einschließlich deren physiologisch verträglichen Salze, zur Prophylaxe oder Behandlung von Diabetes, insbesondere Diabetes mellitus Typ 1 und Typ 2, und/oder diabetischen Komplikationen geeignet.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 1 bis 100 mg, vorzugsweise 1 bis 30 mg, und bei oraler Gabe im Bereich von 1 bis 1000 mg, vorzugsweise 1 bis 100 mg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, WasserlPolyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Lösungen, Suspensionen oder Zäpfchen einarbeiten.

Die erfindungsgemäßen Verbindungen können auch in Kombination mit anderen Wirkstoffen, insbesondere zur Behandlung und/oder Prophylaxe der zuvor angegebenen Krankheiten und Zustände verwendet werden. Für solche Kombinationen kommen als weitere Wirksubstanzen insbesondere solche in Betracht, die beispielsweise die therapeutische Wirksamkeit eines erfindungsgemäßen SGLT-Hemmers im Hinblick auf eine der genannten Indikationen verstärken und/oder die eine Reduzierung der Dosierung eines erfindungsgemäßen SGLT-Hemmers erlauben. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 29.7), alpha-Glucosidasehemmer (z.B. Acarbose, Voglibose), DPPIV Inhibitoren (z.B. LAF237, MK-431), alpha2-Antagonisten, Insulin und Insulinanaloga, GLP-1 und GLP-1 Analoga (z.B. Exendin-4) oder Amylin. Daneben sind weitere als Kombinationspartner geeignete Wirkstoffe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha Agonisten, PPAR-delta Agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder ß3-Agonisten wie SB-418790 oder AD-9677 ebenso wie Agonisten des 5HT2c Rezeptors.

Daneben ist eine Kombination mit Medikamenten zur Beeinflussung des Bluthochdrucks, des chronischen Herzversagens oder der Atherosklerose wie z. B. A-II Antagonisten oder ACE Inhibitoren, ECE-Inhibitoren, Diuretika, β-Blocker, Ca-Antagonisten, zentral wirksamen Antihypertensiva, Antagonisten des alpha-2-adrenergen Rezeptors, Inhibitoren der neutralen Endopeptidase, Thrombozytenaggregationshemmer und anderen oder Kombinationen daraus geeignet. Beispiele von Angiotensin II Rezeptor Antagonisten sind Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701, etc.. Angiotensin II Rezeptor Antagonisten werden vorzugsweise zur Behandlung oder Prophylaxe von Bluthochdruck und diabetischen Komplikationen verwendet, oft in Kombination mit einem Diuretikum wie Hydrochlorothiazide.

Zur Behandlung oder Prophylaxe der Gicht ist eine Kombination mit Harnsäuresynthese Inhibitoren oder Urikosurika geeignet.

Zur Behandlung oder Prophylaxe diabetischer Komplikationen kann eine Kombination mit GABA-Rezeptor-Antagonisten, Na-Kanal-Blockem, Topiramat, Protein-Kinase C Inhibitoren, advanced glycation endproduct Inhibitoren oder Aldose Reduktase Inhibitoren erfolgen.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des natriumabhängigen Glucose-Cotransporters SGLT beeinflussbar sind. Hierbei handelt es sich vorzugsweise um eine Stoffwechselerkrankung, insbesondere eine der zuvor angeführten Erkrankungen oder Zustände, ganz besonders Diabetes oder diabetischer Komplikationen.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen und/oder Verdünnungsmitteln aufweist.

So weist beispielsweise ein erfindungsgemäßes Arzneimittel eine Kombination aus einer erfindungsgemäßen Verbindung der Formel I oder eines physiologisch verträglichen Salzes solch einer Verbindung sowie mindestens einem Angiotensin II Rezeptor Antagonisten neben gegebenenfalls einem oder mehreren inerten Trägerstoffen und/oder Verdünnungsmitteln auf.

Die erfindungsgemäße Verbindung, oder eines physiologisch verträglichen Salzes, und der damit zu kombinierende weitere Wirkstoff können zusammen in einer Darreichungsform, beispielsweise einer Tablette oder Kapsel, oder getrennt in zwei gleichen oder verschiedenen Darreichungsformen, beispielsweise als sogenanntes kit-of-parts, vorliegen.

Vorstehend und nachfolgend werden in Strukturformeln H-Atome von Hydroxylgruppen nicht in jedem Fall explizit dargestellt. Die nachfolgenden Beispiele sollen die vorliegende Erfindung näher erläutern ohne diese zu beschränken:

### Herstellung der Ausgangsverbindungen:

### Beispiel I

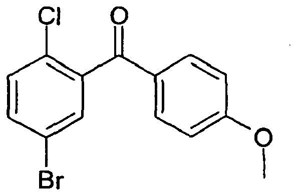

### (5-Brom-2-chlor-phenyl)-(4-methoxy-phenyl)-methanon

Zu einer Mischung von 100 g 5-Brom-2-chlor-benzoesäure in 500 ml Dichlormethan werden 38,3 ml Oxalylchlorid und 0,8 ml Dimethylformamid gegeben. Das Reaktionsgemisch wird 14 h gerührt, danach filtriert und von allen flüchtigen Bestandteilen im Rotationsverdampfer getrennt. Der Rückstand wird in 150 ml Dichlormethan gelöst, die Lösung auf -5°C abgekühlt, und es werden 46,5 g Anisol zugegeben. Danach werden 51,5 g Aluminiumtrichlorid portionsweise so zugegeben, dass die Temperatur nicht über 5°C steigt. Die Lösung wird noch 1 h bei 1-5°C gerührt und anschließend auf Eis gegossen. Die organische Phase wird abgetrennt und die wässrige noch drei Mal mit Dichlormethan extrahiert. Die vereinigten organischen Phasen werden mit wässriger 1 M Salzsäure, zwei Mal mit 1 M Natronlauge und mit gesättigter Natriumchlorid-Lösung gewaschen. Danach wird die organische Phase getrocknet, das Lösungsmittel entfernt und der Rückstand in Ethanol umkristallisiert.
Ausbeute: 86,3 g (64% der Theorie)
Massenspektrum (ESI⁺): m/z = 325/327/329 (Br+Cl) [M+H]⁺

Analog Beispiel I werden folgende Verbindungen erhalten:
(1) (5-Brom-2-iod-phenyl)-(4-ethoxy-phenyl)-methanon 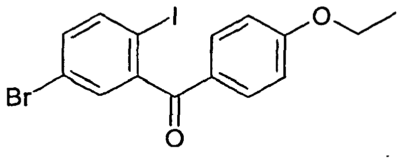 Massenspektrum (ESI⁺): m/z = 431/433 (Br) [M+H]⁺
(2) (5-Brom-2-chlor-phenyl)-(4-iod-phenyl)-methanon

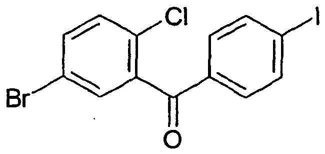

### Beispiel II

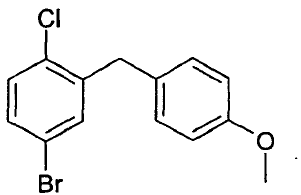

### 4-Brom-1-chlor-2-(4-Methoxy-benzyl)-benzol

Eine Lösung von 86,2 g (5-Brom-2-chlor-phenyl)-(4-methoxy-phenyl)-methanon und 101,5 ml Triethylsilan in 75 ml Dichlormethan und 150 ml Acetonitril wird auf 10°C abgekühlt. Dann werden unter Rühren 50,8 ml Bortrifluoridetherat so zugegeben, dass die. Temperatur nicht über 20°C steigt. Die Lösung wird 14 h bei Raumtemperatur gerührt, bevor noch einmal 9 ml Triethylsilan und 4,4 ml Bortrifluoridetherat zugegeben werden. Die Lösung wird weitere 3 h bei 45-50°C gerührt und dann auf Raumtemperatur abgekühlt. Es wird eine Lösung von 28 g Kaliumhydroxid in 70 ml Wasser zugesetzt und 2 h gerührt. Danach wird die organische Phase abgetrennt und die wässrige noch drei Mal mit Diisopropylether extrahiert. Die vereinten organischen Phasen werden zwei Mal mit 2 M Kalilauge und einmal mit wässriger Natriumchlorid-Lösung gewaschen und anschließend über Natriumsulfat getrocknet. Nach Entfernen des Lösungsmittels wird der Rückstand in Ethanol verrührt, wieder abgetrennt und bei 60°C getrocknet.
Ausbeute: 50,0 g (61% der Theorie)
Massenspektrum (ESI⁺): m/z = 310/312/314 (Br+Cl) [M+H]⁺

Analog Beispiel II werden folgende Verbindungen erhalten:
(1) 4-Brom-1-iod-2-(4-ethoxy-benzyl)-benzol 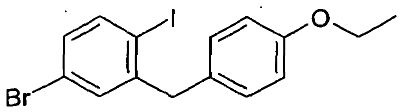 Massenspektrum (ESI⁺): m/z =4341436 [M+NH₄]⁺
(2) 4-Brom-1-chlor-2-(4-iod-benzyl)-benzol

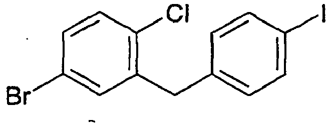

### Beispiel III

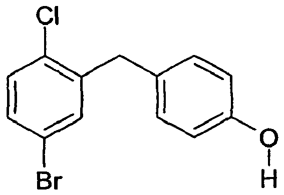

### 4-(5-Brom-2-chlor-benzyl)-Phenol

Eine Lösung von 14,8 g 4-Brom-1-chlor-2-(4-methoxy-benzyl)-benzol in 150 ml Dichlormethan wird im Eisbad abgekühlt. Dann werden 50 ml einer 1 M Lösung von Bortribromid in Dichlormethan zugegeben, und die Lösung wird 2 h bei Raumtemperatur gerührt. Die Lösung wird danach wieder im Eisbad gekühlt, und es wird gesättigte Kaliumcarbonat-Lösung zugetropft. Bei Raumtemperatur wird mit wässriger 1 M Salzsäure auf einen pH-Wert von 1 eingestellt, die organische Phase abgetrennt und die wässrige noch drei Mal mit Ethylacetat extrahiert. Die vereinten organischen Phasen werden über Natriumsulfat getrocknet, und das Lösungsmittel wird vollständig entfernt
Ausbeute: 13,9 g (98% der Theorie)
Massenspektrum (ESI⁻): m/z = 295/297/299 (Br+CI) [M-H]⁻

### Beispiel IV

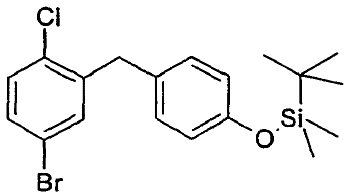

### [4-(5-Brom-2-chlor-benzyl)-phenoxyl]-tert-butyl-dimethyl-silan

Eine Lösung von 13,9 g 4-(5-Brom-2-chlor-benzyl)-phenol in 140 ml Dichlormethan wird im Eisbad abgekühlt. Dann werden 7,54 g tert-Butyldimethylsilylchlorid in 20 ml Dichlormethan gefolgt von 9,8 ml Triethylamin und 0,5 g Dimethylaminopyridin zugegeben. Die Lösung wird 16 h bei Raumtemperatur gerührt und dann mit 100 ml Dichlormethan verdünnt. Die organische Phase wird zwei Mal mit wässriger 1 M Salzsäure und einmal mit wässriger Natriumhydrogencarbonatlösung gewaschen und anschließend über Natriumsulfat getrocknet. Nach Entfernen des Lösungsmittels wird der Rückstand über Kieselgel filtriert (Cyclohexan/Ethylacetat 100:1).
Ausbeute: 16,8 g (87% der Theorie)
Massenspektrum (EI): m/z = 410/412/414 (Br+CI) [M]⁺

### Beispiel V

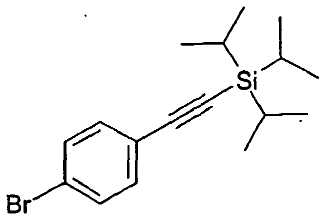

### 1-Brom-4-triisopropylsilylethinyl-benzol

Unter Argon werden zu einer sauerstofffreien Lösung von 15,0 g 1-Brom-4-iod-benzol in 150 ml trockenem Tetrahydrofuran 11,6 ml Triisopropylacetylen und 14,4 ml Triethylamin gefolgt von 0,2 g Kupferiodid und 0,73 g Bis-(triphenylphosphin)-palladiumdichlorid gegeben. Die Lösung wird 16 h bei Raumtemperatur gerührt und dann über Celite filtriert und eingeengt. Der Rückstand wird über Kieselgel chromatografiert (Cyclohexan).
Ausbeute: 17,4 g (100% der Theorie)
Massenspektrum (ESI⁺): m/z = 336/338 (Br) [M]⁺

Analog Beispiel V werden folgende Verbindungen erhalten:
(1) 4-Brom-1-(triisopropylsilylethinyl)-2-(4-ethoxy-benzyl)-benzol 4-Brom-1-iod-2-(4-ethoxy-benzyl)-benzol wird hierbei als Ausgangsmaterial für die zuvor beschriebene Kupplungsreaktion verwendet. 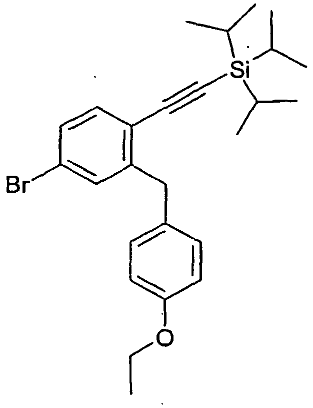 Massenspektrum (ESI⁺): m/z = 471/473 (Br) [M+H]⁺
(2) [4-(5-Brom-2-chlor-benzyl)-phenylethinyl]-triisopropyl-silan 4-Brom-1-chlor-2-(4-iod-benzyl)-benzol wird als Ausgangsmaterial verwendet.

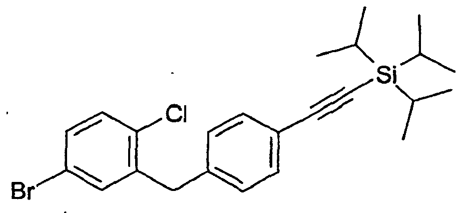

Diese Verbindung kann auch gemäß Beispiel X erhalten werden.

### Beispiel VI

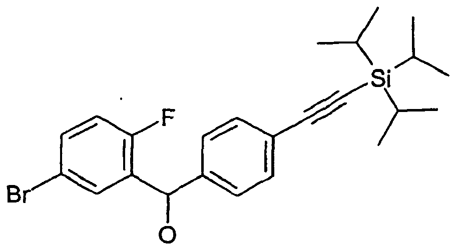

### (5-Brom-2-fluor-phenyl)-{4-[(triisopropylsilyl)-ethinyl]-phenyl}-methanol

Zu einer -78°C-kalten Lösung von 17,4 g 1-Brom-4-trüsopropylsilylethinyl-benzol in 120 ml trockenem Tetrahydrofuran werden unter Argon 33,8 ml einer 1,6 M Lösung von n-Butyllithium in Hexan getropft. Die Lösung 1 h bei -70°C gerührt. Danach werden 10,8 g 5-Brom-2-fluor-benzaldehyd in 30 ml Tetrahydrofuran gelöst über 15 min zugetropft. Die resultierende Lösung wird über Nacht im Kühlbad auf Raumtemperatur erwärmen gelassen. Danach wird Wasser zugegeben und mit Ethylacetat extrahiert. Die vereinigten organischen Phase werden über Natriumsulfat getrocknet, und das Lösungsmittel wird entfernt. Der Rückstand wird über Kieselgel (Cyclohexan/Ethylacetat 4:1) gereinigt.
Ausbeute: 14,3 g (60% der Theorie)
Massenspektrum (ESI⁺): m/z = 461/463 (Br) [M+H]⁺

Analog Beispiel VI werden folgende Verbindungen erhalten:
(1) (3-Brom-phenyl)-{4-[(triisopropylsilyl)-ethinyl]-phenyl}-methanol 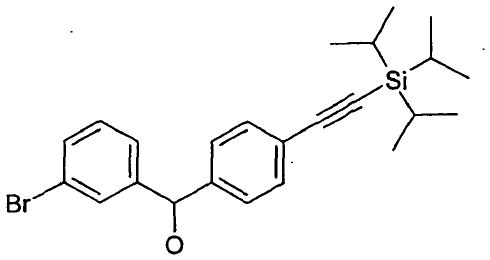 Massenspektrum (ESI⁻): m/z = 487/489 (Br) [M+HCOO]⁻
(2) (5-Brom-2-methoxy-phenyl)-{4-[(triisopropylsityl)-ethinyl]-phenyl}-methanol 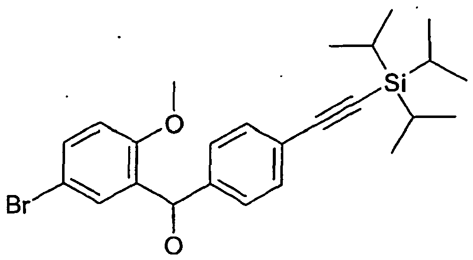
Massenspektrum (ESI⁺): m/z = 473/475 (Br) [M+H]⁺

### Beispiel VII

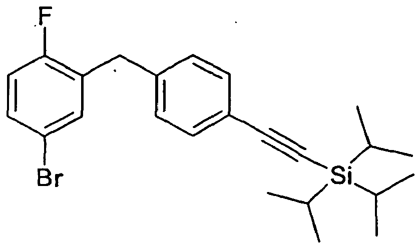

### [4-(5-Brom-2-fluor-benzyl)-phenylethinyl]-triisopropyl-silan

Eine Lösung von 5,6 g (5-Brom-2-fluor-phenyl)-{4-[(triisopropylsilyl)-ethinyl]-phenyl}-methanol und 4,1 ml Triethylsilan in 50 ml Dichlormethan wird im Eisbad abgekühlt. Dann werden 4,7 ml Trifluoressigsäure langsam zugetropft, und die Lösung wird 4 h bei Raumtemperatur gerührt. Die Lösung wird mit Dichlormethan verdünnt und mit wässeriger Natriumhydrogencarbonatlösung gewaschen. Nach Trocknen über Natriumsulfat wird das Lösungsmittels entfernt und der Rückstand über Kieselgel gereinigt (Cyclohexan).
Ausbeute: 2,6 g (48% der Theorie)
Massenspektrum (EI): m/z = 445/447 (Br) [M]⁺

Analog Beispiel VII werden folgende Verbindungen erhalten:
(1) [4-(3-Brom-benzyl)-phenylethinyl]-triisopropyl-silan 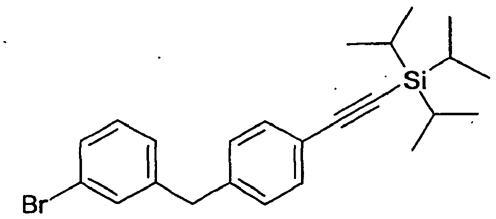 Massenspektrum (ESI⁺): m/z = 4271429 (Br) [M+H]⁺
(2) [4-(5-Brom-2-methoxy-benzyl)-phenylethinyl]-triisopropyl-silan

Abweichend vom zuvor beschriebenen Verfahren wird hier die Reaktionslösung im Eisbad statt bei Raumtemperatur bis zur vollständigen Umsetzung gerührt. 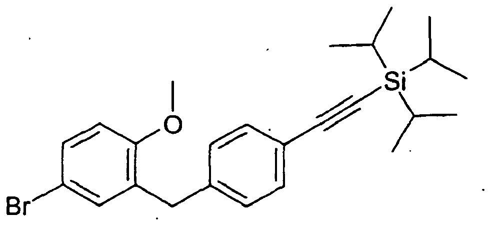 Massenspektrum (ESI⁺): m/z = 457/459 (Br) [M+H]⁺

### Beispiel VIII

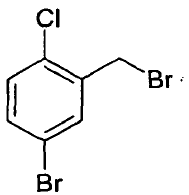

### 4-Brom-2-brommethyl-1-chlor benzol

Zu einer 5°C-kalten Lösung von 5,0 g 4-Brom-1-chlor-2-hydroxymethyl-benzol und 5,9 g Triphenylphosphin in 50 ml Tetrahydrofuran werden langsam 4,0 g N-Bromsuccinimid gegeben. Nach 1 h Rühren bei Raumtemperatur wird der Niederschlag abfiltriert und das Lösungsmittel im Vakuum entfernt. Der Rückstand wird über Kieselgel gereinigt (Cyclohexan/Ethylacetat 50:1).
Ausbeute: 4,9 g (76% der Theorie)
Massenspektrum (EI): m/z = 282/284/286 (Br+CI) [M]⁺

### Beispiel IX

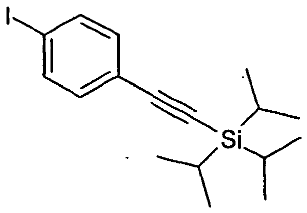

### (4-lod-phenylethinyl)-triisopropyl-silan

Unter Argon werden zu einer Lösung von 20,0 g (4-Brom-phenylethinyl)-trüsopropyl-silan 18,0 g Natriumiodid (trocken), 0,6 g Kupferiodid und 0,8 g N,N'-Dimethyl-cyclohexan-1,2-diamin gegeben. Die Lösung wird 24 h unter Rückfluss gerührt und dann auf Raumtemperatur abgekühlt. Es wird 1 %ige Ammoniaklösung (100 ml) zugegeben und mit Ethylacetat extrahiert. Nach Trocknen über Natriumsulfat wird das Lösungsmitttel entfernt und der Rückstand über Kieselgel gereinigt (Cycfohexan).
Ausbeute: 21,0 g (92% der Theorie)
Massenspektrum (EI): m/z = 384 [M]⁺

### Beispiel X

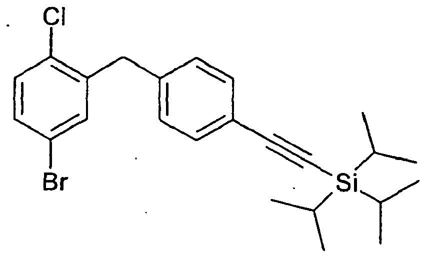

### [4-(5-Brom-2-chlor-benzyl)-phenylethinyl]-triisopropyl-silan

Unter Argon werden zu einer -25°C-kalten Lösung von 0,50 g (4-lod-phenylethinyl)-trüsopropyl-silan in 2,2 ml trockenem Tetrahydrofuran 0,66 ml einer 2 M Lösung von Isopropylmagnesiumchlorid in Tetrahydrofuran getropft. Die Lösung wird 30 min bei -25°C gerührt und dann mit 0,26 ml einer 1 M Lösung von CuCN*2 LiCl in Tetrahydrofuran (hergestellt durch Lösen von CuCN und LiCl im Verhältnis 1:2) versetzt. Kurz danach werden 0,35 g 4-Brom-2-brommethyl-1-chlorbenzol zugegeben und das Reaktionsgemisch im Kühlbad auf -5°C erwärmt. Nach 6 h Rühren bei -5°C wird die Lösung auf Raumtemperatur erwärmt und über Nacht gerührt. Danach wird eine Mischung aus gesättigter Ammoniumchloridlösung und 25%iger Ammoniaklösung (9:1) zugegeben und das resultierende Gemisch zu Wasser gegeben. Die organische Phase wird abgetrennt und die wässrige mit Ethylacetat extrahiert, die vereinten organischen Phasen werden über Natriumsulfat getrocknet, und das Lösungsmittel wird entfernt. Der Rückstand wird über Kieselgel gereinigt (Cyclohexan).
Ausbeute: 0,28 g (50% der Theorie)
Massenspektrum (EI): m/z = 461/463/465 (Br+Cl) [M+H]⁺

### Beispiel XI

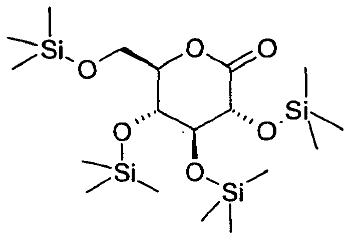

### 2,3,4,6-Tetrakis-O-(trimethylsilyl)-D-glucopyranon

Eine Lösung von 20 g D-Glucono-1,5-lacton und 98,5 ml N-Methylmorpholin in 200 ml Tetrahydrofuran wird auf -5°C abgekühlt. Dann werden 85 ml Trimethylsilylchlorid so zugetropft, dass die Temperatur nicht über 5°C steigt. Die Lösung wird danach 1 h bei Raumtemperatur, 5 h bei 35°C und noch einmal 14 h bei Raumtemperatur gerührt. Nach Zugabe von 300 ml Toluol wird die Lösung im Eisbad abgekühlt, und es werden 500 ml Wasser so zugegeben, dass die Temperatur nicht über 10°C steigt. Die organische Phase wird anschließend abgetrennt und jeweils einmal mit wässriger Natriumdihydrogenphosphatlösung, Wasser und gesättigter wässriger Natriumchloridlösung gewaschen. Das Lösungsmittel wird entfernt, der Rückstand in 250 ml Toluol aufgenommen und das Lösungsmittel erneut vollständig entfernt.
Ausbeute: 52,5 g (ca. 90% rein)
Massenspektrum (ESI⁺): m/z = 467 [M+H]⁺

### Beispiel XII

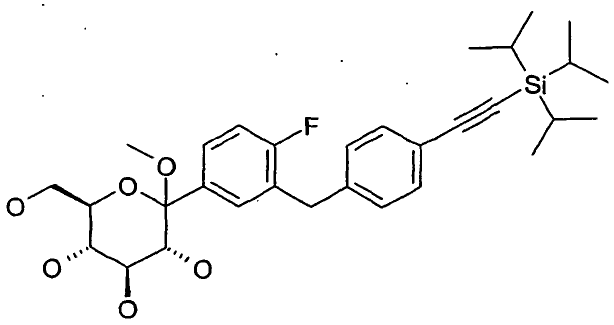

### 1-Fluor-4-(1-methoxy-D-glucopyranos-1-yl)-2-(4-triisopropylsilylethinyl-benzyl)-benzol

Eine Lösung von 4,46 g [4-(5-Brom-2-fluor-benzyl)-phenylethinyl]-triisopropyl-silan in 30 ml trockenem Diethylether wird unter Argon auf -80°C abgekühlt. Zu der gekühlten Lösung werden langsam 11,8 ml einer 1,7 M Lösung von tert-Butyllithium in Pentan getropft, und dann wird die Lösung 45 min bei -80°C gerührt. Zu dieser Lösung wird nun über eine Umdrücknadel eine -80°C-kalte Lösung von 5,19 g 2,3,4,6-Tetrakis-O-(trimethylsilyl)-D-glucopyranon in 50 ml Diethylether getropft. Die resultierende Lösung wird 3 h bei -78°C gerührt. Danach wird eine Lösung von 1,7 ml Methansulfonsäure in 50 ml Methanol zugegeben, das Kühlbad entfernt und die Lösung 16 h bei Raumtemperatur gerührt. Die Lösung wird anschließend mit Ethyldüsopropylamin neutralisiert und bis zur Trockene eingeengt. Der Rückstand wird über Kieselgel gereinigt (Dichlormethan/Methanol 50:1->4:1).
Ausbeute: 2,8 g (50% der Theorie)
Massenspektrum (ESI⁺): m/z = 576 [M+NH₄]⁺

Analog Beispiel XII werden folgende Verbindungen erhalten:
(1) 1-Methoxy-4-(1-methoxy-D-glucopyranos-1-yl)-2-(4-triisopropylsilylethinyl-benzyl)-benzol Vorteilhaft wird hierbei das Reaktionsgemisch nur mit einem geringen Überschuss an Methansulfonsäure versetzt. 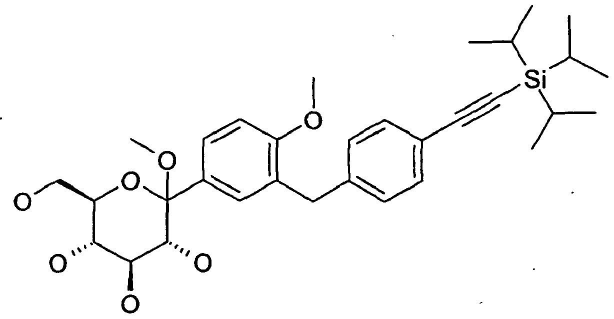 Massenspektrum (ESI⁺): m/z = 588 [M+NH₄]⁺
(2) 1-Chlor-4-(1-methoxy-D-glucopyranos-1-yl)-2-(4-triisopropylsilyleihinyl-benzyl)-benzol 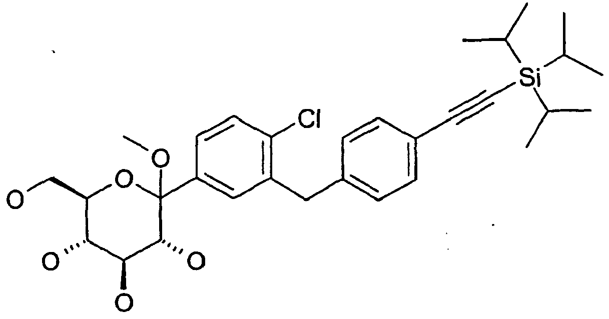
Massenspektrum (ESI⁺): m/z = 592/594 (Cl) [M+NH₄]⁺

### Beispiel XIII

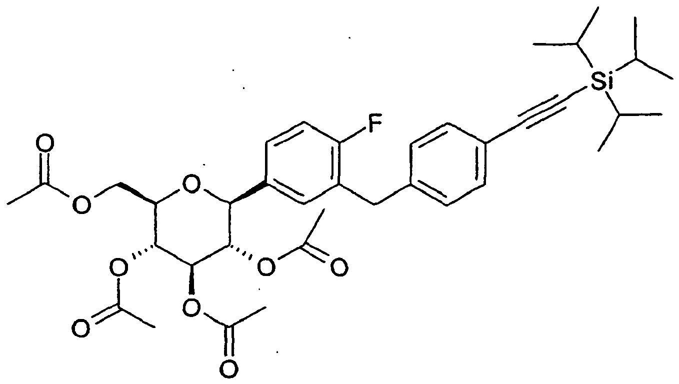

### 1-Fluor-4-(2,3,4,6-tetra-O-acetyl-β-D-glucopyranos-1yl)-2-(4-triisopropylsilylethinyl-benzyl)-benzol

Eine Lösung von 0,8 g 1-Fluor-4-(1-methoxy-D-glucopyranos-1-yl)-2-(4-triisopropylsilylethinyl-benzyl)-benzol und 0,5 ml Triethylsilan in 6 ml Dichlormethan und 10 ml Acetonitril wird auf -10°C abgekühlt. Zu der gekühlten Lösung werden 0,27 ml Bortrifluoridetherat getropft. Die Lösung wird dann 3 h im Eisbad gerührt. Zu der Lösung wird wässrige Natriumhydrogencarbonatlösung gegeben und dann mit Ethylacetat extrahiert. Die organische Phase wird über Natriumsulfat getrocknet, das Lösungsmittel entfernt und der Rückstand in 6 ml Dichlormethan aufgenommen. Dann werden 1,2 ml Pyridin, 1,3 ml Essigsäureanhydrid und 8 mg 4-Dimethylaminopyridin zugegeben. Die Lösung wird 1 h bei Raumtemperatur gerührt und dann mit Wasser versetzt. Das Gemisch wird mit Dichlormethan extrahiert, die organische Phase mit 1 M Salzsäure gewaschen und über Natriumsulfat getrocknet Nach Entfernen des Lösungsmittels wird der Rückstand über Kieselgel chromatografiert (Cyclohexan/Ethylacetat 4:1->1:1).
Ausbeute: 0,23 g (23% der Theorie)
Massenspektrum (ESI⁺): m/z = 714 [M+NH₄]⁺

Analog Beispiel XIII werden folgende Verbindungen erhalten:
(1) 1-Methoxy-4-(2,3,4,6-tetra-O-acetyl-β-D-glucopyranos-1-yl)-2-(4-triisopropylsilylethinyl-benzyl)-benzol 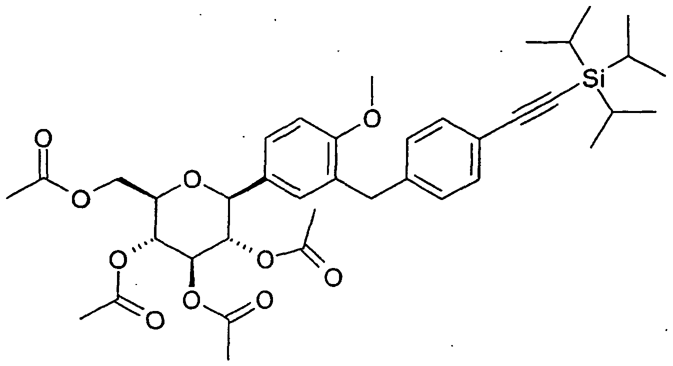 Massenspektrum (ESI⁺): m/z = 726 [M+NH₄]⁺
(2) 1-Chlor-4-(2,3,4,6-tetra-O-acetyl-β-D-glucopyranos-1-yl)-2-(4-triisopropylsilylethinyl-benzyl)-benzol 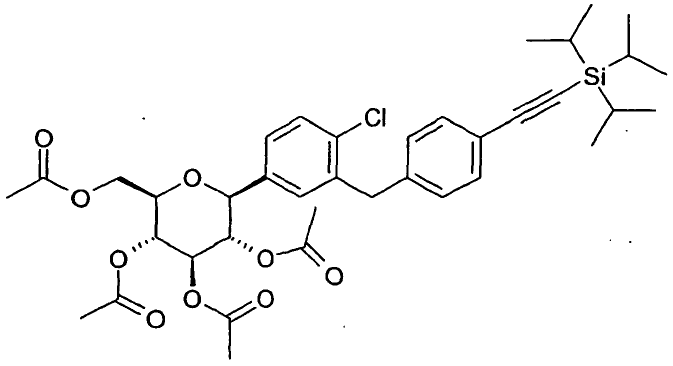
Massenspektrum (ESI⁺): m/z = 730/732 (CI) [M+NH₄]⁺

### Beispiel XIV

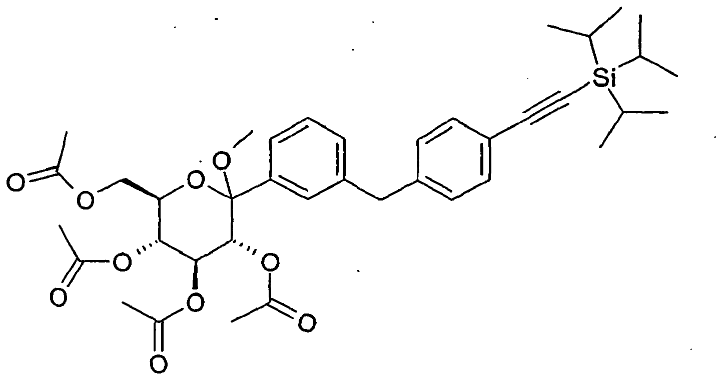

### 1-(2,3,4,6-Tetra-O-acetyl-1-methoxy-D-cllucopyranos-1-yl)-3-(4-triisopropylsilylethinyl-benzyl)-benzol

Eine Lösung von 2,6 g [4-(3-Brom-benzyl)-phenylethinyl]-triisopropyl-silan in 20 ml trockenem Diethylether wird unter Argon auf -80°C abgekühlt. Zu der gekühlten Lösung werden 7,9 ml einer 1,7 M Lösung von tert-Butyllithium in Pentan langsam getropft, und dann wird die Lösung 30 min bei -80°C gerührt. Zu dieser Lösung wird nun über eine Umdrücknadel eine -80°C-kalte Lösung von 3,2 g 2,3,4,6-Tetrakis-O-(trimethylsilyl)-D-glucopyranon in 30 ml Diethylether getropft. Die resultierende Lösung wird 2 h bei -78°C gerührt und danach wird noch einmal eine -80°C-kalte Lösung von 1,0 g g 2,3,4,6-Tetrakis-O-(trimethylsilyl)-D-glucopyranon in 10 ml Diethylether zugetropft. Nach einer weiteren Stunde Rührens bei -78°C wird eine Lösung von 2 ml Methansulfonsäure in 20 ml Methanol zugegeben, das Kühlbad entfernt und die Lösung 16 h bei Raumtemperatur gerührt. Die Lösung wird anschließend mit Ethyldüsopropylamin neutralisiert, das Lösungsmittel vollständig entfernt und der Rückstand in 50 ml Toluol aufgenommen. Es werden 8,5 ml Ethyldiisopropylamin zugesetzt, und die Lösung wird im Eisbad abgekühlt. Danach werden 4,3 ml Essigsäureanhydrid und 0,15 g 4-Dimethylaminopyridin zugegeben. Die Lösung wird 2 h bei Raumtemperatur gerührt und dann mit wässriger Natriumhydrogencarbonatlösung versetzt. Es wird mit Ethylacetat extrahiert, die organischen Phasen werden über Natriumsulfat getrocknet, und das Lösungsmittel wird entfernt. Der Rückstand wird über Kieselgel chromatografiert (Cyclohexan/Ethylacetat 4:1->1:3).
Ausbeute: 2,0 g (46% der Theorie)
Massenspektrum (ESI⁺): m/z = 726 [M+NH₄]⁺

Analog Beispiel XIV wird folgende Verbindung erhalten:
(1) 1-(Triisopropysilylethinyl)-4-(2,3,4,6-tetra-O-acetyl-1-methoxy-D-glucopyranos-1-yl)-2-(4-ethoxy-benzyl)-benzol 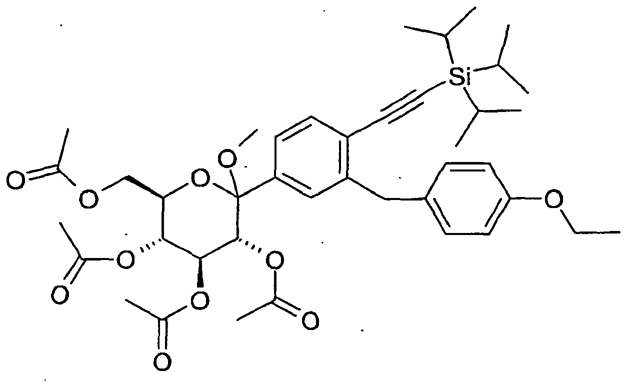
Massenspektrum (ESI⁺): m/z = 770 [M+NH₄]⁺

### Beispiel XV

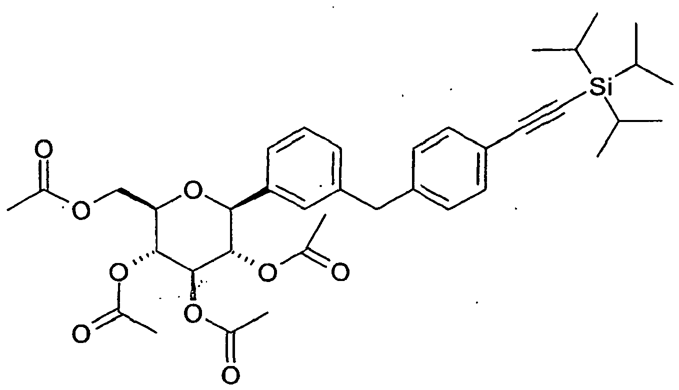

### 1-(2,3,46-Tetra-O-acetyl-β-D-glucopyranos-1-yl)-3-(4-triisopropylsilylethinyl-benzyl)-benzol

Zu einer eisgekühlten Lösung von 1,0 g 1-(2,3,4,6-tetra-O-acetyl-1-methoxy-D-glucopyranos-1-yl)-3-(4-triisopropytsilylethinyl-benzyl)-benzol und 25 µl Wasser in 10 ml Acetonitril werden 1,2 ml Triethylsilan und 0,36 ml Bortrifluoridetherat getropft. Die Lösung wird dann 3 h im Eisbad und 1 h bei Raumtemperatur gerührt. Danach wird die Lösung erneut im Eisbad abgekühlt, und es werden noch einmal 1,2 ml Triethylsilan und 0,36 ml Bortrifluoridetherat zugegeben. Die Lösung wird weitere 0,5 h im Eisbad und 2 h bei Raumtemperatur gerührt. Zu der Lösung wird dann wässrige Natriumhydrogencarbonatlösung gegeben, und die resultierende Lösung wird mit Ethylacetat extrahiert. Die organische Phase wird über Natriumsulfat getrocknet und das Lösungsmittel entfernt.
Ausbeute: 0,78 g (81% der Theorie)
Massenspektrum (ESI⁺): m/z = 696 [M+NH₄]⁺

Analog Beispiel XV wird folgende Verbindung erhalten:
(1) 1-(Triisopropylsilylehinyl)4-(2,3,4,6-tetra-O-acetyl-β-D-glucopyranos-1-yl)-2-(4-ethoxybenzyl)-benzol

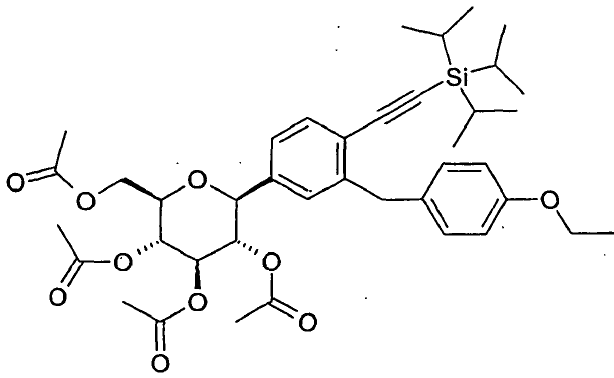

### Beispiel XVI

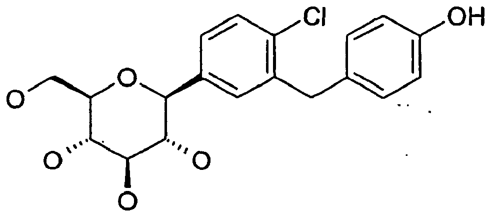

### 1-Chlor-4-(β-D-g lucopyranos-1-yl)-2-(4-hydroxybenzyl)-benzol

Eine Lösung von 4,0 g [4-(5-Brom-2-chlor-benzyl)-phenoxy]-tert-butyl-dimethyl-silan in 42 ml trockenem Diethylether wird unter Argon auf -80°C abgekühlt. Zu der gekühlten Lösung werden 11,6 ml einer 1,7 M Lösung von tert-Butyllithium in Pentan langsam getropft, und dann wird die Lösung 30 min bei -80°C gerührt. Diese Lösung wird nun über eine Umdrücknadel, die mit Trockeneis gekühlt wird, zu einer -80°C-kalten Lösung von 4,78 g 2,3,4,6-Tetrakis-O-(trimethylsilyl)-D-glucopyranon in 38 ml Diethylether getropft. Die resultierende Lösung wird 3 h bei -78°C gerührt. Danach wird eine Lösung von 1,1 ml Methansulfonsäure in 35 ml Methanol zugegeben und die Lösung 16 h bei Raumtemperatur gerührt. Die Lösung wird anschließend mit festem Natriumhydrogencarbonat neutralisiert, es wird Ethylacetat zugegeben und das Methanol zusammen mit dem Ether entfernt. Zur verbleibenden Lösung wird wässrige Natriumhydrogencarbonatlösung gegeben und vier Mal mit Ethylacetat extrahiert. Die organischen Phasen werden über Natriumsulfat getrocknet und eingeengt Der Rückstand wird in 30 ml Acetonitril und 30 ml Dichlormethan gelöst und die Lösung auf -10°C abgekühlt. Nach Zugabe von 4,4 ml Triethylsilan werden 2,6 ml Bortrifluoridetherat so zugetropft, dass die Temperatur nicht über -5°C steigt. Nach vollständiger Zugabe wird die Lösung noch 5 h bei -5 bis -10°C gerührt und anschließend durch Zugabe von wässriger Natriumhydrogencarbonatlösung gequencht. Die organische Phase wird abgetrennt und die wässrige vier Mal mit Ethylacetat extrahiert. Die vereinigten organischen Phase werden über Natriumsulfat getrocknet, das Lösungsmittel wird entfernt und der Rückstand über Kieselgel gereinigt. Das danach erhaltene Produkt ist ein ca. 6:1-ß/a-Gemisch, welches durch vollständige Acetylierung der Hydroxygruppen mit Acetanhydrid und Pyridin in Dichlormethan und Umkristallisieren des Produkts in Ethanol in das reine β-Anomer überführt werden kann. Das so erhaltene Produkt wird durch Umsetzung in Methanol mit 4 M Kalilauge in die Titelverbindung überführt.
Ausbeute: 1,6 g (46% der Theorie)
Massenspektrum (ESI⁺): m/z = 398/400 (CI) [M+H]⁺

### Beispiel XVII

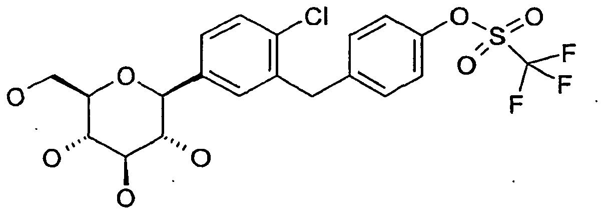

### 1-Chlor-4-(ß-D-glucopyranos-1-yl)-2-[4-(trifluormethylsulfonyloxy)-benzyl]-benzol

Zu einer Lösung von 0,38 g 1-Chlor-4-(β-D-glucopyranos-1-yl)-2-(4-hydroxybenzyl)-benzol, 0, 21 ml Triethylamin und 0,39 g N,N-Bis-(trifluormethansulfonyl)-anilin in 10 ml trockenem Dichlormethan werden 10 mg 4-Dimethylaminopyridin gegeben. Die Lösung wird 4 h bei Raumtemperatur gerührt und dann mit wässriger Natriumchloridlösung versetzt. Es wird mit Ethylacetat extrahiert, die organischen Extrakte werden über Natriumsulfat getrocknet, und das Lösungsmittel wird entfernt. Der Rückstand wird über Kieselgel chromatografiert (Dichlormethan/Methanol 1:0->4:1).
Ausbeute: 0,33 g (64% der Theorie)
Massenspektrum (ESI⁺): m/z = 530/532 (CI) [M+NH₄]⁺

### Beispiel XVIII

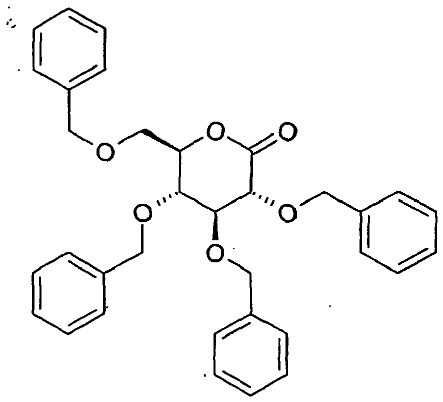

### 2,3,4,6-Tetra-O-benzyl-D-glucopyranon

Zu einer Lösung von 10,0 g 2,3,4,6-Tetra-O-benzyl-α-D-glucopyranose in 140 ml Dichlormethan werden 4 g frisch aktiviertes Molekularsieb 4Ä und 3,3 g N-Methylmorpholin-N-oxid gegeben. Die Lösung wird 20 min bei Raumtemperatur gerührt, bevor 0,3 g Tetrapropylammoniumperrhutenat zugesetzt werden. Nach 2 h Rühren bei Raumtemperatur wird die Lösung mit Dichlormethan verdünnt und über Celite filtriert. Das Filtrat wird mit wässriger Natriumthiosulfatlösung und Wasser gewaschen und dann über Natriumsulfat getrocknet. Nach Entfernen des Lösungsmittels wird der Rückstand über Kieselgel chromatografiert (Cyclohexan/Ethylacetat 4:1).
Ausbeute: 8,2 g (82% der Theorie)
Massenspektrum (ESI⁺): m/z = 539 [M+H]⁺

### Beispiel XIX

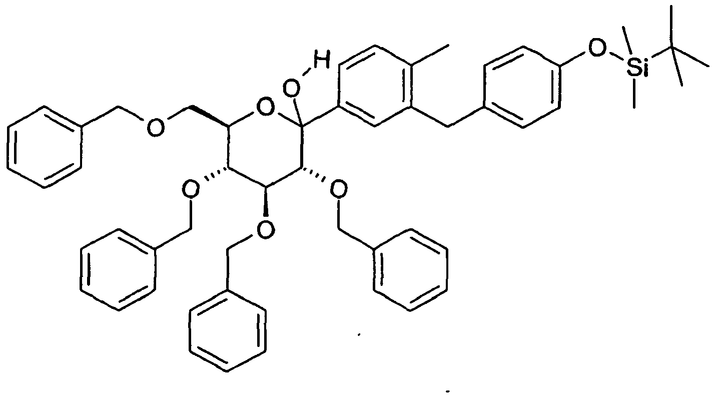

### 1-(2,3,4,6-Tetra-O-benzyl-1-hydro-D-glucopyranos-1-yl)-3-[4-(tert-butyl-dimethyl-silyloxy)-benzyl]-4-methyl-benzol

Eine Lösung von 0,34 g [4-(5-Brom-2-methyl-benzyl)-phenoxy]-tert-butyl-dimethyl-silan in 3 ml trockenem Tetrahydrofuran wird unter Argon auf -80°C abgekühlt. Zu der gekühlten Lösung werden 0,54 ml einer 1,6 M Lösung von n-Butyllithium in Hexan getropft, und die Lösung wird 1,5 h bei -78°C gerührt. Zu dieser Lösung wird mittels Umdrücknadel eine - 80°C-kalte Lösung von 0,43 g 2,3,4,6-Tetra-O-benzyl-D-glucopyranon in 2,5 ml Tetrahydrofuran getropft. Die resultierende Lösung wird 5 h bei -78°C gerührt. Die Reaktion wird mit einer Lösung von 0,1 ml Essigsäure in 1 ml Tetrahydrofuran gequencht und auf Raumtemperatur erwärmt. Nun wird wässrige Natriumhydrogencarbonatlösung zugegeben und vier Mal mit Ethylacetat extrahiert. Die organischen Phasen werden über Natriumsulfat getrocknet und eingeengt. Der Rückstand wird durch Chromatographie auf Kieselgel gereinigt (Cyclohexan/Ethylacetat 15:1->4:1).
Ausbeute: 0,48 g (ca. 88% rein)
Massenspektrum (ESI⁺): m/z = 868 [M+H]⁺

### Beispiel XX

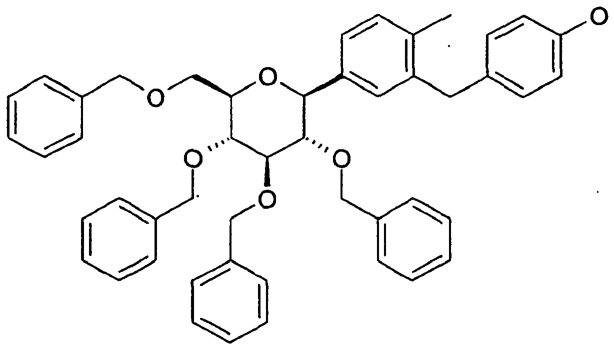

### 1-(2,3,4,6-Tetra-O-benzyl-β-D-glucopyranos-1-yl)-3-(4-hydroxy-benzyl)-4-methyl-benzol

Eine Lösung von 0,48 g (ca. 88% rein) 1-(2,3,4,6-Tetra-O-benzyl-1-hydroxy-D-glucopyranosyl)-3-[4-(tert-butyl-dimethyl-silyloxy)-benzyl]-4-methyl-benzal in 3,5 ml trockenem Acetonitril wird unter Argon auf -40°C abgekühlt. Zur gekühlten Lösung werden 0,13 ml Triisopropylsilan und 0,08 ml Bortrifluoridetherat getropft. Die Lösung wird 3 h bei - 35°C gerührt, bevor noch einmal 0,02 ml Triisopropylsilan und 0,01 ml Bortrifluoridetherat zugegeben werden. Nach weiteren 2 h bei -40°C wird wässriges Kaliumcarbonat zugesetzt und die Lösung 1 h bei Raumtemperatur gerührt. Danach wird mit Wasser verdünnt und vier Mal mit Ethylacetat extrahiert. Die organische Phase wird über Natriumsulfat getrocknet, konzentriert und über Kieselgel chromatografiert (Cyclohexan/Ethylacetat 10:1->4:1). Ausbeute: 0,24 g (68% der Theorie ). Massenspektrum (ESI⁺): m/z = 738 [M+NH₄]⁺

### Beispiel XXI

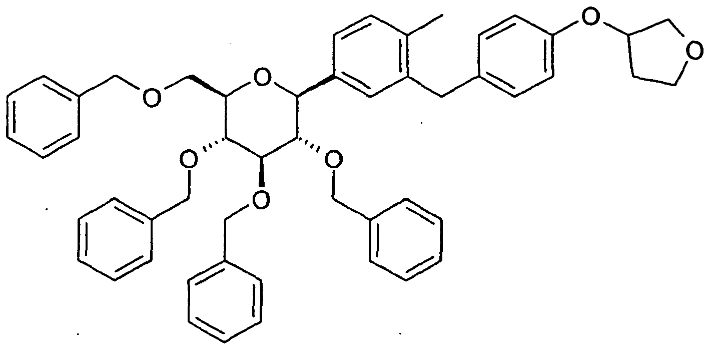

### 1-(2,3,4,6-Tetra-O-benzyl-β-D-glucopyranos-1-yl)-3-[4-(tetrahydrofuran-3-yloxy)-benzyl]-4 methyl-benzol

Zu einem Gemisch von 0,24 g 1-(2,3,4,6-Tetra-O-benzyl-β-D-glucopyranos-1-yl)-3-(4-hydroxy-benzyl)-4-methyl-benzol und 0,13 g Cesiumcarbonat in 2,5 ml Dimethylformamid werden 0,10 g Toluol-4-sulfonsäure-tetrahydrofuran-3-ylester gegeben. Das Gemisch wird 4 h bei 65°C gerührt, bevor Wasser zugesetzt wird. Es wird drei Mal mit Ethylacetat extrahiert, die organische Phase über Natriumsulfat getrocknet und das Lösungsmittel entfernt. Der Rückstand wird über Kieselgel gereinigt (Cyclohexan/Ethylacetat 10:1->4:1).
Ausbeute: 0,23 g (78% der Theorie). Massenspektrum (ESI⁺): m/z = 808 [M+H]⁺

### Beispiel XXII

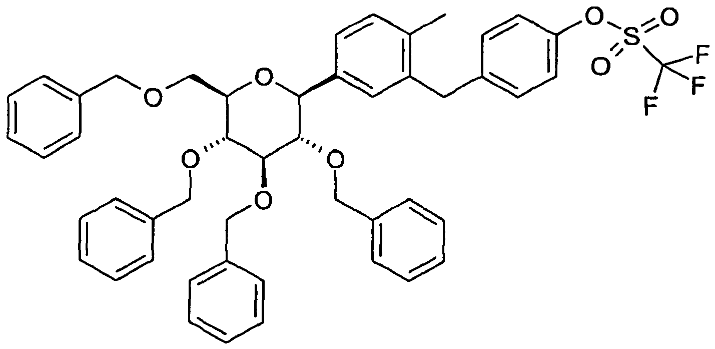

### 1-(2,3,4,6-Tetra-O-benzyl-β-D-glucopyranos-1-yl)-3-[4-(trifluormethylsulfonyloxy)-benzyl]-4-methyl-benzol

Eine Lösung von 0,62 g 1-(2,3,4,6-Tetra-O-benzyl-β-D-glucopyranos-1-yl)-3-(4-hydroxybenzyl)-4-methyl-benzol in 4,5 ml trockenem Dichlormethan wird unter Argon auf -10°C abgekühlt. Zur gekühlten Lösung werden 0,14 ml Pyridin und eine Lösung von 0,3 g Trifluormethansulfonsäureanhydrid in 0,5 ml Dichlormethan gegeben. Die Lösung wird 0,5 h bei -5 bis -10°C gerührt, bevor wässrige Natriumhydrogencarbonatlösung zugegeben wird. Es wird drei Mal mit Dichlormethan extrahiert, die gesammelten organischen Phasen werden mit wässriger 1 M Salzsäure gewaschen und über Natriumsulfat getrocknet. Nach Entfernen des Lösungsmittels wird der Rückstand über Kieselgel chromatografiert (Cyclohexan/Ethylacetat 15:1->7:1).
Ausbeute: 0,62 g (84% der Theorie)
Massenspektrum (ESI⁺): m/z = 853 [M+H]⁺

### Beispiel XXIII

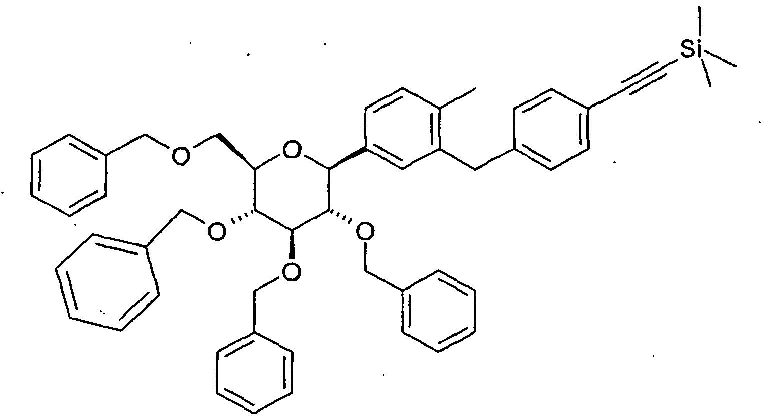

### 1-(2,3,4,6-Tetra-O-benzyl-β-D-glucopyranos-1-yl)-3-[4-(trimethylsilylethinyl)-benzyl]-4-methyl-benzol

Unter Argon werden zu einer Lösung von 0,60 g 1-(2,3,4,6-Tetra-O-benzyl-β-D-glucopyranos-1-yl)-3-[4-(trifluormethylsulfonyloxy)-benzyl]-4-methyl-benzol in 3 ml Dimethylformamid 27 mg Kupferiodid, 49 mg Bis-(triphenyfphosphin)-palladiumdichlorid, 0,30 ml Triethylamin und zuletzt 0,14 ml Trimethylsilylacetylen gegeben. Der Kolben wird dicht verschlossen und 4 h bei 90°C gerührt. Danach werden noch einmal 20 mg Bis-(triphenylphosphin)-palladiumdichlorid und 0,6 ml Trimethylsilylacetylen zugegeben, und die Lösung wird weitere 4 h bei 90°C gerührt. Anschließend wird wässrige Natriumhydrogencarbonatlösung zugesetzt, drei Mal mit Ethylacetat extrahiert, und die gesammelten organischen Phasen werden über Natriumsulfat getrocknet. Nach Entfernen des Lösungsmittels wird der Rückstand über Kieselgel chromatografiert' (Cyclohexan/Ethylacetat 40:1-> 10:1).
Ausbeute: 0,45 g (80% der Theorie)
Massenspektrum (ESl⁺): m/z = 818 [M+NH₄]⁺

### Herstellung der Endverbindungen:

### Beispiel 1

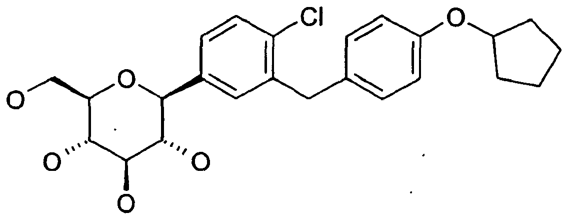

### 1-Chlor-2-(4-cyclopentyloxybenzyl)-4-(β-D-glucopyranos-1-yl)-benzol

Zu einem Gemisch von 0,25 g 1-Chlor-4-(β-D-glucopyranos-1-yl)-2-(4-hydroxybenzyl)-benzol und 0,4 g Cesiumcarbonat in 2,5 ml Dimethylformamid werden 0,16 ml lodcyclopentan gegeben. Das Gemisch wird 4 h bei 45°C gerührt, bevor noch einmal 0,1 g Cesiumcarbonat und 0,05 ml lodcyclopentan zugesetzt werden. Nach weiteren 14 h Rühren bei 45°C wird wässrige Natriumchloridlösung zugegeben und mit Ethylacetat extrahiert. Die organische Phase wird über Natriumsulfat getrocknet, das Lösungsmittel entfernt und der -Rückstand über Kieselgel gereinigt (Dichlormethan/Methanol 1:0->5:1).
Ausbeute: 0,23 g (78% der Theorie)
Massenspektrum (ESI⁺): m/z = 466/468 (CI) [M+NH₄]⁺

Analog Beispiel 1 werden folgende Verbindungen erhalten:
(2) 1-Chlor-4-(ß-D-glucopyranos-1-yl)-2-[4-((*R*)-tetrahydrofuran-3-yloxy)-benzyl]-benzol Die Reaktion wird mit (*S*)-Toluol-4-sulfonsäure-tetrahydrofuran-3-ylester als Kupplungspartner durchgeführt. 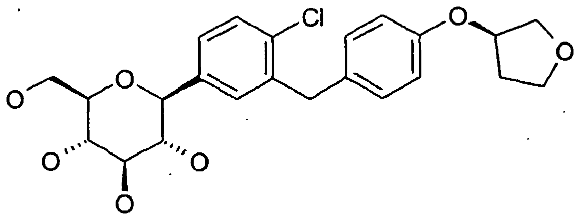 Massenspektrum (ESI⁺): m/z = 451/453 (CI) [M+H]⁺
(3) 1-Chlor-4-(β-D-glucopyranos-1-yl)-2-[4-((*S*)-tetrahydrofuran-3-yloxy)-benzyl]-benzol Die Reaktion wird mit (*R*)-Toluol-4-sulfonsäure-tetrahydrofuran-3-ylester als Kupplungspartner durchgeführt. 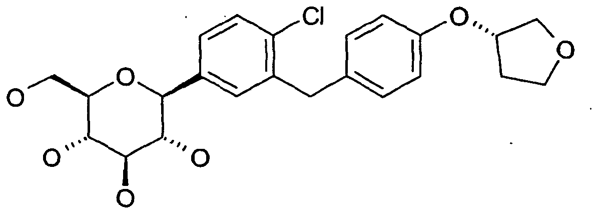 Massenspektrum (ESI⁺): m/z= 451/453 (CI) [M+H]⁺
(4) 1-Chlor-4-(β-D-glucopyranos-1-yl)-2-[4-(tetrahydrofuran-2-on-3-yloxy)-benzyl]-benzol Die Reaktion wird mit 3-Brombutyrolacton als Kupplungspartner durchgeführt. 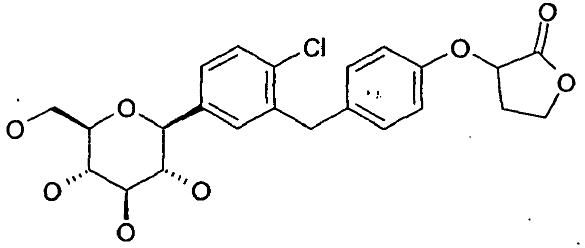 Massenspektrum (ESI⁺): m/z = 465/467 (CI) [M+H] ⁺
(5) 1-Chlor-4-(β-D-glucopyranos-1-yl)-2-(4-cyclobutyloxy-benzyl)-benzol 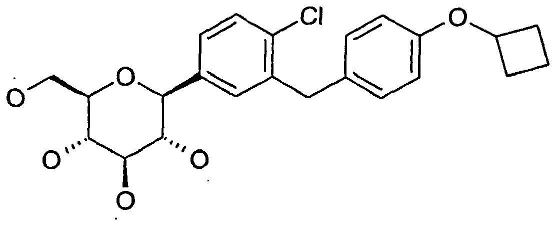 Massenspektrum (ESI⁺): m/z = 452/454 (CI) [M+NH₄]⁺
(6) 1-Chlor-4-(β-O-glucopyranos-1-yl)-2-(4-cyclohexyloxy-benzyl)-benzol 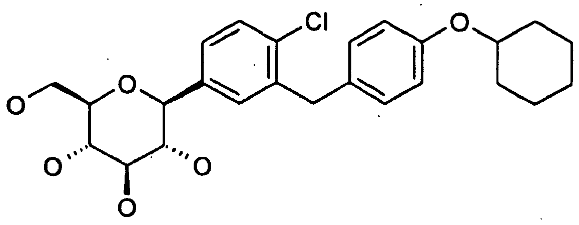 Massenspektrum (ESI⁺): m/z = 480/482 (CI) [M+NH₄]⁺
(7) 1-Chlor-4-(β-D-glucopyranos-1-yl)-2-[4-(tetrahydropyran-4-yloxy)-benzyl]-benzol 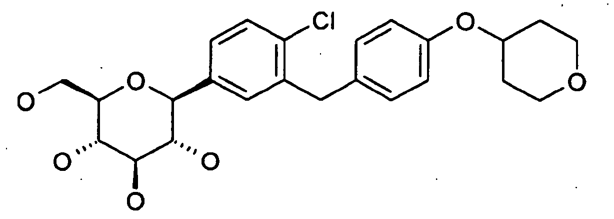 Massenspektrum (ESI⁺): m/z = 487/489 (CI) [M+Na]⁺
(8) 1-Chlor-4-(β-D-glucopyranos-1-yl)-2-[4-(1-acetyl-piperidin-4-yloxy)-benzyl]-benzol Die Umsetzung wird mit 1-Acetyl-4-methylsulfonyloxy-piperidin als Elektrophil durchgeführt. 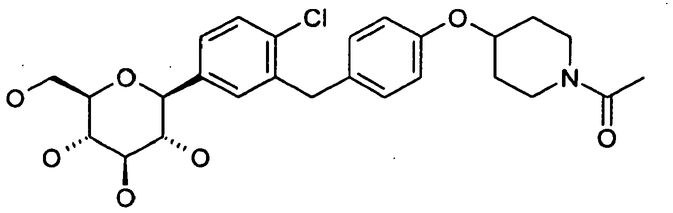 Massenspektrum (ESI⁺): m/z = 506/508 (CI) [M+H]⁺

### Beispiel 10

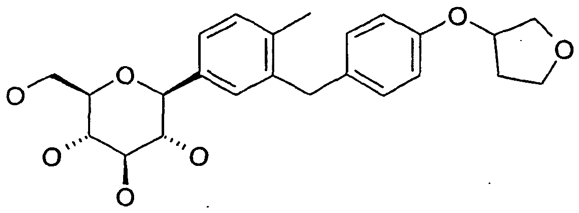

### 1-(β-D-Glucopyranos-1-yl)-4-methyl-3-[4-(tetrahydrofuran-3-yloxy)-benzyl]-benzol

Ein Gemisch von 0,21 g 1-(2,3,4,6-Tetra-O-benzyl-β-D-glucopyranos-1-yl)-3-[4-(tetrahydrofuran-3-ylox)-benzyl]-4-methyl-benzol und 0,1 g 10% Palladiumhydroxid auf Kohle in 3 ml Ethylacetat wird 24 h unter einem Wasserstoffdruck von 1 atm bei Raumtemperatur geschüttelt. Danach wird noch einmal die gleiche Menge Katalysator zugegeben und für weitere 24 h unter Wasserstoffatmosphäre geschüttelt. Dann wird der Katalysator abfiltriert, das Filtrat eingeengt und der Rückstand über Kieselgel chromatografiert (Dichlormethan/Methanol 1:0->5:1).
Ausbeute: 0,06 g (49% der Theorie)
Massenspektrum (ESI⁺): m/z = 448 [M+NH₄]⁺

### Beispiel 11

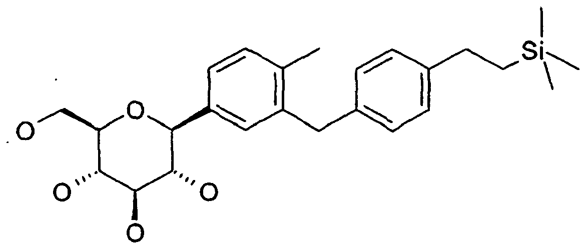

### 1-(β-D-Glucopyranos-1-yl)-4-methyl-3-[4-(2-trimethylsilyl-ethyl)-benzyl]-benzol

Ein Gemisch von 0,29 g 1-(2,3,4,6-tetra-O-benzyl-β-D-glucopyranos-1-yl)-4-methyl-3-[4-(trimethylsilylethinyl)-benzyl]-benzol und 0,25 g 10% Palladiumhydroxid auf Kohle in 3 ml Ethylacetat wird 24 h unter einem Wasserstoffdruck von 1 atm bei Raumtemperatur geschüttelt. Danach werden noch einmal 0,2 g Katalysator zugegeben, und die Lösung wird für weitere 20 h unter Wasserstoffatmosphäre geschüttelt. Dann wird der Katalysator abfiltriert, das Filtrat eingeengt und der Rückstand über Kieselgel chromatografiert (Dichlormethan/Methanol 1:0->5:1).
Ausbeute: 0,08 g (51% der Theorie)
Massenspektrum (ESI⁺): m/z = 462 [M+NH₄]⁺

### Beispiel 12

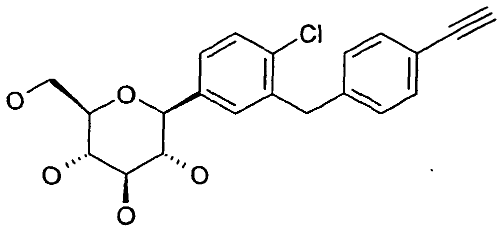

### 1-Chlor-4-(β-D-glucopyranos-1-yl)-2-(4-ethinyl-benzyl)-benzol

Unter Argon werden zu einer Lösung von 0,32 g 1-Chlor-4-(β-D-glucopyranos-1-yl)-2-[4-(trifluormethylsulfonyloxy)-benzyl]-benzol in 3 ml Dimethylformamid 25 mg Kupferiodid, 44 mg Bis-(triphenylphosphin)-palladiumdichlorid, 0,30 ml Triethylamin und zuletzt 0,14 ml Trimethylsilylacetylen gegeben. Der Kolben wird dicht verschlossen und 8 h bei 90°C gerührt. Danach werden noch einmal 25 mg Bis-(triphenylphosphin)-palladiumdichlorid und 0,1 ml Trimethylsilylacetylen zugegeben, und die Lösung wird weitere 10 h bei 90°C gerührt. Anschließend wird wässrige Natriumhydrogencarbonatlösung zugesetzt, drei Mal mit Ethylacetat extrahiert, und die gesammelten organischen Phasen werden über Natriumsulfat getrocknet. Nach Entfernen des Lösungsmittels wird der Rückstand in 5 ml Methanol gelöst und mit 0,12 g Kaliumcarbonat versetzt. Das Gemisch wird 1 h bei Raumtemperatur gerührt und dann mit 1 M Salzsäure neutralisiert. Danach wird das Methanol abgedampft, der Rückstand mit wässriger Natriumchloridlösung versetzt und mit Ethylacetat extrahiert. Die gesammelten organischen Extrakte werden über Natriumsulfat getrocknet, und das Lösungsmittel wird entfernt. Der Rückstand wird über Kieselgel chromatografiert (Dichlormethan/Methanol 1:0->5:1).
Ausbeute: 0,095 g (40% der Theorie)
Massenspektrum (ESI⁺): m/z = 406/408 (CI) [M+NH₄]⁺

Diese Verbindung kann auch gemäß Beispiel 14 erhalten werden.

### Beispiel 13

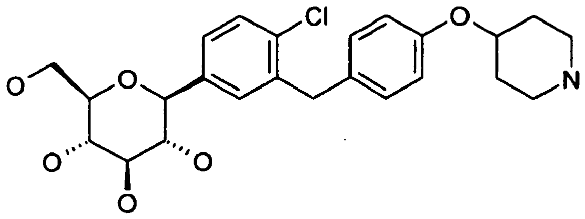

### 1-Chlor-4-(β-D-glucopyranos-1-yl)-2-[4-(piperidin-4-yloxy)-benzyl]-benzol

Zu einer Lösung von 0,19 g 1-Chlor-4-(ß-D-glucopyranos-1-yl)-2-[4-(1-tert-butlyoxycarbonylpiperidin-4-yloxy)-benzyl]-benzol in 4 ml Dichlormethan werden 2 ml Trifluoressigsäure gegeben. Die Lösung wird 1,5 h bei Raumtemperatur gerührt und dann mit Ethylacetat verdünnt und mit wässriger Kaliumcarbonatlösung basisch gestellt. Die organische Phase wird abgetrennt und die wässrige mit Ethylacetat extrahiert. Die vereinten organischen Phasen werden über Natriumsulfat getrocknet und das Lösungsmittels vollständig entfernt.
Ausbeute: 0,060 g (38% der Theorie)
Massenspektrum (ESI⁺): m/z = 464/466 (CI) [M+H]⁺

### Beispiel 14

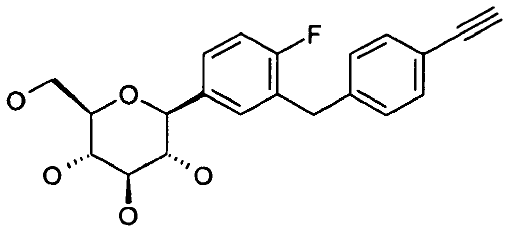

### 1-Fluor-4-(β-glucopyranos-1-yl)-2-(4-ethinyl-benzyl)-benzol

Zu einer Lösung von 0,23 g 1-Fluor-4-(2,3,4,6-tetra-O-acetyl-ß-D-glucopyranos-1-yl)-2-(triisopropylsilylethinyl-benzyl)-benzol in 1,5 ml Tetrahydrofuran werden 0,33 ml einer 1 M Lösung von Tetrabutylammoniumfluorid in Tetrahydrofuran gegeben. Die Lösung wird 1 h bei Raumtemperatur gerührt. Dann werden 1 ml Methanol und 1,5 ml 4 M Kalilauge zugegeben und die Lösung eine weitere Stunde bei Raumtemperatur gerührt. Die Lösung wird mit 1 M Salzsäure neutralisiert und danach das Methanol abgedampft. Der Rückstand wird mit wässriger Natriumchloridlösung versetzt und mit Ethylacetat extrahiert. Die gesammelten organischen Extrakte werden über Natriumsulfat getrocknet, und das Lösungsmittel wird entfernt. Der Rückstand wird über Kieselgel chromatografiert (Dichlormethan/Methanol 19:1->2:1).
Ausbeute: 0,060 g (49% der Theorie)
Massenspektrum (ESI⁺): m/z = 390 [M+NH₄]⁺

Analog Beispiel 14 werden folgende Verbindungen erhalten:
(15) 1-(β-D-Glucopyranos-1-yl)-3-(4-ethinyl-benzyl)-benzol 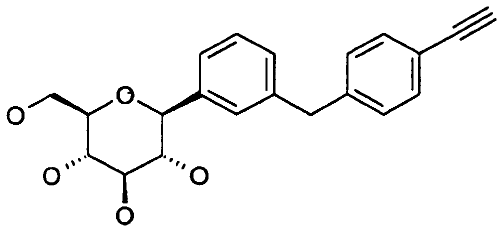 Massenspektrum (ESI⁺): m/z = 372 [M+NH₄]⁺
(17) 1-Methoxy-4-(β-D-glucopyranos-1-yl)-2-(4-ethinyl-benzyl)-benzol 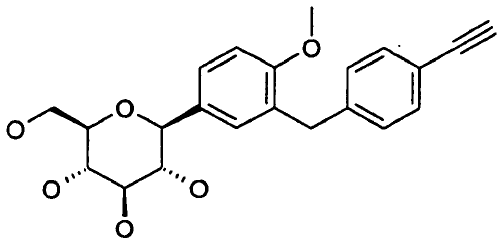 Massenspektrum (ESI⁺): m/z = 402 [M+NH₄]⁺

Die Verbindung gemäß Beispiel (12) (1-Chlor-4-(β-D-glucopyranos-1-yl)-2-(4-ethinyl-benzyl)-benzol) kann auch analog Beispiel 14 synthetisiert werden. Optional kann hierbei die Zwischenstufe, 1-Chlor-4-(2,3,4,6-tetra-O-acteyl-β-D-glucopyranos-1-yl)-2-(4-ethinyl-benzyl)-benzol, die nach der Desilylierung mit Tetrabutylammoniumfluorid erhalten wird, durch Umkristallisieren in Ethanol gereinigt werden. 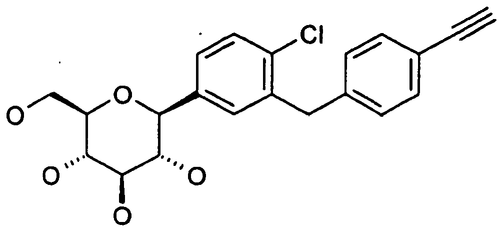 Massenspektrum (ESI⁺): m/z = 406/408 (CI) [M+NH₄]⁺

Analog den vorstehend genannten Beispielen und anderen literaturbekannten Verfahren werden auch folgende Verbindungen hergestellt:

| Bsp. | Struktur | Bsp. | Struktur |
|---|---|---|---|
| (20) | 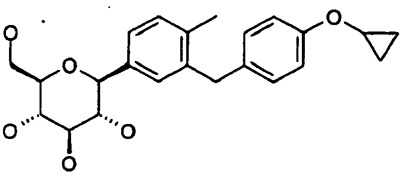 | (21) | 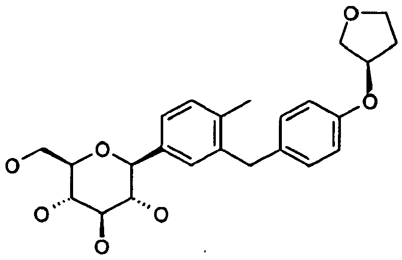 |
| (22) | 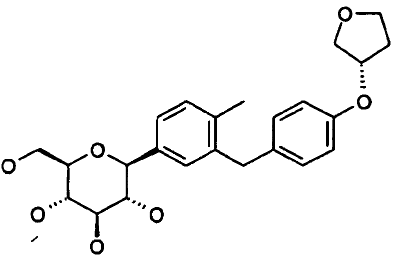 | (23) | 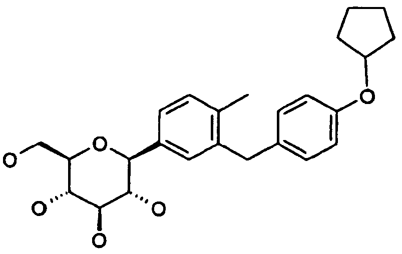 |
| (24) | 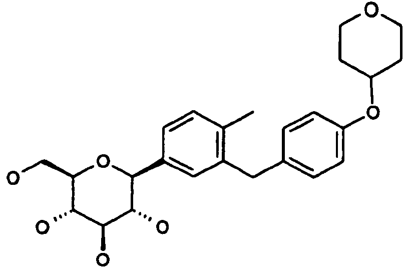 | (25) | 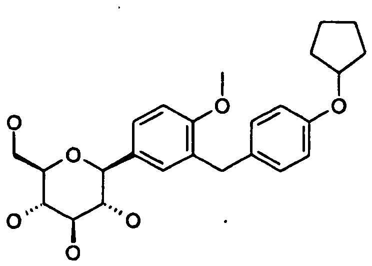 |
| (26) | 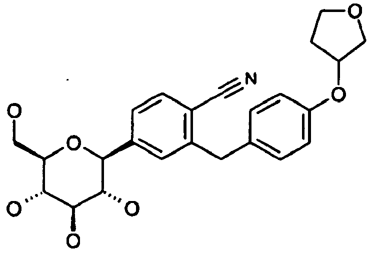 | | |
| (28) | 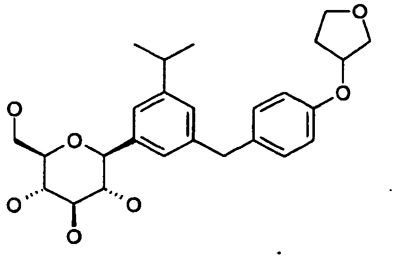 | (29) | 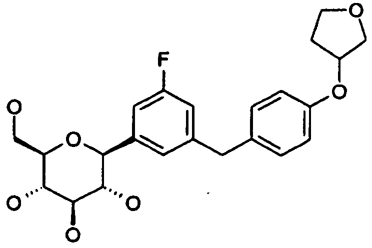 |
| (30) | 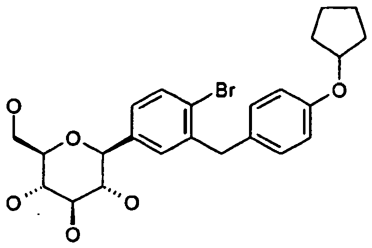 | (31) | 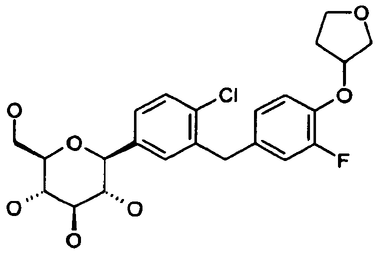 |
| (32) | 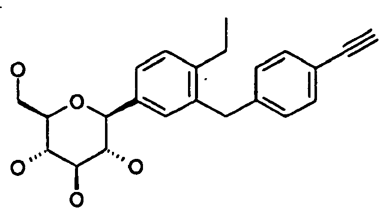 | (33) | 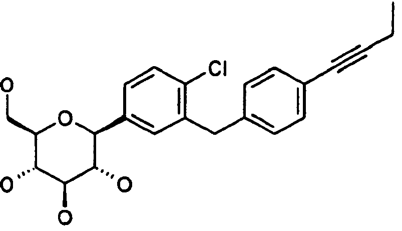 |
| (34) | 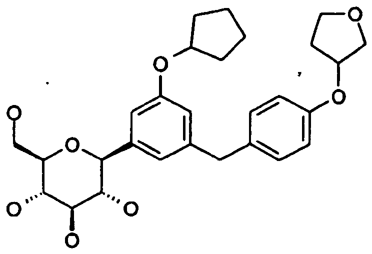 | (35) | 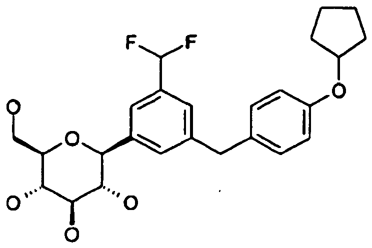 |
| | | (37) | 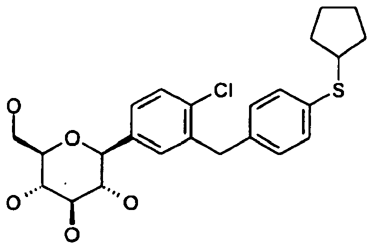 |
| (38) | 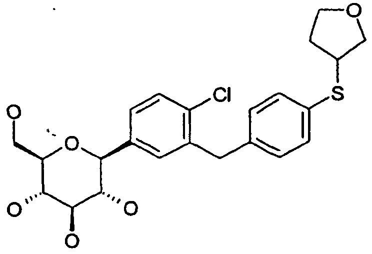 | (39) | 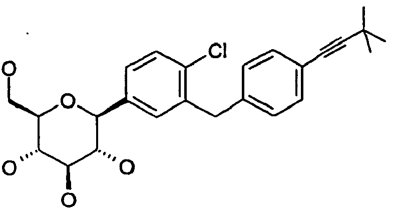 |
| (40) | 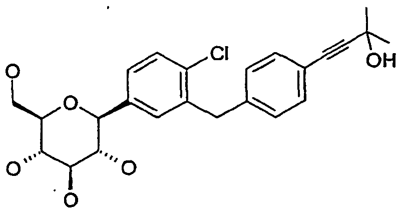 | (41) | 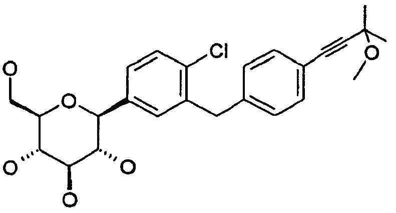 |
| (42) | 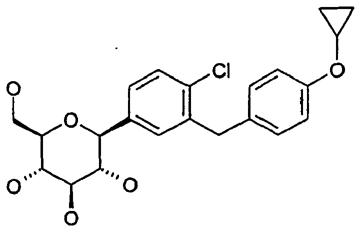 | (43) | 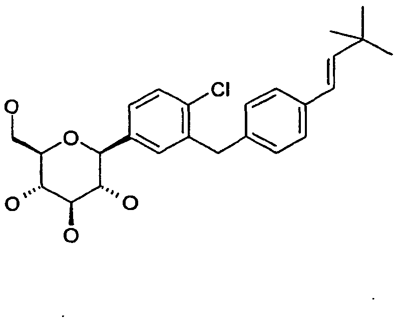 |
| (44) | 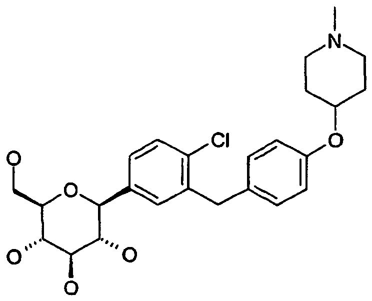 | (45) | 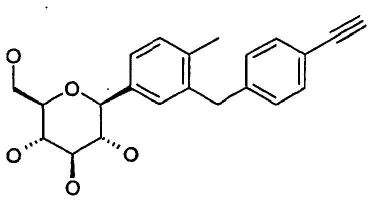 |
| (46) | 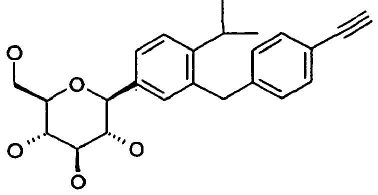 | (47) | 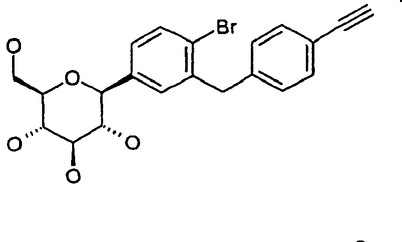 |
| (48) | 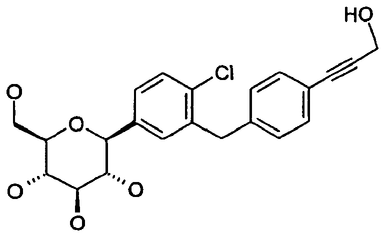 | (49) | 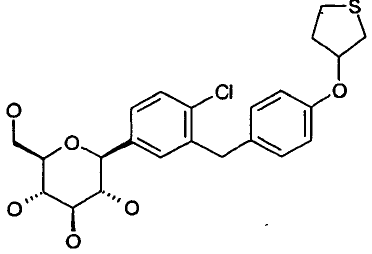 |
| (50) | 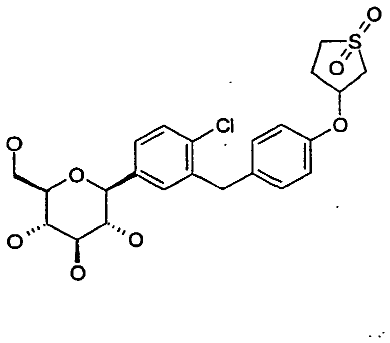 | (51) | 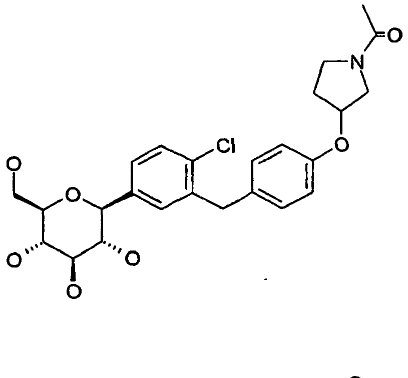 |
| (52) | 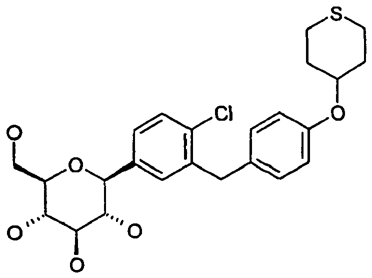 | (53) | 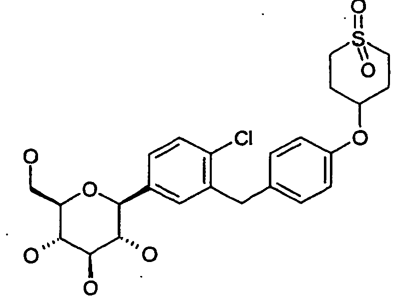 |
| (54) | 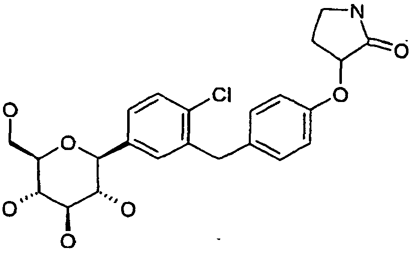 | (55) | 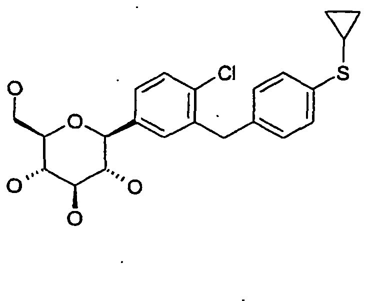 |
| (56) | 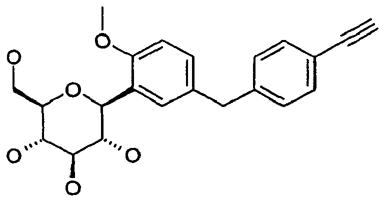 | (57) | 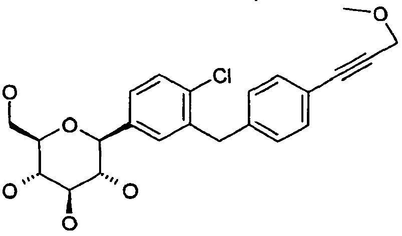 |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

### Tabletten mit 100 mg Wirksubstanz

### Zusammensetzung:

| 1 Tablette enthält: | | |
|---|---|---|
| | Wirksubstanz | 100.0 mg |
| | Milchzucker | 80.0 mg |
| | Maisstärke | 34.0 mg |
| | Polyvinylpyrrolidon | 4.0 mg |
| | Magnesiumstearat | 2.0 mg |
| | | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die pressfertige Mischung wird zu Tabletten verarbeitet.
Tablettengewicht: 220 mg
Durchmesser: 10 mm, biplan mit beidseitiger Facette
   und einseitiger Teilkerbe.

### Beispiel B

### Tabletten mit 150 mg Wirksubstanz

### Zusammensetzung:

| 1 Tablette enthält: | | |
|---|---|---|
| | Wirksubstanz | 150.0 mg |
| | Milchzucker pulv. | 89.0 mg |
| | Maisstärke | 40.0 mg |
| | Kolloide Kieselgelsäure | 10.0 mg |
| | Polyvinylpyrrolidon | 10.0 mg |
| | Magnesiumstearat | 1.0 mg |
| | | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel C

### Hartgelatine-Kapseln mit 150 mg Wirksubstanz

### Zusammensetzung:

| 1 | Kapsel enthält: | | |
|---|---|---|---|
| | Wirkstoff | | 150.0 mg |
| | Maisstärke getr. | ca. | 180.0 mg |
| | Milchzucker pulv. | ca. | 87.0 mg |
| | Magnesiumstearat | | 3.0 mg |
| | ca. | | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.

Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel D

### Suppositorien mit 150 mg Wirksubstanz

### Zusammensetzung:

| | | |
|---|---|---|
| 1 | Zäpfchen enthält: | |
| | Wirkstoff | 150.0 mg |
| | Polyäthylenglykol 1500 | 550.0 mg |
| | Polyäthylenglykol 6000 | 460.0 mg |
| | Polyoxyäthylensorbitanmonostearat | 840.0 mg |
| | | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel E

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCI gelöst, mit Kochsalz isotonisch gestellt, sterilfiltriert und in 2 ml Ampullen abgefüllt.

### Beispiel F

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

## Patentansprüche

1. Glucopyranosyl-substituierte Benzol-Derivate der allgemeinen Formel I 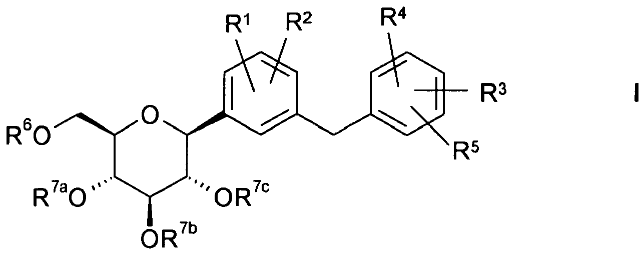 in der
R¹ ausgewählt ist aus den Bedeutungen Wasserstoff, Fluor, Chlor, Brom, lod, C₁₋₄-Alkyl, C₂₋₆-Alkinyl, C₁₋₄-Alkoxy, C₂₋₄-Alkenyl-C₁₋₄-alkoxy, C₂₋₄-Alkinyl-C₁₋₄-alkoxy, durch 1 bis 3 Fluoratome substituiertes Methyl, durch 1 bis 5 Fluoratome substituiertes Ethyl, durch 1 bis 3 Fluoratome substituiertes Methoxy, durch 1 bis 5 Fluoratome substituiertes Ethoxy, durch eine Hydroxy- oder C₁₋₃-Alkoxygruppe substituiertes C₁₋₄-Alkyl, durch eine Hydroxy- oder C₁₋₃-Alkoxygruppe substituiertes C₂₋₄-Alkoxy, C₂₋₆-Alkenyl, C₃₋₆-Cycloalkyl, C₃₋₆-Cycloalkyl-C₁₋₃-alkyl, C₃₋₇-Cycloalkyloxy, C₃₋₆-Cycloalkyl-C₁₋₃-alkoxy, C₅₋₇-Cycloalkenyloxy, Hydroxy, Amino, Nitro oder Cyan, wobei in den C₅₋₆-Cycloalkylgruppen eine Methylengruppe durch O ersetzt sein kann
R² Wasserstoff, Fluor, Chlor, Brom, Hydroxy, C₁₋₄-Alkyl, C₁₋₄-Alkoxy, Cyan oder Nitro bedeutet, wobei die Alkyl- oder Alkoxygruppe ein oder mehrfach mit Fluor substituiert sein kann, und
R³ ausgewählt ist aus den Bedeutungen Tri-(C₁₋₄-alkyl)silyl-C₁₋₆-alkyl, C₂₋₆-Alkin-1-yl, C₂₋₆-Alken-1-yl, Amino, C₁₋₃-Alkylamino, Di-(C₁₋₃-alkyl)amino, Pyrrolidin-1-yl, Pyrrolidin-2-on-1-yl, Piperidin-1-yl, Piperidin-2-on-1-yl, Morpholin-4-yl, Morpholin-3-on-4-yl, Piperazin-1-yl, 4-(C₁₋₃-Alkyl)piperazin-1-yl, Nitro, C₃₋₇-Cycloalkyloxy, C₅₋₇-Cycloalkenyloxy, C₃₋₇-Cycloalkylsulfanyl, C₃₋₇-Cycloalkylsulfinyl, C₃₋₇-Cycloalkylsulfonyl, C₅₋₇-Cycloalkenylsulfanyl, C₅₋₇-Cycloalkenylsulfinyl und C₅₋₇-Cycloalkenylsulfonyl,
wobei die vorstehend genannten Alkinyl- und Alkenylgruppen ein- oder mehrfach mit Fluor oder Chlor, vorzugsweise Fluor, substituiert sein können, und
wobei die vorstehend genannten Alkinyl- und Alkenylgruppen ein- oder zweifach mit gleichen oder verschiedenen Resten L1 substituiert sein können;
wobei die vorstehend genannten Cycloalkyl- und Cycloalkenyl-Ringe unabhängig voneinander ein- oder zweifach mit Substituenten ausgewählt aus Fluor und C₁₋₃-Alkyl substituiert sein können, und
wobei in den vorstehend genannten Cycloalkyl- und Cycloalkenyl-Ringen ein oder zwei Methylengruppen unabhängig voneinander durch O, S, CO, SO, SO₂ oder NR^{N}, vorzugsweise O, CO, S, SO₂ oder NR^{N}, ganz besonders bevorzugt durch O oder CO ersetzt sein können,
R⁴, R⁵ unabhängig voneinander Wasserstoff, Fluor, Chlor, Brom, lod, Cyan, Nitro, C₁₋₃-Alkyl, C₁₋₃-Alkoxy, durch 1 bis 3 Fluoratome substituiertes Methyl- oder Methoxy bedeuten,
R^{N} H, C₁₋₄-Alkyl, C₁₋₄-Alkylcarbonyl oder C₁₋₄-Alkylsulfonyl bedeutet,
L1 unabhängig voneinander ausgewählt sind aus der Gruppe bestehend aus Hydroxy-, Cyan-, Nitro-, C₃₋₇-Cycloalkyl, Aryl-, Heteroaryl-, C₁₋₄-Alkylcarbonyl-, Arylcarbonyl-, Heteroarylcarbonyl-, Aminocarbonyl-, C₁₋₄-Alkylaminocarbonyl-, Di-(C₁-₃-alkyl)aminocarbonyl-, Pyrrolidin-1-ylcarbonyl-, Piperidin-1-ylcarbonyl-, Morpholin-4-ylcarbonyl, Arylaminocarbonyl-, Heteroarylaminocarbonyl-, C₁₋₄-Alkoxycarbonyl-, Aryl-C₁₋₃-alkoxycarbonyl-, Heteroaryl-C₁₋₃-alkoxycarbonyl-, C₁₋₄-Alkyloxy-, Aryloxy-, Heteroaryloxy-, C₁₋₄-Alkylsulfanyl-, Arylsulfanyl-, Heteroarylsulfanyl-, C₁₋₄-Alkylsulfinyl-, Arylsulfinyl-, Heteroarylsulfinyl-, C₁₋₄-Alkylsulfonyl-, Arylsulfonyl- und Heteroarylsulfonyl-; und
L2 unabhängig voneinander ausgewählt sind aus der Gruppe bestehend aus Fluor, Chlor, Brom, lod, C₁₋₃-Alkyl, Difluormethyl, Trifluormethyl, C₁₋₃-Alkoxy, Difluormethoxy, Trifluormethoxy und Cyan; und
R⁶ Wasserstoff, (C₁₋₆-Alkyl)oxycarbonyl oder C₁-₆-Alkylcarbonyl bedeutet,
R^{7a}, R^{7b}, R^{7c} Wasserstoff bedeuten,
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl-, Isochinolinyl- oder Tetrazolylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder lsochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L2 substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

2. Glucopyranosyl-substituierte Benzol-Derivate der allgemeinen Formel 1.2 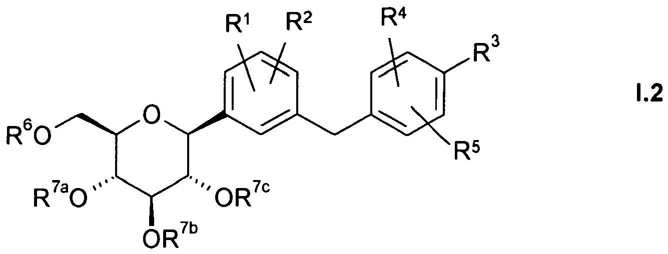 worin die Reste R¹ bis R⁶ und R^{7a}, R^{7b} und R^{7c} wie in Anspruch 1 definiert sind.

3. Glucopyranosyl-substituierte Benzol-Derivate der allgemeinen Formel 1.2c 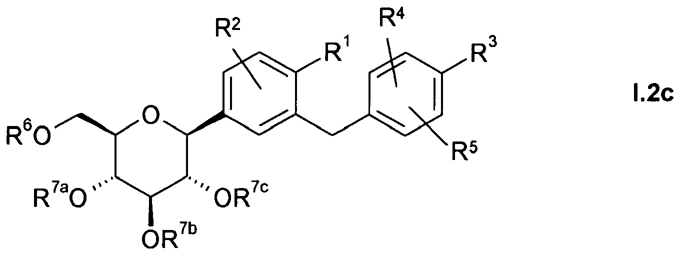 worin die Reste R¹ bis R⁶ und R^{7a}, R^{7b} und R^{7c} wie in Anspruch 1 definiert sind.

4. Glucopyranosyl-substituierte Benzol-Derivate gemäß einem oder mehreren der Ansprüche 1 bis 3, **dadurch gekennzeichnet, dass** die Gruppe R¹ ausgewählt ist aus den Bedeutungen Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy, Cyclohexyloxy.

5. Glucopyranosyl-substituierte Benzol-Derivate gemäß Anspruch 1, 2 oder 3, **dadurch gekennzeichnet, dass** die Gruppe R¹ ausgewählt ist aus den Bedeutungen Methyl und Chlor.

6. Glucopyranosyl-substituierte Benzol-Derivate gemäß einem oder mehreren der Ansprüche 1 bis 5, **dadurch gekennzeichnet, dass** die Gruppe R³ ausgewählt ist aus den Bedeutungen Tri-(C₁₋₄-alkyl)silyl-C₁₋₆-alkyl, C₂₋₆-Alkin-1-yl, C₂₋₆-Alken-1-yl, C₃₋₇-Cycloalkyloxy, C₅₋₇-Cycloalkenyloxy, C₃₋₇-Cycloalkylsulfanyl, C₅₋₇-Cycloalkenylsulfanyl, wobei die vorstehend genannten Alkinyl- und Alkenylgruppen ein- oder mehrfach mit Fluor oder einfach mit Chlor oder dem Rest L1 substituiert sein können, und wobei in den Cycloalkyl- und Cycloalkenylgruppen ein oder zwei Methylengruppen unabhängig voneinander durch O, CO, S, SO₂ oder NR^{N}, insbesondere O oder CO, ersetzt sein können, und
L1 und R^{N} wie in Anspruch 1 definiert sind.

7. Glucopyranosyl-substituierte Benzol-Derivate gemäß einem oder mehreren der Ansprüche 1 bis 5, **dadurch gekennzeichnet, dass** die Gruppe R³ ausgewählt ist aus den Bedeutungen Trimethylsilylethyl, Ethinyl, 1-Propin-1-yl, 1-Butin-1-yl, tert.-Butylethinyl, 2-Hydroxyprop-2-ylethinyl, 2-Methoxyprop-2-ylethinyl, 3-Hydroxy-1-propin-1-yl, 3-Methoxy-1-propin-1-yl, Ethenyl, 1-Propenyl, 1-Butenyl, tert.-Butylethenyl, Cyclopropyloxy, Cyclobutyloxy, Cyclopentyloxy, Cyclohexyloxy, Tetrahydrofuranyloxy, Tetrahydrothiophenyloxy, 1,1-Dioxotetrahydrothiophenyloxy, Tetrahydropyranyloxy, Tetrahydrothiopyranyloxy, 1,1-Dioxotetrahydrothiopyranyloxy, Tetrahydrofuranonyloxy, Piperidinyloxy, Piperidinonyloxy, Pyrrolidin-3-yloxy, Pyrrolidinon-3-yloxy, Tetrahydrofuranyl-sulfanyl, Cyclopropylsulfanyl, Cyclobutylsulfanyl, Cyclopentylsulfanyl und Cyclohexylsulfanyl, wobei die -NH-Gruppe in einem Piperidinyl-, Piperidinonyl-, Pyrrolidinyl- oder Pyrrolidinonyl-Ring mit C₁₋₃-Alkyl oder Acetyl substituiert sein kann.

8. Glucopyranosyl-substituierte Benzol-Derivate gemäß einem oder mehreren der Ansprüche 1 bis 5, **dadurch gekennzeichnet, dass** die Gruppe R³ ausgewählt ist aus den Bedeutungen Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy.

9. Glucopyranosyl-substituierte Benzol-Derivate gemäß einem der Ansprüche 1 bis 5, **dadurch gekennzeichnet, dass** die Gruppe R³ ausgewählt ist aus den Bedeutungen Ethinyl, Trimethylsilylethyl, Cyclobutyloxy, Cyclopentyloxy, Cyclohexyloxy, Tetrahydrofuran-3-yloxy und Tetrahydropyran-4-yloxy.

10. Glucopyranosyl-substituierte Benzol-Derivate gemäß einem der Ansprüche 1 bis 9, **dadurch gekennzeichnet, dass** der Rest R² Wasserstoff, Fluor, Chlor, Brom, Methyl, Hydroxy, Methoxy, Ethoxy, Trifluormethoxy, Cyan, Nitro oder durch 1 bis 3 Fluoratome substituiertes Methyl bedeutet.

11. Glucopyranosyl-substituierte Benzol-Derivate gemäß Anspruch 10, **dadurch gekennzeichnet, dass** der Rest R² Wasserstoff oder Methyl bedeutet.

12. Glucopyranosyl-substituierte Benzol-Derivate gemäß einem der Ansprüche 1 bis 11, **dadurch gekennzeichnet, dass** die Reste R⁴ und/oder R⁵ unabhängig voneinander Wasserstoff oder Fluor bedeuten.

13. Glucopyranosyl-substituierte Benzol-Derivate gemäß Anspruch 12, **dadurch gekennzeichnet, dass** der Rest R⁴ Wasserstoff bedeutet.

14. Glucopyranosyl-substituierte Benzol-Derivate gemäß Anspruch 12, **dadurch gekennzeichnet, dass** der Rest R⁵ Wasserstoff bedeutet.

15. Glucopyranosyl-substituierte Benzol-Derivate gemäß einem der Ansprüche 1 bis 13, **dadurch gekennzeichnet, dass** der Rest R⁶ Wasserstoff bedeutet.

16. Glucopyranosyl-substituierte Benzol-Derivate gemäß einem der Ansprüche 1, 2, 3, 4 oder 10, **dadurch gekennzeichnet, dass** der Rest R^{N} H, Methyl, Ethyl oder Acetyl bedeutet.

17. Glucopyranosyl-substituierte Benzol-Derivate gemäß einem oder mehreren der Ansprüche 1 bis 16, **dadurch gekennzeichnet, dass** der Rest L1 ausgewählt ist aus der Gruppe bestehend aus Hydroxy-, Cyan-, C₃₋₆-Cycloalkyl, C₁₋₄-Alkylcarbonyl-, Aminocarbonyl-, C₁₋₄-Alkylaminocarbonyl-, Di-(C₁₋₃-alkyl)aminocarbonyl-, Pyrrolidin-1-ylcarbonyl-, Piperidin-1-ylcarbonyl-, Morpholin-4-ylcarbonyl, C₁₋₄-Alkoxycarbonyl-, C₁₋₄-Alkyloxy-, C₁₋₄-Alkylsulfanyl-, C₁₋₄-Alkylsulfinyl-, und C₁₋₄-Alkylsulfonyl-.

18. Glucopyranosyl-substituierte Benzol-Derivate gemäß einem oder mehreren der Ansprüche 1 bis 16, **dadurch gekennzeichnet, dass** der Rest L1 ausgewählt ist aus der Gruppe bestehend aus Hydroxy-, C₁₋₄-Alkyloxy- und C₁₋₄-Alkylsulfanyl-.

19. Glucopyranosyl-substituierte Benzol-Derivate gemäß einem oder mehreren der Ansprüche 1 bis 18 ausgewählt aus der Gruppe der Verbindungen (1) bis (8), (10) bis (15) und (17):
(1) 1-Chlor-2-(4-cyclopentyloxybenzyl)-4-(β-D-glucopyranos-1-yl)-benzol
(2) 1-Chlor-4-(β-D-glucopyranos-1-yl)-2-[4-((*R*)-tetrahydrofuran-3-yloxy)-benzyl]-benzol
(3) 1-Chlor-4-(β-D-glucopyranos-1-yl)-2-[4-((*S*)-tetrahydrofuran-3-yloxy)-benzyl]-benzol
(4) 1-Chlor-4-(β-D-glucopyranos-1-yl)-2-[4-(tetrahydrofuran-2-on-3-yloxy)-benzyl]-benzol
(5) 1-Chlor-4-(β-D-glucopyranos-1-yl)-2-(4-cyclobutyloxy-benzyl)-benzol
(6) 1-Chlor-4-(β-D-glucopyranos-1-yl)-2-(4-cyclohexyloxy-benzyl)-benzol
(7) 1-Chlor-4-(β-D-glucopyranos-1-yl)-2-[4-(tetrahydropyran-4-yloxy)-benzyl]-benzol
(8) 1-Chlor-4-(β-D-glucopyranos-1-yl)-2-[4-(1-acetyl-piperidin-4-yloxy)-benzyl]-benzol
(10) 1-(β-D-Glucopyranos-1-yl)-4-methyl-3-[4-(tetrahydrofuran-3-yloxy)-benzyl]-benzol
(11) 1-(β-D-Glucopyranos-1-yl)-4-methyl-3-[4-(2-trimethylsilyl-ethyl)-benzyl]-benzol
(12) 1-Chlor-4-(β-D-glucopyranos-1-yl)-2-(4-ethinyl-benzyl)-benzol
(13) 1-Chlor-4-(β-D-glucopyranos-1-yl)-2-[4-(piperidin-4-yloxy)-benzyl]-benzol
(14) 1-Fluor-4-(β-D-glucopyranos-1-yl)-2-(4-ethinyl-benzyl)-benzol
(15) 1-(β-D-Glucopyranos-1-yl)-3-(4-ethinyl-benzyl)-benzol
(17) 1-Methoxy-4-(β-D-glucopyranos-1-yl)-2-(4-ethinyl-benzyl)-benzol

20. Glucopyranosyl-substituiertes Benzol-Derivat gemäß Anspruch 19, **dadurch gekennzeichnet, daß** das Glucopyranosyl-substituierte Benzol-Derivat die Verbindung (2) ist:
(2) 1-Chlor-4-(β-D-glucopyranos-1-yl)-2-[4-((*R*)-tetrahydrofuran-3-yloxy)-benzyl]-benzol,

21. Glucopyranosyl-substituiertes Benzol-Derivat gemäß Anspruch 19, **dadurch gekennzeichnet, daß** das Glucopyranosyl-substituierte Benzol-Derivat die Verbindung (3) ist:
(3) 1-Chlor-4-(β-D-glucopyranos-1-yl)-2-[4-((*S*)-tetrahydrofuran-3-yloxy)-benzyl]-benzol.

22. Physiologisch verträgliche Salze der Verbindungen nach mindestens einem der Ansprüche 1 bis 21 mit anorganischen oder organischen Säuren.

23. Arzneimittel, enthaltend eine Verbindung nach mindestens einem der Ansprüche 1 bis 21 oder ein physiologisch verträgliches Salz gemäß Anspruch 22 neben gegebenenfalls einem oder mehreren inerten Trägerstoffen und/oder Verdünnungsmitteln.

24. Verwendung mindestens einer Verbindung nach mindestens einem der Ansprüche 1 bis 21 oder eines physiologisch verträglichen Salzes gemäß Anspruch 22 zur Herstellung eines Arzneimittels, das zur Behandlung oder Vorbeugung von Stoffwechselerkrankungen ausgewählt aus der Gruppe bestehend aus Diabetes mellitus Typ 1 und Typ 2, diabetische Komplikationen, metabolische Azidose oder Ketose, reaktiver Hypoglykämie, Hyperinsulinämie, Glukosestoffwechselstörung, Insulinresistenz, Metabolischem Syndrom, Dyslipidämien unterschiedlichster Genese, Atherosklerose und verwandte Erkrankungen, Adipositas, Bluthochdruck, chronisches Herzversagen, Ödeme, Hyperurikämie geeignet ist.

25. Verwendung nach Anspruch 24, **dadurch gekennzeichnet, dass** die Stoffwechselerkrankung ausgewählt ist aus der Gruppe bestehend aus Diabetes mellitus Typ 1 und Typ 2 und diabetische Komplikationen.

26. Verwendung mindestens einer Verbindung nach mindestens einem der Ansprüche 1 bis 21 oder eines physiologisch verträglichen Salzes gemäß Anspruch 22 zur Herstellung eines Arzneimittels zur Inhibition des natriumabhängigen Glucose-Cotransporter SGLT2.

27. Verwendung mindestens einer Verbindung nach mindestens einem der Ansprüche 1 bis 21 oder eines physiologisch verträglichen Salzes gemäß Anspruch 22 zur Herstellung eines Arzneimittels zum Verhindern der Degeneration von pankreatischen beta-Zellen und/oder zum Verbessern und/oder Wederherstellen der Funktionalität von pankreatischen beta-Zellen.

28. Verwendung mindestens einer Verbindung nach mindestens einem der Ansprüche 1 bis 21 oder eines physiologisch verträglichen Salzes gemäß Anspruch 22 zur Herstellung von Diuretika und/oder Antihypertensiva.

29. Verfahren zur Herstellung der Verbindungen der allgemeinen Formel I gemäß den Ansprüchen 1 bis 21, **dadurch gekennzeichnet, dass** eine Verbindung der allgemeinen Formel 11 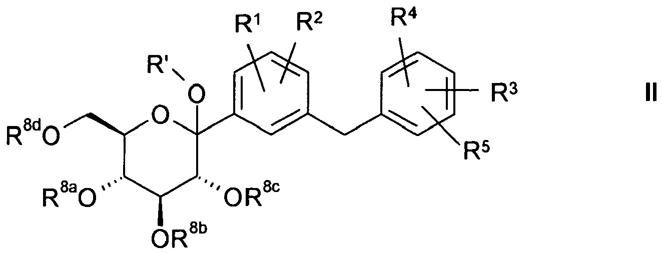 in der
R' H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
R^{8a} , R^{8b}, R^{8c}, R^{8d} unabhängig voneinander ausgewählt sind aus der Gruppe Wasserstoff, (C₁₋₁₈-Alkyl)carbonyl, (C₁₋₁₈-Alkyl)oxycarbonyl, Arylcarbonyl und Aryl-(C₁-₃-alkyl)-carbonyl, oder eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁-₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵ die in den Ansprüchen 1 bis 21 angegebenen Bedeutungen besitzen, mit einem Reduktionsmittel in Gegenwart einer Lewis- oder Brønsted-Säure umgesetzt wird, wobei die eventuell vorhandenen Schutzgruppen gleichzeitig oder nachträglich abgespalten werden;
gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre physiologisch verträglichen Salze überführt wird.

30. Verfahren gemäß Anspruch 29, **dadurch gekennzeichnet, dass** die Verbindung der allgemeinen Formel II gemäß dem in Anspruch 31 oder 32 beschriebenen Verfahren erhalten wird.

31. Verfahren zur Herstellung von Verbindungen der allgemeinen Formel 11 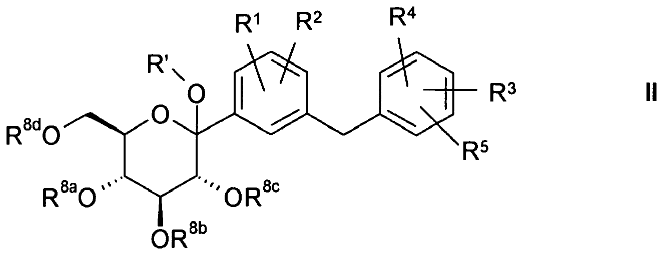 in der
R' H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
R^{8a}, R^{8b}, R^{8c}, R^{8d} unabhängig voneinander ausgewählt sind aus der Gruppe Wasserstoff, (C₁₋₁₈-Alkyl)carbonyl, (C₁₋₁₈-Alkyl)oxycarbonyl, Arylcarbonyl und Aryl-(C₁-₃-alkyl)-carbonyl, oder eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵ die in den Ansprüchen 1 bis 21 angegebenen Bedeutungen besitzen,
bei dem eine Organometall-Verbindung (V), die durch Halogen-Metall-Austausch oder durch Insertion eines Metalls in die Kohlenstoff-Halogen-Bindung einer Halogen-Benzylbenzol-Verbindung der allgemeinen Formel IV 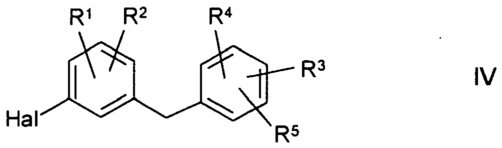 in der Hal Cl, Br und I bedeutet und R¹ bis R⁵ wie zuvor definiert sind, und gegebenenfalls anschließender Ummetallierung erhältlich ist, an ein Gluconolacton der allgemeinen Formel VI 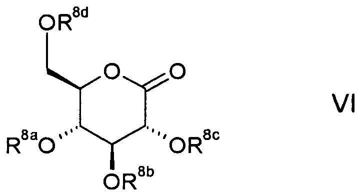 in der R^{8a}, R^{8b}, R^{8c}, R^{8d} wie zuvor definiert sind,
addiert wird, und
anschließend das erhaltene Addukt mit Wasser oder einem Alkohol R'-OH, wobei R' gegebenenfalls substituiertes C₁₋₄-Alkyl bedeutet, in Gegenwart einer Säure umgesetzt wird und optional das in der Umsetzung mit Wasser erhaltene Produkt, in dem R' H bedeutet, in einer nachfolgenden Reaktion mit einem Acylierungsmittel in das Produkt der Formel II überführt wird, worin R' (C₁₋₁₈-Alkyl)carbonyl, (C₁₋₁₈-Alkyl)oxycarbonyl, Arylcarbonyl oder Aryl-(C₁₋₃-alkyl)-carbonyl, das wie angegeben substituiert sein kann, bedeutet.

32. Verfahren gemäß Anspruch 31, **dadurch gekennzeichnet, dass** die Organometall-Verbindung (V) eine Lithium- oder Magnesium-Verbindung ist.

33. Verfahren zur Herstellung der Verbindungen der allgemeinen Formel I gemäß den Ansprüchen 1 bis 21, in der R⁶ Wasserstoff bedeutet, **dadurch gekennzeichnet, dass** eine Verbindung der allgemeinen Formel III 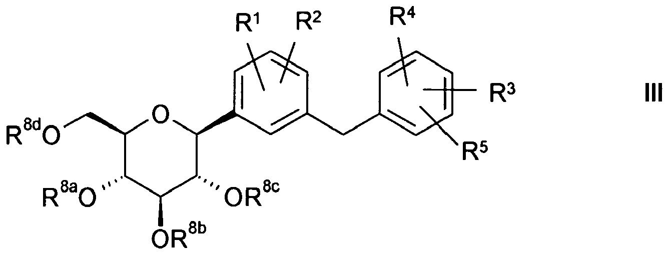 in der
R^{8a}, R^{8b}, R^{8c}, R^{8d} unabhängig voneinander ausgewählt sind aus der Gruppe Wasserstoff, (C₁₋₁₈-Alkyl)carbonyl, (C₁₋₁₈-Alkyl)oxycarbonyl, Arylcarbonyl und Aryl-(C₁-3-alkyl)-carbonyl, jedoch mindestens einer der Reste R^{8a}, R^{8b}, R^{8c}, R^{8d} nicht Wasserstoff bedeutet, oder eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketaloder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵ die in den Ansprüchen 1 bis 21 angegebenen Bedeutungen besitzen,
hydrolysiert wird, und
gewünschtenfalls eine so erhaltene Verbindung der allgemeinen Formel I, in der R⁶ ein Wasserstoffatom darstellt, mittels Acylierung in eine entsprechende Acylverbindung der allgemeinen Formel I übergeführt wird, und/oder
erforderlichenfalls ein bei den vorstehend beschriebenen Umsetzungen verwendeter Schutzrest wieder abgespalten wird und/oder
gewünschtenfalls eine so erhaltene Verbindung der allgemeinen Formel I in ihre Stereoisomere aufgetrennt wird und/oder
gewünschtenfalls eine so erhaltene Verbindung der allgemeinen Formel I in ihre Salze, insbesondere für die pharmazeutische Anwendung in ihre physiologisch verträglichen Salze, überführt wird.

34. Verfahren gemäß Anspruch 33, **dadurch gekennzeichnet, dass** die Verbindung der Formel III mittels eines Verfahrens gemäß Anspruch 29 oder 30 erhalten wird.

35. Verbindung der allgemeinen Formel IV 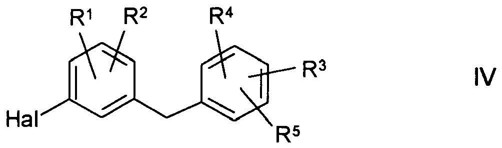 in der Hal Chlor, Brom oder lod bedeutet und
R¹ Wasserstoff, Fluor, Chlor, Brom, Cyan, Methyl, Ethyl, Isopropyl, Difluormethyl, Trifluormethyl, Ethinyl, Prop-1-in-1-yl, But-1-in-1-yl, Hydroxy, Methoxy, Ethoxy, Difluormethoxy, Cyclopropyloxy, Cyclobutyloxy, Cyclopentyloxy oder Cyclohexyloxy bedeutet; und
R² Wasserstoff oder Methyl bedeutet; und
R³ ausgewählt ist aus Trimethylsilylethyl, Ethinyl, 2-Hydroxyprop-2-ylethinyl, 2-Methoxyprop-2-ylethinyl, 3-Hydroxy-1-propin-1-yl, 3-Methoxy-1-propin-1-yl, Cyclopropyloxy, Cyclobutyloxy, Cyclopentyloxy, Cyclohexyloxy, Tetrahydrofuran-3-yloxy, Tetrahydropyran-4-yloxy, Piperidin-4-yloxy, N-Methylpiperidin-4-yloxy und N-Acetylpiperidin-4-yloxy; und
R⁴ Wasserstoff oder Fluor bedeutet; und
R⁵ Wasserstoff oder Fluor bedeutet.

36. Verbindung der Formel IV gemäß Anspruch 35 **gekennzeichnet durch** die Formel 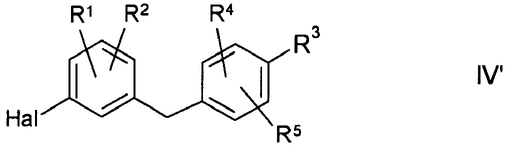 in der Hal Chlor, Brom oder lod bedeutet und die Reste R¹, R², R³, R⁴ und R⁵ wie in Anspruch 35 definiert sind.

37. Verbindung der allgemeinen Formel II 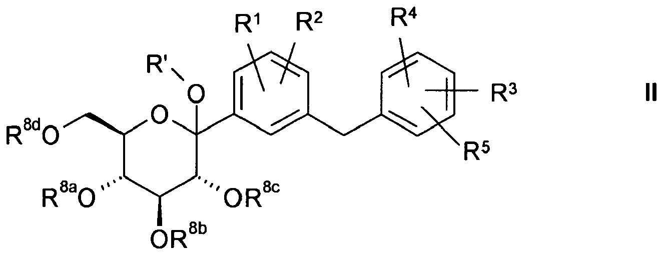 in der
R' H, C₁₋₄-Alkyl, (C₁₋₁₈-Alkyl)carbonyl, (C₁₋₁₈-Alkyl)oxycarbonyl, Arylcarbonyl oder Aryl-(C₁₋₃-alkyl)-carbonyl bedeutet, worin die Alkyl- oder Arylgruppen ein- oder mehrfach mit Halogen substituiert sein können;
R^{8a}, R^{8b} , R^{8c}, R^{8d} unabhängig voneinander ausgewählt sind aus der Gruppe Wasserstoff, (C₁₋₁₈-Alkyl)carbonyl, (C₁₋₁₈-Alkyl)oxycarbonyl, Arylcarbonyl und Aryl-(C₁₋₃- alkyl)-carbonyl, oder eine Benzyl-Gruppe oder eine R^{a}R^{b}R^{c}Si-Gruppe oder eine Ketal- oder Acetalgruppe bedeutet, wobei jeweils zwei benachbarte Reste R^{8a}, R^{8b}, R^{8c}, R^{8d} eine cyclische Ketal- oder Acetalgruppe oder eine 1,2-Di(C₁₋₃-alkoxy)-1,2-di(C₁₋₃-alkyl)-ethylen-Brücke bilden können, wobei die genannte Ethylen-Brücke zusammen mit zwei Sauerstoffatomen und den zugehörigen beiden Kohlenstoffatomen des Pyranose-Rings einen substituierten Dioxan-Ring bildet, und wobei Alkyl-, Aryl- und/oder Benzylgruppen ein- oder mehrfach mit Halogen oder C₁₋₃-Alkoxy substituiert sein können und Benzyl-Gruppen auch mit einer Di-(C₁₋₃-alkyl)amino-Gruppe substituiert sein können; und
R^{a}, R^{b}, R^{c} unabhängig voneinander C₁₋₄-Alkyl, Aryl oder Aryl-C₁₋₃-alkyl bedeuten, wobei die Alkyl- oder Arylgruppen ein- oder mehrfach mit Halogen substituiert sein können;
wobei unter den bei der Definition der vorstehend genannten Reste erwähnten Arylgruppen Phenyl- oder Naphthylgruppen, vorzugsweise Phenylgruppen zu verstehen sind;
und R¹ bis R⁵ wie in einem oder mehreren der Ansprüche 1 und 4 bis 14 definiert sind.

38. Glucopyranosyl-substituierte Benzol-Derivate der allgemeinen Formel I 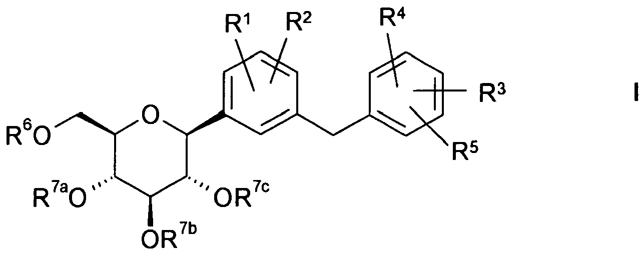 in der R¹ bis R⁵ wie in einem oder mehreren der Ansprüche 1 bis 21 definiert sind und R⁶, R^{7a},
R^{7b}, R^{7c} unabhängig voneinander eine Bedeutung ausgewählt aus der Gruppe (C₁-₁₈-Alkyl)carbonyl, (C₁₋₁₈-Alkyl)oxycarbonyl, Arylcarbonyl und Aryl-(C₁₋₃-alkyl)-carbonyl besitzen,
L2 unabhängig voneinander ausgewählt aus der Gruppe bestehend aus Fluor, Chlor, Brom, lod, C₁₋₃-Alkyl, Difluormethyl, Trifluormethyl, C₁₋₃-Alkoxy, Difluormethoxy, Trifluormethoxy und Cyan; und
wobei unter den bei der Definition der vorstehend genannten Reste erwähnten Arylgruppen Phenyl- oder Naphthylgruppen zu verstehen sind, welche unabhängig voneinander ein- oder zweifach mit gleichen oder verschiedenen Resten L2 substituiert sein können; und
wobei, soweit nichts anderes erwähnt wurde, die vorstehend erwähnten Alkylgruppen geradkettig oder verzweigt sein können,
deren Tautomere, deren Stereoisomere, deren Gemische und deren Salze.

39. Glucopyranosyl-substituierte Benzol-Derivate gemäß Anspruch 38 **gekennzeichnet durch** die Formel 1.2 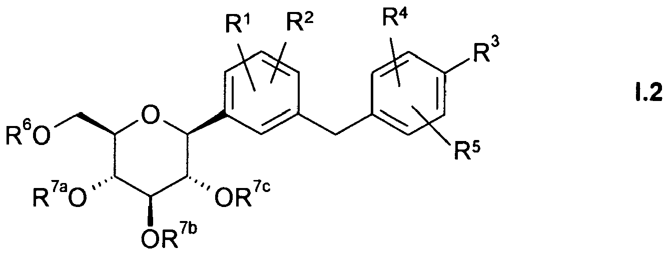 in der R¹ bis R⁵ und R⁶, R^{7a}, R^{7b}, R^{7c} wie in Anspruch 38 definiert sind.

40. Glucopyranosyl-substituierte Benzol-Derivate gemäß Anspruch 38 **gekennzeichnet durch** die Formel 1.2c 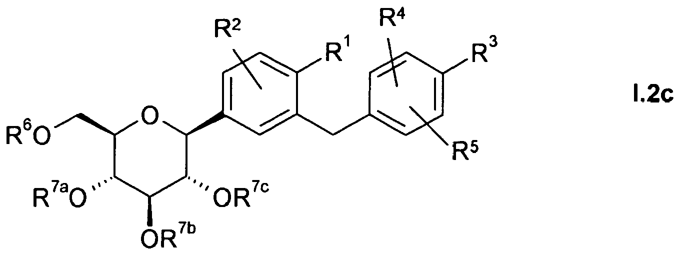 in der R¹ bis R⁵ und R⁶, R^{7a}, R^{7b}, R^{7c} wie in Anspruch 38 definiert sind.

41. Arzneimittel, das eine Verbindung gemäß einem oder mehreren der Ansprüche 1 bis 21 oder ein physiologisch verträgliches Salz solch einer Verbindung sowie mindestens einen Wirkstoff ausgewählt aus der Gruppe der Antidiabetika neben gegebenenfalls einem oder mehreren inerten Trägerstoffen und/oder Verdünnungsmitteln aufweist.

42. Arzneimittel, das eine Verbindung gemäß einem oder mehreren der Ansprüche 1 bis 21 oder ein physiologisch verträgliches Salz solch einer Verbindung sowie mindestens einen Wirkstoff ausgewählt aus der Gruppe Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer, Fibrate, Nikotinsäure und deren Derivate, PPAR-alpha Agonisten, PPAR-delta Agonisten, ACAT Inhibitoren oder Cholesterolresorptionsinhibitoren, gallensäurebindende Substanzen, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannabinoid1 Rezeptors, MCH-1 Rezeptorantagonisten, MC4 Rezeptor Agonisten, NPY5 oder NPY2 Antagonisten oder ß3-Agonisten und Agonisten des 5HT2c Rezeptors neben gegebenenfalls einem oder mehreren inerten Trägerstoffen und/oder Verdünnungsmitteln aufweist.

43. Arzneimittel, das eine Verbindung gemäß einem oder mehreren der Ansprüche 1 bis 21 oder ein physiologisch verträgliches Salz solch einer Verbindung sowie mindestens einen Wirkstoff ausgewählt aus der Gruppe der Medikamente zur Beeinflussung des Bluthochdrucks, des chronischen Herzversagens oder der Atherosklerose wie z.B. A-II Antagonisten oder ACE Inhibitoren, ECE-Inhibitoren, Diuretika, ß-Blocker, Ca-Antagonisten, zentral wirksamen Antihypertensiva, Antagonisten des alpha-2-adrenergen Rezeptors, Inhibitoren der neutralen Endopeptidase, und Thrombozytenaggregationshemmer oder Kombinationen daraus neben gegebenenfalls einem oder mehreren inerten Trägerstoffen und/oder Verdünnungsmitteln aufweist.

44. Arzneimittel gemäß Anspruch 43, **dadurch gekennzeichnet, daß** der Wirkstoff aus der Gruppe der Angiotensin II Rezeptor Antagonisten ausgewählt ist aus der Gruppe bestehend aus Candesartan Cilexetil, Kalium Losartan, Eprosartan Mesylat, Valsartan, Telmisartan, Irbesartan, EXP-3174, L-158809, EXP-3312, Olmesartan, Medoxomil, Tasosartan, KT-3-671, GA-0113, RU-64276, EMD-90423, BR-9701.

45. Arzneimittel, das eine Verbindung gemäß einem oder mehreren der Ansprüche 1 bis 21 oder ein physiologisch verträgliches Salz solch einer Verbindung sowie mindestens einen Wirkstoff ausgewählt aus der Gruppe Harnsäuresynthese Inhibitoren und Urikosurika neben gegebenenfalls einem oder mehreren inerten Trägerstoffen und/oder Verdünnungsmitteln aufweist.

46. Arzneimittel, das eine Verbindung gemäß einem oder mehreren der Ansprüche 1 bis 21 oder ein physiologisch verträgliches Salz solch einer Verbindung sowie mindestens einen Wirkstoff ausgewählt aus der Gruppe GABA-Rezeptor-Antagonisten, Na-Kanal-Blockern, Topiramat, Protein-Kinase C Inhibitoren, advanced glycation endproduct Inhibitoren und Aldose Reduktase Inhibitoren neben gegebenenfalls einem oder mehreren inerten Trägerstoffen und/oder Verdünnungsmitteln aufweist.

47. Arzneimittel nach einem der Ansprüche 41 bis 46, **dadurch gekennzeichnet, daß** die erfindungsgemäße Verbindung, oder dessen physiologisch verträgliches Salz, und der damit zu kombinierende weitere Wirkstoff zusammen in einer Darreichungsform oder getrennt in zwei gleichen oder verschiedenen Darreichungsformen vorliegen.

## Claims

1. Glucopyranosyl-substituted benzene derivatives of general formula I 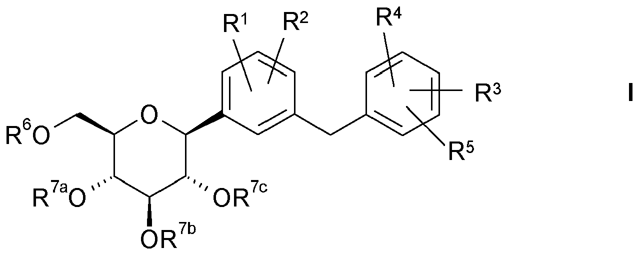 wherein
R¹ is selected from among the meanings hydrogen, fluorine, chlorine, bromine, iodine, C₁₋₄-alkyl, C₂₋₆-alkynyl, C₁₋₄-alkoxy, C₂₋₄-alkenyl-C₁₋₄-alkoxy, C₂₋₄-alkynyl-C₁₋₄-alkoxy, methyl substituted by 1 to 3 fluorine atoms, ethyl substituted by 1 to 5 fluorine atoms, methoxy substituted by 1 to 3 fluorine atoms, ethoxy substituted by 1 to 5 fluorine atoms, C₁₋₄-alkyl substituted by a hydroxy or C₁₋₃-alkoxy group, C₂₋₄-alkoxy substituted by a hydroxy or C₁₋₃-alkoxy group, C₂₋₆-alkenyl, C₃₋₆-cycloalkyl, C₃₋₆-cycloalkyl-C₁₋₃-alkyl, C₃₋₇-cydoalkyloxy, C₃₋₆-cycloalkyl-C₁₋₃-alkoxy, C₅₋₇-cycloalkenyloxy, hydroxy, amino, nitro or cyano, while in the C₅₋₆-cycloalkyl groups a methylene group may be replaced by O,
R² denotes hydrogen, fluorine, chlorine, bromine, hydroxy, C₁₋₄-alkyl, C₁₋₄-alkoxy, cyano or nitro, while the alkyl or alkoxy group may be mono- or polysubstituted by fluorine, and
R³ is selected from the meanings tri-(C₁₋₄-alkyl)silyl-C₁₋₆-alkyl, C₂₋₆-alkyn-1-yl, C₂₋₆-alken-1-yl, amino, C₁₋₃-alkylamino, di-(C₁₋₃-alkyl)amino, pyrrolidin-1-yl, pyrrolidin-2-on-1-yl, piperidin-1-yl, piperidin-2-on-1-yl, morpholin-4-yl, morpholin-3-on-4-yl, piperazin-1-yl, 4-(C₁₋₃-alkyl)piperazin-1-yl, nitro, C₃₋₇-cycloalkyloxy, C₅₋₇-cycloalkenyloxy, C₃₋₇-cycloalkylsulphanyl, C₃₋₇-cycloalkylsulphinyl, C₃₋₇-cydoalkyl-sulphonyl, C₅₋₇-cycloalkenylsulphanyl, C₅₋₇-cydoalkenylsulphinyl and C₅₋₇-cycloalkenylsulphonyl,
while the above-mentioned alkynyl and alkenyl groups may be mono- or polysubstituted by fluorine or chlorine, preferably fluorine, and
while the above-mentioned alkynyl and alkenyl groups may be mono- or disubstituted by identical or different groups L1;
while the above-mentioned cycloalkyl and cycloalkenyl rings independently of one another may be mono- or disubstituted by substituents selected from fluorine and C₁₋₃-alkyl, and
while in the above-mentioned cycloalkyl and cycloalkenyl rings one or two methylene groups may be replaced independently of one another by O, S, CO, SO, SO₂ or NR^{N}, preferably O, CO, S, SO₂ or NR^{N}, most preferably by O or CO,
R⁴, R⁵ independently of one another denote hydrogen, fluorine, chlorine, bromine, iodine, cyano, nitro, C₁₋₃-alkyl, C₁₋₃-alkoxy, methyl or methoxy substituted by 1 to 3 fluorine atoms,
R^{N} denotes H, C₁₋₄-alkyl, C₁₋₄-alkylcarbonyl or C₁₋₄-alkylsulphonyl,
L1 independently of one another are selected from among hydroxy-, cyano-, nitro-, C₃₋₇-cycloalkyl, aryl-, heteroaryl-, C₁₋₄-alkylcarbonyl-, arylcarbonyl-, heteroarylcarbonyl-, aminocarbonyl-, C₁₋₄-alkylaminocarbonyl-, di-(C₁₋₃-alkyl)aminocarbonyl-, pyrrolidin-1-ylcarbonyl-, piperidin-1-ylcarbonyl-, morpholin-4-ylcarbonyl, arylaminocarbonyl-, heteroarylaminocarbonyl-, C₁₋₄-alkoxycarbonyl-, aryl-C₁₋₃-alkoxycarbonyl-, heteroaryl-C₁₋₃-alkoxycarbonyl-, C₁₋₄-alkyloxy-, aryloxy-, heteroaryloxy-, C₁₋₄-alkylsulphanyl-, arylsulphanyl-, heteroarylsulphanyl-, C₁₋₄-alkylsulphinyl-, arylsulphinyl-, heteroarylsulphinyl-, C₁₋₄-alkylsulphonyl-, arylsulphonyl- and heteroarylsulphonyl-; and
L2 independently of one another are selected from among fluorine, chlorine, bromine, iodine, C₁₋₃-alkyl, difluoromethyl, trifluoromethyl, C₁₋₃-alkoxy, difluoromethoxy, trifluoromethoxy and cyano; and
R⁶ denotes hydrogen, (C₁₋₆-alkyl)oxycarbonyl or C₁₋₆-alkylcarbonyl,
R^{7a}, R^{7b}, R^{7c} denote hydrogen,
while by the aryl groups mentioned in the definition of the above groups are meant phenyl or naphthyl groups which may be mono- or disubstituted independently of one another by identical or different groups L2; and
by the heteroaryl groups mentioned in the definition of the above groups are meant a pyrrolyl, furanyl, thienyl, pyridyl, indolyl, benzofuranyl, benzothiophenyl, quinolinyl, isoquinolinyl or tetrazolyl group,
or is meant a pyrrolyl, furanyl, thienyl or pyridyl group, wherein one or two methyne groups are replaced by nitrogen atoms,
or is meant an indolyl, benzofuranyl, benzothiophenyl, quinolinyl or isoquinolinyl group, wherein one to three methyne groups are replaced by nitrogen atoms,
while the above-mentioned heteroaryl groups independently of one another may be mono- or disubstituted by identical or different groups L2;
while, unless otherwise stated, the above-mentioned alkyl groups may be straight-chain or branched,
the tautomers, the stereoisomers thereof, the mixtures thereof and the salts thereof.

2. Glucopyranosyl-substituted benzene derivatives of general formula 1.2 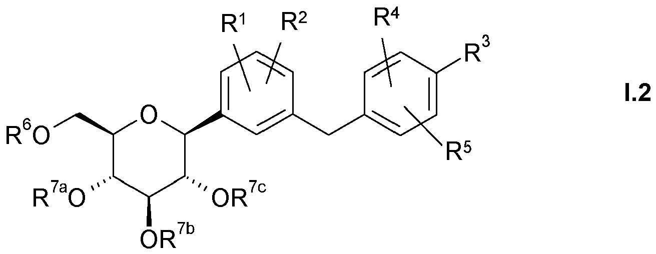 wherein the groups R¹ to R⁶ and R^{7a}, R^{7b} and R^{7c} are defined as in claim 1.

3. Glucopyranosyl-substituted benzene derivatives of general formula 1.2c 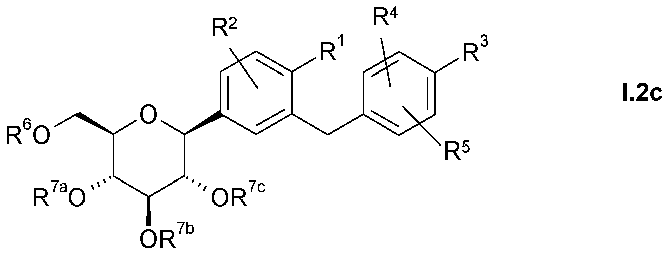 wherein the groups R¹ to R⁶ and R^{7a}, R^{7b} and R^{7c} are defined as in claim 1.

4. Glucopyranosyl-substituted benzene derivatives according to one or more of claims 1 to 3, **characterised in that** the group R¹ is selected from among the meanings hydrogen, fluorine, chlorine, bromine, cyano, methyl, ethyl, isopropyl, difluoromethyl, trifluoromethyl, ethynyl, prop-1-yn-1-yl, but-1-yn-1-yl, hydroxy, methoxy, ethoxy, difluoromethoxy, cyclopropyloxy, cyclobutyloxy, cyclopentyloxy, cyclohexyloxy.

5. Glucopyranosyl-substituted benzene derivatives according to claim 1, 2 or 3, **characterised in that** the group R¹ is selected from among the meanings methyl and chlorine.

6. Glucopyranosyl-substituted benzene derivatives according to one or more of claims 1 to 5, **characterised in that** the group R³ is selected from among the meanings tri-(C₁₋₄-alkyl)silyl-C₁₋₆-alkyl, C₂₋₆-alkyn-1-yl, C₂₋₆-alken-1-yl, C₃₋₇-cydoalkyloxy, C₅₋₇-cycloalkenyloxy, C₃₋₇-cycloalkylsulphanyl, C₅₋₇-cycloalkenylsulphanyl, while the above-mentioned alkynyl and alkenyl groups may be mono- or polysubstituted by fluorine or monosubstituted by chlorine or the group L1, and wherein in the cycloalkyl and cycloalkenyl groups one or two methylene groups may be replaced independently of one another by O, CO, S, SO₂ or NR^{N}, particularly O or CO, and L1 and R^{N} are defined as in claim 1.

7. Glucopyranosyl-substituted benzene derivatives according to one or more of claims 1 to 5, **characterised in that** the group R³ is selected from among the meanings trimethylsilylethyl, ethynyl, 1-propyn-1-yl, 1-butyn-1-yl, tert. butylethynyl, 2-hydroxyprop-2-ylethynyl, 2-methoxyprop-2-ylethynyl, 3-hydroxy-1-propyn-1-yl, 3-methoxy-1-propyn-1-yl, ethenyl, 1-propenyl, 1-butenyl, tert.-butylethenyl, cyclopropyloxy, cyclobutyloxy, cyclopentyloxy, cyclohexyloxy, tetrahydrofuranyloxy, tetrahydrothiophenyloxy, 1,1-dioxotetrahydrothiophenyloxy, tetrahydropyranyloxy, tetrahydrothiopyranyloxy, 1,1-dioxotetrahydrothiopyranyloxy, tetrahydrofuranonyloxy, piperidinyloxy, piperidinonyloxy, pyrrolidin-3-yloxy, pyrrolidinon-3-yloxy, tetrahydrofuranyl-sulphanyl, cyclopropylsulphanyl, cyclobutylsulphanyl, cyclopentylsulphanyl and cyclohexylsulphanyl, while the -NH group in a piperidinyl, piperidinonyl, pyrrolidinyl or pyrrolidinonyl ring may be substituted by C₁₋₃-alkyl or acetyl.

8. Glucopyranosyl-substituted benzene derivatives according to one or more of claims 1 to 5, **characterised in that** the group R³ is selected from among the meanings trimethylsilylethyl, ethynyl, 2-hydroxyprop-2-ylethynyl, 2-methoxyprop-2-ylethynyl, 3-hydroxy-1-propyn-1-yl, 3-methoxy-1-propyn-1-yl, cyclopropyloxy, cyclobutyloxy, cyclopentyloxy, cyclohexyloxy, tetrahydrofuran-3-yloxy, tetrahydropyran-4-yloxy, piperidin-4-yloxy, N-methylpiperidin-4-yloxy and N-acetylpiperidin-4-yloxy.

9. Glucopyranosyl-substituted benzene derivatives according to one of claims 1 to 5, **characterised in that** the group R³ is selected from among the meanings ethynyl, trimethylsilylethyl, cyclobutyloxy, cyclopentyloxy, cyclohexyloxy, tetrahydrofuran-3-yloxy and tetrahydropyran-4-yloxy.

10. Glucopyranosyl-substituted benzene derivatives according to one of claims 1 to 9, **characterised in that** the group R² denotes hydrogen, fluorine, chlorine, bromine, methyl, hydroxy, methoxy, ethoxy, trifluoromethoxy, cyano, nitro or methyl substituted by 1 to 3 fluorine atoms.

11. Glucopyranosyl-substituted benzene derivatives according to claim 10, **characterised in that** the group R² denotes hydrogen or methyl.

12. Glucopyranosyl-substituted benzene derivatives according to one of claims 1 to 11, **characterised in that** the groups R⁴ and/or R⁵ independently of one another denote hydrogen or fluorine.

13. Glucopyranosyl-substituted benzene derivatives according to claim 12, **characterised in that** the group R⁴ denotes hydrogen.

14. Glucopyranosyl-substituted benzene derivatives according to claim 12, **characterised in that** the group R⁵ denotes hydrogen.

15. Glucopyranosyl-substituted benzene derivatives according to one of claims 1 to 13, **characterised in that** the group R⁶ denotes hydrogen.

16. Glucopyranosyl-substituted benzene derivatives according to one of claims 1, 2, 3, 4 or 10, **characterised in that** the group R^{N} denotes H, methyl, ethyl or acetyl.

17. Glucopyranosyl-substituted benzene derivatives according to one or more of claims 1 to 16, **characterised in that** the group L1 is selected from among hydroxy-, cyano-, C₃₋₆-cycloalkyl, C₁₋₄-alkylcarbonyl-, aminocarbonyl-, C₁₋₄-alkylaminocarbonyl-, di-(C₁₋₃-alkyl)aminocarbonyl-, pyrrolidin-1-ylcarbonyl-, piperidin-1-ylcarbonyl-, morpholin-4-ylcarbonyl-, C₁₋₄-alkoxycarbonyl-, C₁₋₄-alkyloxy-, C₁₋₄-alkylsulphanyl-, C₁₋₄-alkylsulphinyl-, and C₁₋₄-alkylsulphonyl-.

18. Glucopyranosyl-substituted benzene derivatives according to one or more of claims 1 to 16, **characterised in that** the group L1 is selected from among hydroxy-, C₁₋₄-alkyloxy- and C₁₋₄-alkylsulphanyl-.

19. Glucopyranosyl-substituted benzene derivatives according to one or more of claims 1 to 18 selected from among the compounds (1) to (8), (10) to (15) and (17):
(1) 1-chloro-2-(4-cyclopentyloxybenzyl)-4-(β-D-glucopyranos-1-yl)-benzene
(2) 1-chloro-4-(β-D-glucopyranos-1-yl)-2-[4-(*(R)*-tetrahydrofuran-3-yloxy)-benzyl]-benzene
(3) 1-chloro-4-(β-D-glucopyranos-1-yl)-2-[4-(*(S)*-tetrahydrofuran-3-yloxy)-benzyl]-benzene
(4) 1-chloro-4-(β-D-glucopyranos-1-yl)-2-[4-(tetrahydrofuran-2-on-3-yloxy)-benzyl]-benzene
(5) 1-chloro-4-(β-D-glucopyranos-1-yl)-2-(4-cyclobutyloxy-benzyl)-benzene
(6) 1-chloro-4-(β-D-glucopyranos-1-yl)-2-(4-cyclohexyloxy-benzyl)-benzene
(7) 1-chloro-4-(β-D-glucopyranos-1-yl)-2-[4-(tetrahydropyran-4-yloxy)-benzyl]-benzene
(8) 1-chloro-4-(β-D-glucopyranos-1-yl)-2-[4-(1-acetyl-piperidin-4-yloxy)-benzyl]-benzene
(10) 1-(β-D-Glucopyranos-1-yl)-4-methyl-3-[4-(tetrahydrofuran-3-yloxy)-benzyl]-benzene
(11) 1-(β-D-Glucopyranos-1-yl)-4-methyl-3-[4-(2-trimethylsilyl-ethyl)-benzyl]-benzene
(12) 1-chloro-4-(β-D-glucopyranos-1-yl)-2-(4-ethynyl-benzyl)-benzene
(13) 1-chloro-4-(β-D-glucopyranos-1-yl)-2-[4-(piperidin-4-yloxy)-benzyl]-benzene
(14) 1-fluoro-4-(β-D-glucopyranos-1-yl)-2-(4-ethynyl-benzyl)-benzene
(15) 1-(β-D-glucopyranos-1-yl)-3-(4-ethynyl-benzyl)-benzene
(17) 1-methoxy-4-(β-D-glucopyranos-1-yl)-2-(4-ethynyl-benzyl)-benzene.

20. Glucopyranosyl-substituted benzene derivative according to claim 19, **characterised in that** the glucopyranosyl-substituted benzene derivative is the compound (2):
(2) 1-chloro-4-(β-D-glucopyranos-1-yl)-2-[4-((R)-tetrahydrofuran-3-yloxy)-benzyl]-benzene

21. Glucopyranosyl-substituted benzene derivative according to claim 19, **characterised in that** the glucopyranosyl-substituted benzene derivative is the compound (3):
(3) 1-chloro-4-(β-D-glucopyranos-1-yl)-2-[4-((S)-tetrahydrofuran-3-yloxy)-benzyl]-benzene.

22. Physiologically acceptable salts of the compounds according to at least one of claims 1 to 21 with inorganic or organic acids.

23. Medicament, containing a compound according to at least one of claims 1 to 21 or a physiologically acceptable salt according to claim 22, optionally together with one or more inert carriers and/or diluents.

24. Use of at least one compound according to at least one of claims 1 to 21 or a physiologically acceptable salt according to claim 22 for preparing a medicament which is suitable for the treatment or prevention of metabolic disorders selected from among type 1 and type 2 diabetes mellitus, complications of diabetes, metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

25. Use according to claim 24, **characterised in that** the metabolic disorder is selected from among type 1 and type 2 diabetes mellitus and complications of diabetes.

26. Use of at least one compound according to at least one of claims 1 to 21 or a physiologically acceptable salt according to claim 22 for preparing a medicament for inhibiting the sodium-dependent glucose cotransporter SGLT2.

27. Use of at least one compound according to at least one of claims 1 to 21 or a physiologically acceptable salt according to claim 22 for preparing a medicament for preventing the degeneration of pancreatic beta cells and/or for improving and/or restoring the functionality of pancreatic beta cells.

28. Use of at least one compound according to at least one of claims 1 to 21 or a physiologically acceptable salt according to claim 22 for preparing diuretics and/or antihypertensives.

29. Process for preparing the compounds of general formula I according to claims 1 to 21, **characterised in that** a compound of general formula II 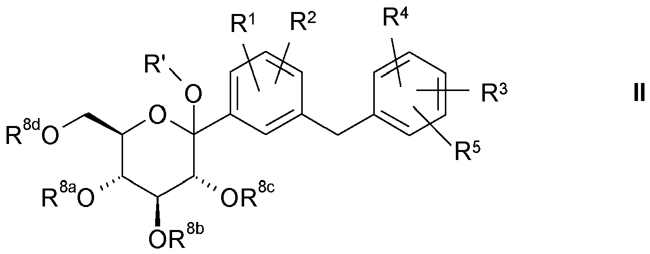 wherein
R' denotes H, C₁₋₄-alkyl, (C₁₋₁₈-alkyl)carbonyl, (C₁₋₁₈-alkyl)oxycarbonyl, arylcarbonyl and aryl-(C₁₋₃-alkyl)-carbonyl, wherein the alkyl or aryl groups may be mono- or polysubstituted by halogen;
R^{8a} ,R^{8b}, R^{8c}, R^{8d} independently of one another are selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl, or denote a benzyl group or a R^{a}R^{b}R^{c}Si group or a ketal or acetal group, while in each case two adjacent groups R^{8a}, R^{8b}, R^{8c}, R^{8d} may form a cyclic ketal or acetal group or a 1,2-di(C₁₋₃-alkoxy)-1,2-di(C₁₋₃-alkyl)-ethylene bridge, while the above-mentioned ethylene bridge forms, together with two oxygen atoms and the two associated carbon atoms of the pyranose ring, a substituted dioxane ring, and alkyl, aryl and/or benzyl groups may be mono- or polysubstituted by halogen or C₁₋₃-alkoxy and benzyl groups may also be substituted by a di-(C₁₋₃-alkyl)amino group; and
R^{a} R^{b}, R^{c} independently of one another denote C₁₋₄-alkyl, aryl or aryl-C₁₋₃-alkyl, wherein the aryl or alkyl groups may be mono- or polysubstituted by halogen;
while by the aryl groups mentioned in the definition of the above groups are meant phenyl or naphthyl groups, preferably phenyl groups;
and R¹ to R⁵ have the meanings given in claims 1 to 21,
is reacted with a reducing agent in the presence of a Lewis or Brønsted acid, while any protective groups present are cleaved simultaneously or subsequently;
if desired a compound of general formula I thus obtained wherein R⁶ denotes a hydrogen atom, is converted by acylation into a corresponding acyl compound of general formula I, and/or
if necessary any protective group used in the reactions described above is cleaved and/or
if desired a compound of general formula I thus obtained is resolved into its stereoisomers and/or
if desired a compound of general formula I thus obtained is converted into the physiologically acceptable salts thereof.

30. Process according to claim 29, **characterised in that** the compound of general formula II is obtained by the process described in claim 31 or 32.

31. Process for preparing compounds of general formula II 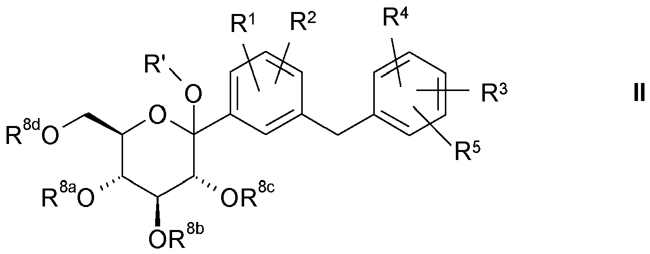 wherein
R' denotes H, C₁₋₄-alkyl, (C₁₋₁₈-alkyl)carbonyl, (C₁₋₁₈-alkyl)oxycarbonyl, arylcarbonyl or aryl-(C₁₋₃-alkyl)-carbonyl, wherein the alkyl or aryl groups may be mono- or polysubstituted by halogen;
R^{8a} ,R^{8b}, R^{8c}, R^{8d} independently of one another are selected from among hydrogen, (C₁-₁₈-alkyl)carbonyl, (C₁₋₁₈-alkyl)oxycarbonyl, arylcarbonyl and aryl-(C₁₋₃-alkyl)-carbonyl, or denote a benzyl group or a R^{a}R^{b}R^{c}Si group or a ketal or acetal group, while in each case two adjacent groups R^{8a}, R^{8b}, R^{8c}, R^{8d} may form a cyclic ketal or acetal group or a 1,2-di(C₁₋₃-alkoxy)-1,2-di(C₁₋₃-alkyl)-ethylene bridge, while the above-mentioned ethylene bridge forms, together with two oxygen atoms and the two associated carbon atoms of the pyranose ring, a substituted dioxane ring, and wherein alkyl, aryl and/or benzyl groups may be mono- or polysubstituted by halogen or C₁₋₃-alkoxy and benzyl groups may also be substituted by a di-(C₁₋₃-alkyl)amino group; and
R^{a}, R^{b}, R^{c} independently of one another denote C₁₋₄-alkyl, aryl or aryl-C₁₋₃-alkyl, wherein the aryl or alkyl groups may be mono- or polysubstituted by halogen;
while by the aryl groups mentioned in the definition of the above groups are meant phenyl or naphthyl groups, preferably phenyl groups;
and R¹ to R⁵ have the meanings given in claims 1 to 21,
wherein an organometallic compound (V) which may be obtained by halogen-metal exchange or by the insertion of a metal in the carbon-halogen bond of a halogen-benzylbenzene compound of general formula IV 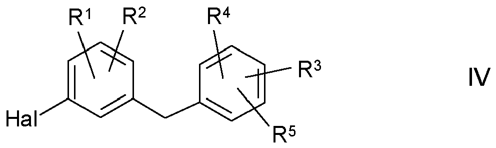 wherein Hal denotes Cl, Br and I and R¹ to R⁵ are as hereinbefore defined, and optionally subsequent transmetallation, is added to a gluconolactone of general formula VI 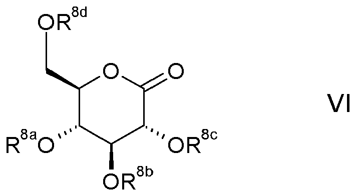 wherein R^{8a}, R^{8b}, R^{8c}, R^{8d} are as hereinbefore defined,
and
then the adduct obtained is reacted with water or an alcohol R'-OH, where R' denotes optionally substituted C₁₋₄-alkyl, in the presence of an acid and optionally the product obtained in the reaction with water wherein R' denotes H is converted in a subsequent reaction with an acylating agent into the product of formula II wherein R' denotes (C₁₋₁₈-alkyl)carbonyl, (C₁₋₁₈-alkyl)oxycarbonyl, arylcarbonyl or aryl-(C₁₋₃-alkyl)-carbonyl, which may be substituted as specified.

32. Process according to claim 31, **characterised in that** the organometallic compound (V) is a lithium or magnesium compound.

33. Process for preparing the compounds of general formula I according to claims 1 to 21, wherein R⁶ denotes hydrogen, **characterised in that** a compound of general formula III 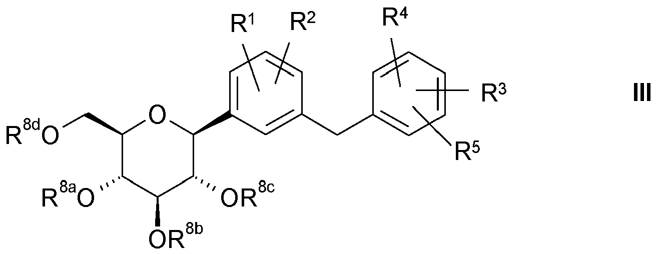 wherein
R^{8a}, R^{8b}, R^{8c}, R^{8d} independently of one another are selected from among hydrogen, (C₁₋₁₈-alkyl)carbonyl, (C₁₋₁₈-alkyl)oxycarbonyl, arylcarbonyl and aryl-(C₁₋₃-alkyl)-carbonyl, but at least one of the groups R^{8a}, R^{8b}, R^{8c}, R^{8d} does not denote hydrogen, or denotes a benzyl group or a R^{a}R^{b}R^{c}Si group or a ketal or acetal group, while in each case two adjacent groups R^{8a}, R^{8b}, R^{8c}, R^{8d} may form a cyclic ketal or acetal group or a 1,2-di(C₁₋₃-alkoxy)-1,2-di(C₁₋₃-alkyl)-ethylene bridge, while the above-mentioned ethylene bridge forms a substituted dioxane ring together with two oxygen atoms and the associated two carbon atoms of the pyranose ring, and wherein alkyl, aryl and/or benzyl groups may be mono- or polysubstituted by halogen or C₁₋₃-alkoxy and benzyl groups may also be substituted by a di-(C₁₋₃-alkyl)amino group; and
R^{a}, R^{b}, R^{c} independently of one another represent C₁₋₄-alkyl, aryl or aryl-C₁-₃-alkyl, while the alkyl or aryl groups may be mono- or polysubstituted by halogen;
while by the aryl groups mentioned in the definition of the above groups are meant phenyl or naphthyl groups, preferably phenyl groups;
and R¹ to R⁵ have the meanings given in claims 1 to 21,
is hydrolysed, and
if desired a compound of general formula I thus obtained wherein R⁶ denotes a hydrogen atom is converted by acylation into a corresponding acyl compound of general formula I, and/or
if necessary any protective group used in the reactions described above is cleaved and/or
if desired a compound of general formula I thus obtained is resolved into its stereoisomers and/or
if desired a compound of general formula I thus obtained is converted into the salts thereof, particularly for pharmaceutical use into the physiologically acceptable salts thereof.

34. Process according to claim 33, **characterised in that** the compound of formula III is obtained by a process according to claim 29 or 30.

35. Compound of general formula IV 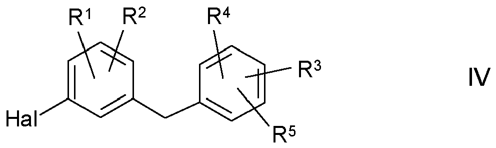 wherein Hal denotes chlorine, bromine or iodine and
R¹ denotes hydrogen, fluorine, chlorine, bromine, cyano, methyl, ethyl, isopropyl, difluoromethyl, trifluoromethyl, ethynyl, prop-1-yn-1-yl, but-1-yn-1-yl, hydroxy, methoxy, ethoxy, difluoromethoxy, cyclopropyloxy, cyclobutyloxy, cyclopentyloxy or cyclohexyloxy; and
R² denotes hydrogen or methyl; and
R³ is selected from among trimethylsilylethyl, ethynyl, 2-hydroxyprop-2-ylethynyl, 2-methoxyprop-2-ylethynyl, 3-hydroxy-1-propyn-1-yl, 3-methoxy-1-propyn-1-yl, cyclopropyloxy, cyclobutyloxy, cyclopentyloxy, cyclohexyloxy, tetrahydrofuran-3-yloxy, tetrahydropyran-4-yloxy, piperidin-4-yloxy, N-methylpiperidin-4-yloxy and N-acetylpiperidin-4-yloxy; and
R⁴ denotes hydrogen or fluorine; and
R⁵ denotes hydrogen or fluorine.

36. Compound of formula IV according to claim 35, **characterised by** the formula 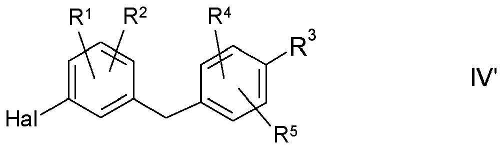 wherein Hal denotes chlorine, bromine or iodine and the groups R¹, R², R³, R⁴ and R⁵ are defined as in claim 35.

37. Compound of general formula II 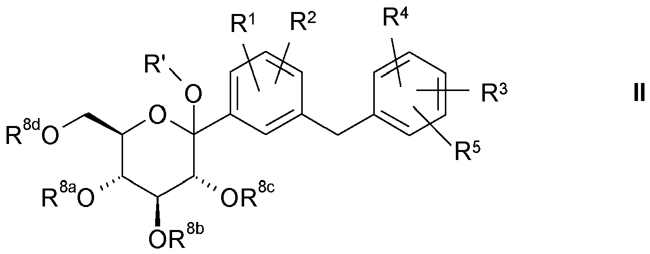 wherein
R' denotes H, C₁₋₄-alkyl, (C₁₋₁₈-alkyl)carbonyl, (C₁₋₁₈-alkyl)oxycarbonyl, arylcarbonyl or aryl-(C₁₋₃-alkyl)-carbonyl, wherein the alkyl or aryl groups may be mono- or polysubstituted by halogen;
R^{8a}, R^{8b}, R^{8e}, R^{8d} independently of one another are selected from among hydrogen, (C₁₋₁₈-alkyl)carbonyl, (C₁₋₁₈-alkyl)oxycarbonyl, arylcarbonyl and aryl-(C₁-₃-alkyl)-carbonyl, or denotes a benzyl group or a R^{a}R^{b}R^{c}Si group or a ketal or acetal group, while in each case two adjacent groups R^{8a}, R^{8b}, R^{8c}, R^{8d} may form a cyclic ketal or acetal group or a 1,2-di(C₁₋₃-alkoxy)-1,2-di(C₁₋₃-alkyl)-ethylene bridge, while the above-mentioned ethylene bridge forms a substituted dioxane ring together with two oxygen atoms and the associated two carbon atoms of the pyranose ring, and alkyl, aryl and/or benzyl groups may be mono- or polysubstituted by halogen or C₁₋₃-alkoxy and benzyl groups may also be substituted by a di-(C₁-₃-alkyl)amino group; and
R^{a}, R^{b}, R^{c} independently of one another denote C₁₋₄-alkyl, aryl or aryl-C₁₋₃-alkyl, while the alkyl or aryl groups may be mono- or polysubstituted by
halogen;
while by the aryl groups mentioned in the definition of the above groups are meant phenyl or naphthyl groups, preferably phenyl groups;
and R¹ to R⁵ are defined as in one or more of claims 1 and 4 to 14.

38. Glucopyranosyl-substituted benzene derivatives of general formula I 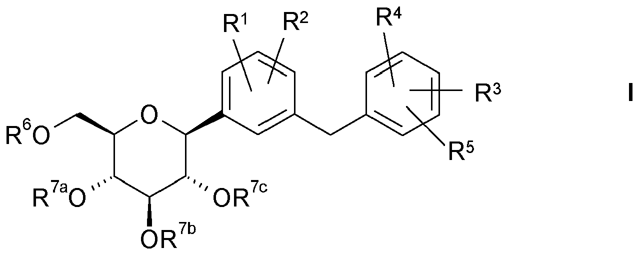 wherein R¹ to R⁵ are defined as in one or more of claims 1 to 21, and
R⁶, R^{7a}, R^{7b}, R^{7c} independently of one another have a meaning selected from among (C₁₋₁₈-alkyl)carbonyl, (C₁₋₁₈-alkyl)oxycarbonyl, arylcarbonyl and aryl-(C₁₋₃-alkyl)-carbonyl,
L2 independently of one another are selected from among fluorine, chlorine, bromine, iodine, C₁₋₃-alkyl, difluoromethyl, trifluoromethyl, C₁₋₃-alkoxy, difluoromethoxy, trifluoromethoxy and cyano; and
by the aryl groups mentioned in the definition of the above groups are meant phenyl or naphthyl groups which may be mono- or disubstituted independently of one another by identical or different groups L2; and
unless otherwise stated, the above-mentioned alkyl groups may be straight-chain or branched,
the tautomers, the stereoisomers thereof, the mixtures thereof and the salts thereof.

39. Glucopyranosyl-substituted benzene derivatives according to claim 38, **characterised by** the formula 1.2 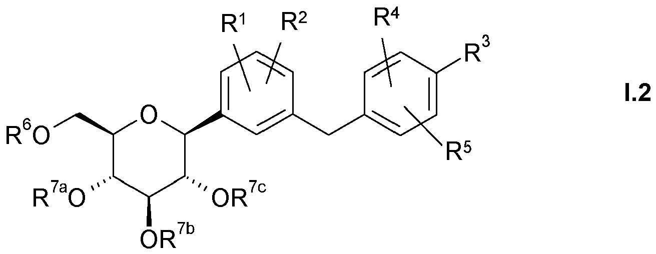 wherein R¹ to R⁵ and R⁶, R^{7a}, R^{7b}, R^{7c} are defined as in claim 38.

40. Glucopyranosyl-substituted benzene derivatives according to claim 38, **characterised by** the formula 1.2c 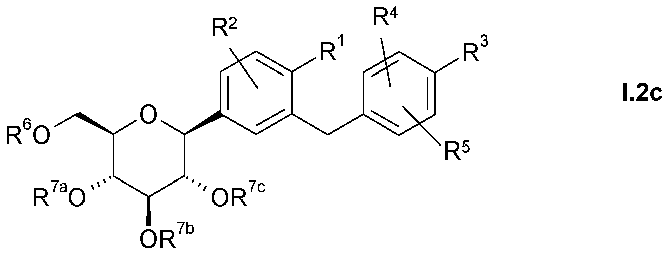 wherein R¹ to R⁵ and R⁶, R^{7a}, R^{7b}, R^{7c} are defined as in claim 38.

41. Medicament which contains a compound according to one or more of claims 1 to 21 or a physiologically acceptable salt of such a compound and at least one active substance selected from among antidiabetic agents, optionally together with one or more inert carriers and/or diluents.

42. Medicament which comprises a compound according to one or more of claims 1 to 21 or a physiologically acceptable salt of such a compound and at least one active substance selected from among inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase, lipid lowering agents such as for example HMG-CoA-reductase inhibitors, fibrates, nicotinic acid and the derivatives thereof, PPAR-alpha agonists, PPAR-delta agonists, ACAT inhibitors or cholesterol absorption inhibitors, bile acid-binding substances, inhibitors of ileac bile acid transport, HDL-raising compounds such as CETP inhibitors or ABC1 regulators or active substances for treating obesity, such as sibutramine or tetrahydrolipostatin, dexfenfluramine, axokine, antagonists of the cannabinoid1 receptor, MCH-1 receptor antagonists, MC4 receptor agonists, NPY5 or NPY2 antagonists or ß3-agonists and agonists of the 5HT2c receptor, optionally together with one or more inert carriers and/or diluents.

43. Medicament which comprises a compound according to one or more of claims 1 to 21 or a physiologically acceptable salt of such a compound and at least one active substance selected from among drugs for influencing high blood pressure, chronic heart failure or atherosclerosis such as e.g. A-II antagonists or ACE inhibitors, ECE inhibitors, diuretics, ß-blockers, Ca-antagonists, centrally acting antihypertensives, antagonists of the alpha-2-adrenergic receptor, inhibitors of neutral endopeptidase, and thrombocyte aggregation inhibitors or combinations thereof, optionally together with one or more inert carriers and/or diluents.

44. Medicament according to claim 43, **characterised in that** the active substance is selected from among the angiotensin II receptor antagonists which are selected from among candesartan cilexetil, potassium losartan, eprosartan mesylate, valsartan, telmisartan, irbesartan, EXP-3174, L-158809, EXP-3312, olmesartan, medoxomil, tasosartan, KT-3-671, GA-0113, RU-64276, EMD-90423, BR-9701.

45. Medicament which comprises a compound according to one or more of claims 1 to 21 or a physiologically acceptable salt of such a compound and at least one active substance selected from among the uric acid synthesis inhibitors and uricosurics, optionally together with one or more inert carriers and/or diluents.

46. Medicament which comprises a compound according to one or more of claims 1 to 21 or a physiologically acceptable salt of such a compound and at least one active substance selected from among GABA-receptor antagonists, Na-channel blockers, topiramat, protein-kinase C inhibitors, advanced glycation end product inhibitors and aldose reductase inhibitors, optionally together with one or more inert carriers and/or diluents.

47. Medicament according to one of claims 41 to 46, **characterised in that** the compound according to the invention, or the physiologically acceptable salt thereof, and the additional active substance to be combined therewith, are present together in one dosage form or separately in two identical or different dosage forms.

## Revendications

1. Dérivés de benzène substitués par un glucopyranosyle de formule générale I 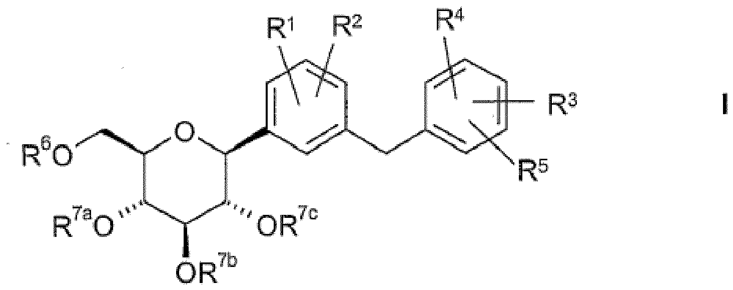 dans laquelle
R¹ est choisi parmi les significations: hydrogène, fluor, chlore, brome, iode, alkyle en C₁ à C₄, alcinyle en C₂ à C₆, alcoxy en C₁ à C₄, alcényle en C₂ à C₄-alcoxy en C₁ à C₄, alcinyle en C₂ à C₄-alcoxy en C₁ à C₄, méthyle pouvant être substitué par 1 à 3 atomes de fluor, éthyle substitué par 1 à 5 atomes de fluor, méthoxy substitué par 1 à 3 atomes de fluor, éthoxy substitué par 1 à 5 atomes de fluor, alkyle en C₁ à C₄ substitué par un groupe hydroxy ou alcoxy en C₁ à C₃, alcoxy en C₂ à C₄ substitué par un groupe hydroxy ou alcoxy en C₁ à C₃, alcényle en C₂ à C₆, cycloalkyle en C₃ à C₆, cycloalkyle en C₃ à C₆-alkyle en C₁ à C₃, cycloalkyloxy en C₃ à C₇, cycloalkyle en C₃ à C₆-alcoxy en C₁ à C₃, cycloalcényloxy en C₅ à C₇, hydroxy, amino, nitro ou cyano, dans laquelle dans les groupes cycloalkyle en C₅ à C₆, un groupe méthylène peut être remplacé par O,
R² désigne un atome d'hydrogène, de fluor, de chlore, de brome, un groupe hydroxy, alkyle en C₁ à C₄, alcoxy en C₁ à C₄, cyano ou nitro, le groupe alkyle ou alcoxy pouvant être mono- ou multi-substitué par un atome de fluor, et
R³ est choisi parmi les significations tri-(alkyle en C₁ à C₄)silyle-alkyle en C₁ à C₆, alcine en C₂ à C₆-1-yle, alcène en C₂ à C₆-1-yle, amino, alkylamino en C₁ à C₃, di- (alkyle en C₁ à C₃)amino, pyrrolidin-1-yle, pyrrolidin-2-on-1-yle, pipéridin-1-yle, pipéridin-2-on-1-yle, morpholin-4-yle, morpholin-3-on-4-yle, pipérazin-1-yle, 4-(alkyle en C₁ à C₃)pipérazin-1-yle, nitro, cycloalkyloxy en C₃ à C₇, cycloalcényloxy en C₅ à C₇, cycloalkylsulfanyle en C₃ à C₇, cycloalkylsulfinyle en C₃ à C₇, cycloalkylsulfonyle en C₃ à C₇, cycloalcénylsulfanyle en C₅ à C₇, cycloalcénylsulfinyle en C₅ à C₇ et cycloalcénylsulfonyle en C₅ à C₇,
les groupes alcinyle et alcényle précédemment cités pouvant être mono- ou multi-substitués par un atome de fluor ou de chlore, de préférence de fluor,et
les groupes alcinyle et alcényle précédemment cités pouvant être mono- ou disubstitués par des radicaux L1 identiques ou différents ;
les cycles cycloalkyle et cycloalcényle précédemment cités pouvant être mono- ou disubstitués indépendamment les uns des autres par des substituants choisis parmi un atome de fluor et un groupe alkyle en C₁ à C₃, et
dans les cycles cycloalkyle et cycloalcényle précédemment cités, un ou deux groupes méthylène pouvant être substitués indépendamment les uns des autres par O, S, CO, SO, SO₂ ou NR^{N}, de préférence, O, CO, S, SO₂ ou NR^{N}, de manière tout particulièrement préférée, par O ou CO,
R⁴, R⁵ désignent, indépendamment l'un de l'autre, un atome d'hydrogène, de fluor, de chlore, de brome, d'iode, un groupe cyano, nitro, alkyle en C₁ à C₃, alcoxy en C₁ à C₃, méthyle ou méthoxy substitué par 1 à 3 atomes de fluor,
R^{N} désigne H, un groupe alkyle en C₁ à C₄, alkylcarbonyle en C₁ à C₄ ou alkylsulfonyle en C₁ à C₄,
les L1 sont choisis, indépendamment les uns des autres dans le groupe comprenant un groupe hydroxy, cyano, nitro, cycloalkyle en C₃ à C₇, aryle, hétéroaryle, alkylcarbonyle en C₁ à C₄, arylcarbonyle, hétéroarylcarbonyle, amino-carbonyle, alkylaminocarbonyle en C₁ à C₄, di-(alkyle en C₁ à C₃)aminocarbonyle, pyrrolidin-1-ylcarbonyle, pipéridin-1-ylcarbonyle, morpholin-4-ylcarbonyle, arylaminocarbonyle, hétéroaryl-aminocarbonyle, alcoxycarbonyle en C₁ à C₄, arylalcoxycarbonyle en C₁ à C3, hétéroaryl-alcoxycarbonyle en C₁ à C₃, alkyloxy en C₁ à C₄, aryloxy, hétéroaryloxy, alkylsulfanyle en C₁ à C₄, arylsulfanyle, hétéroarylsulfanyle, alkyl-sulfinyle en C₁ à C₄, arylsulfinyle, hétéroaryl-sulfinyle, alkylsulfonyle en C₁ à C₄, aryl-sulfonyle et hétéroarylsulfonyle ; et
les L2 sont choisis indépendamment dans le groupe comprenant un atome de fluor, de chlore, de brome, d'iode, un groupe alkyle en C₁ à C₃, difluorométhyle, trifluorométhyle, alcoxy en C₁ à C₃, difluorométhoxy, trifluorométhoxy et cyano ; et
R⁶ désigne un atome d'hydrogène, un groupe (alkyle en C₁ à C₆) oxycarbonyle ou alkylcarbonyle en C₁ à C₆,
R^{7a}, R^{7b}, R^{7c} désignent un atome d'hydrogène,
sachant que les groupes aryle mentionnés dans la définition des radicaux mentionnés précédemment désignent des groupes phényle ou naphtyle qui peuvent être mono- ou disubstitués indépendamment les uns des autres par des radicaux L2 identiques ou différents ; et
les groupes hétéroaryle mentionnés dans la définition des radicaux mentionnés précédemment désignent un groupe pyrrolyle, furanyle, thiényle, pyridyle, indolyle, benzofuranyle, benzothiophényle, quinolinyle, isoquinolinyle ou tétrazolyle,
ou désignent un groupe pyrrolyle, furanyle, thiényle ou pyridyle dans lequel un ou deux groupes méthine sont remplacés par des atomes d'azote,
ou désignent un groupe indolyle, benzofuranyle, benzothiophényle, quinolinyle ou isoquinolinyle dans lequel un à trois groupes méthine sont remplacés par des atomes d'azote,
les groupes hétéroaryle mentionnés précédemment pouvant être mono- ou disubstitués indépendam-ment les uns des autres par des radicaux L2 identiques ou différents ;
où, sauf indication contraire, les groupes alkyle mentionnés précédemment peuvent être à chaîne linéaire ou ramifiée,
leurs tautomères, leurs stéréo-isomères, leurs mélanges et leurs sels.

2. Dérivés de benzène substitués par un glucopyranosyle de formule générale 1.2 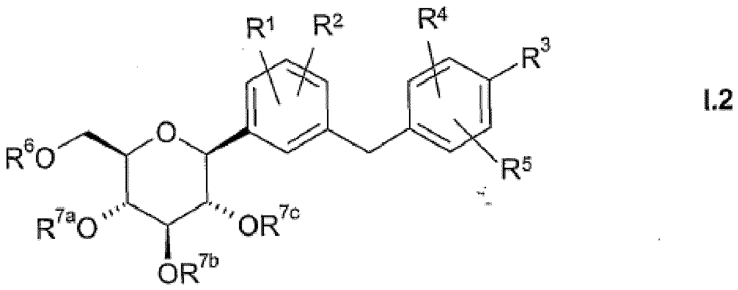 dans laquelle les radicaux R¹ à R⁶ et R^{7a}, R^{7b} et R^{7c} sont tels que définis à la revendication 1.

3. Dérivés de benzène substitués par un glucopyranosyle de formule générale I.2c 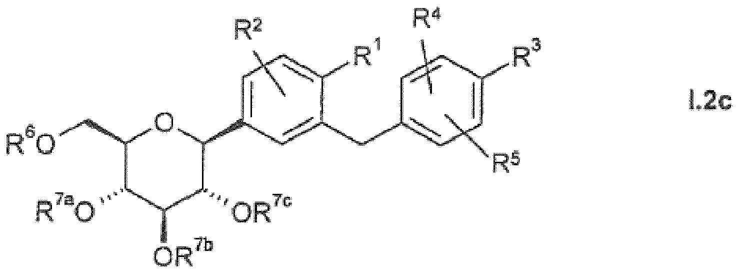 dans laquelle les radicaux R¹ à R⁶ et R^{7a}, R^{7b}, R^{7c} sont tels que définis à la revendication 1.

4. Dérivés de benzène substitués par un glucopyranosyle selon une ou plusieurs des revendications 1 à 3, **caractérisés en ce que** le groupe R¹ est choisi parmi les significations hydrogène, fluor, chlore, brome, cyano, méthyle, éthyle, isopropyle, difluorométhyle, trifluorométhyle, éthinyle, prop-1-in-1-yle, but-1-in-1-yle, hydroxy, méthoxy, éthoxy, difluorométhoxy, cyclopropyloxy, cyclobutyloxy, cyclopentyloxy, cyclohexyloxy.

5. Dérivés de benzène substitués par un glucopyranosyle selon la revendication 1, 2 ou 3, **caractérisés en ce que** le groupe R¹ est choisi parmi les significations méthyle et chlore.

6. Dérivés de benzène substitués par un glucopyranosyle selon une ou plusieurs des revendications 1 à 5, **caractérisés en ce que** le groupe R³ est choisi parmi les significations tri-(alkyle en C₁ à C₄) silyle-alkyle en C₁ à C₆, alcine en C₂ à C₆-1-yle, alcène en C₂ à C₆-1-yle, cycloalkyloxy en C₃ à C₇, cycloalcényloxy en C₅ à C₇, cycloalkylsulfanyle en C₃ à C₇, cycloalcényle en C₅ à C₇-sulfanyle, les groupes alcinyle et alcényle précédemment cités pouvant être mono- ou multisubstitués par un atome de fluor ou monosubstitués par un atome de chlore ou le radical L1 et dans les groupes cycloalkyle et cycloalcényle, un ou deux groupes méthylène pouvant être substitués indépendamment les uns des autres par O, CO, S, SO₂ ou NR^{N}, en particulier par O ou CO, et
L1 et R^{N} sont tels que définis dans la revendication 1.

7. Dérivés de benzène substitués par un glucopyranosyle selon une ou plusieurs des revendications 1 à 5, **caractérisés en ce que** le groupe R³ est choisi parmi les significations triméthylsilyléthyle, éthinyle, 1-propin-1-yle, 1-butin-1-yle, tert-butyléthinyle, 2-hydroxyprop-2-yléthinyle, 2-méthoxyprop-2-yléthinyle, 3-hydroxy-1-propin-1-yle, 3-méthoxy-1-propin-1-yle, éthényle, 1-propényle, 1-butényle, tert-butyl-éthényle, cyclopropyloxy, cyclobutyloxy, cyclopentyloxy, cycloexyloxy, tétrahydrofuranyloxy, tétrahydrothiophényloxy, 1,1-dioxotétrahydrothio-phényloxy, tétrahydropyranyloxy, tétrahydrothio-pyranyloxy, 1,1-dioxotétrahydrothiopyranyloxy, tétrahydrofuranonyloxy, pipéridinyloxy, pipéridinonyloxy, pyrrolidin-3-yloxy, pyrrolidinon-3-yloxy, tétrahydrofuranylsulfanyle, cyclopropyl-sulfanyle, cyclobutylsulfanyle, cyclopentyl-sulfanyle et cyclohexylsulfanyle, le groupe -NH- dans un cycle pipéridinyle, pipéridinonyle, pyrrolidinyle ou pyrrolidinonyle pouvant être substitué par un groupe alkyle en C₁ à C₃ ou acétyle.

8. Dérivés de benzène substitués par un glucopyranosyle selon une ou plusieurs des revendications 1 à 5, **caractérisés en ce que** le groupe R³ est choisi parmi les significations triméthylsilyléthyle, éthinyle, 2-hydroxyprop-2-éthinyle, 2-méthoxyprop-2-yléthinyle, 3-hydroxy-1-propin-1-yle, 3-méthoxy-1-propin-1-yle, cyclopropyloxy, cyclobutyloxy, cyclopentyloxy, cyclohexyloxy, tétrahydrofuran-3-yloxy, tétrahydropyran-4-yloxy, pipéridin-4-yloxy, N-méthylpipéridin-4-yloxy et N-acétylpipéridin-4-yloxy.

9. Dérivés de benzène substitués par un glucopyranosyle selon l'une des revendications 1 à 5, **caractérisés en ce que** le groupe R³ est choisi parmi les significations éthinyle, triméthylsilyléthyle, cyclobutyloxy, cyclopentyloxy, cyclohexyloxy, tétrahydrofuran-3-yloxy et tétrahydropyran-4-yloxy.

10. Dérivés de benzène substitués par un glucopyranosyle selon l'une des revendications 1 à 9, **caractérisés en ce que** le radical R² désigne un atome d'hydrogène, de fluor, de chlore, de brome, un groupe méthyle, hydroxy, méthoxy, éthoxy, trifluorométhoxy, cyano, nitro ou un groupe méthyle substitué par 1 à 3 atomes de fluor.

11. Dérivés de benzène substitués par un glucopyranosyle selon la revendication 10, **caractérisés en ce que** le radical R² désigne un atome d'hydrogène ou un groupe méthyle.

12. Dérivés de benzène substitués par un glucopyranosyle selon l'une des revendications 1 à 11, **caractérisés en ce que** les radicaux R⁴ et/ou R⁵ désignent indépendamment les uns des autres, un atome d'hydrogène ou de fluor.

13. Dérivés de benzène substitués par un glucopyranosyle selon la revendication 12, **caractérisés en ce que** le radical R⁴ désigne un atome d'hydrogène.

14. Dérivés de benzène substitués par un glucopyranosyle selon la revendication 12, caractérisés en ce le radical R⁵ désigne un atome d'hydrogène.

15. Dérivés de benzène substitués par un glucopyranosyle selon l'une des revendications 1 à 13, **caractérisés en ce que** le radical R⁶ désigne un atome d'hydrogène.

16. Dérivés de benzène substitués par un glucopyranosyle selon l'une des revendications 1, 2, 3, 4 ou 10, **caractérisés en ce que** le radical R^{N} désigne H, un groupe méthyle, éthyle ou acétyle.

17. Dérivés de benzène substitués par un glucopyranosyle selon une ou plusieurs des revendications 1 à 16, **caractérisés en ce que** le radical L1 est choisi dans le groupe comprenant un groupe hydroxy, cyano, cycloalkyle en C₃ à C₆, alkylcarbonyle en C₁ à C₄, aminocarbonyle, alkylaminocarbonyle en C₁ à C₄, di-(alkyle en C₁ à C₃)aminocarbonyle, pyrrolidin-1-ylcarbonyle, pipéridin-1-ylcarbonyle, morpholin-4-ylcarbonyle, alcoxycarbonyle en C₁ à C₄, alkyloxy en C₁ à C₄, alkylsulfanyle en C₁ à C₄, alkylsulfinyle en C₁ à C₄ et alkylsulfonyle en C₁ à C₄.

18. Dérivés de benzène substitués par un glucopyranosyle selon une ou plusieurs des revendications 1 à 16, **caractérisés en ce que** le radical L1 est choisi dans le groupe comprenant un groupe hydroxy, alkyloxy en C₁ à C₄ et alkyl-sulfanyle en C₁ à C₄.

19. Dérivés de benzène substitués par un glucopyranosyle selon une ou plusieurs des revendications 1 à 18, **caractérisés par** le groupe de composés (1) à (8), (10) à (15) et (17) :
(1) 1-chloro-2-(4-cyclopentyloxybenzyl)-4-(β-D-glucopyranos-1-yl)-benzène
(2) 1-chloro-4-(β-D-glucopyranos-1-yl)-2-[4-((R)-tétrahydrofuran-3-yloxy)-benzyl]-benzène
(3) 1-chloro-4- (**β**-D-glucopyranos-1-yl)-2-[4-((S)-tétrahydrofuran-3-yloxy)-benzyl]-benzène
(4) 1-chloro-4-(**β**-D-glucopyranos-1-yl)-2- [4-(tétrahydrofuran-2-on-3-yloxy)-benzyl]-benzène
(5) 1-chloro-4-(**β**-D-glucopyranos-1-yl)-2-(4-cyclobutyloxybenzyl)-benzène
(6) 1-chloro-4-(**β**-D-glucopyranos-1-yl)-2-(4-cyclohexyloxybenzyl)-benzène
(7) 1-chloro-4-(**β**-D-glucopyranos-1-yl)-2- [4-(tétrahydropyran-4-yloxy)-benzyl]-benzène
(8) 1-chloro-4-(**β**-D-glucopyranos-1-yl)-2- [4- (1-acétylpipéridin-4-yloxy)-benzyl]-benzène (10) 1-(**β**-D-glucopyranos-1-yl)-4-méthyl-3- [4-(tétrahydrofuran-3-yloxy)-benzyl]-benzène
(11) 1-(**β**-D-glucopyranos-1-yl)-4-méthyl-3- [4-(2-tri-méthylsilyléthyl)-benzyl]-benzène
(12) 1-chloro-4-(**β**-D-glucopyranos-1-yl)-2- (4-éthinylbenzyl)-benzène
(13) 1-chloro-4-(**β**-D-glucopyranos-1-yl)-2- [4-(pipéridin-4-yloxy)-benzyl]-benzène
(14) 1-fluoro-4-(**β**-D-glucopyranos-1-yl)-2- (4-éthinylbenzyl)-benzène
(15) 1-(**β**-D-glucopyranos-1-yl)-3- (4-éthinylbenzyl)-benzène
(17) 1-méthoxy-4-(**β**-D-glucopyranos-1-yl)-2- (4-éthinylbenzyl)-benzène.

20. Dérivé de benzène substitués par un glucopyranosyle selon la revendication 19, **caractérisée en ce que** le dérivé de benzène substitués par un glucopyranosyle est le composé (2):
(2) 1-chloro-4- (β-D-glucopyranos-1-yl)-2-[4- ((R)-tétrahydrofuran-3-yloxy)-benzyl]-benzène.

21. Dérivé de benzène substitués par un glucopyranosyle selon la revendication 19, **caractérisée en ce que** le dérivé de benzène substitués par un glucopyranosyle est le composé (3):
(3) 1-chloro-4- (β-D-glucopyranos-1-yl)-2-[4- ((S)-tétrahydrofuran-3-yloxy)-benzyl]-benzène.

22. Sels physiologiquement acceptables des composés selon au moins l'une des revendications 1 à 21, avec des acides anorganiques ou organiques.

23. Médicament contenant un composé selon au moins l'une des revendications 1 à 21 ou sel physiologiquement acceptable selon la revendication 22, parallèlement éventuellement à un ou plusieurs véhicules et/ou diluants inertes.

24. Utilisation d'au moins un composé selon au moins l'une des revendications 1 à 21 ou d'un sel physiologiquement acceptable selon la revendication 22 pour la production d'un médicament qui est approprié pour le traitement ou la prévention des maladies métaboliques choisies dans le groupe comprenant le diabète sucré de type 1 et de type 2, les complications diabétiques, l'acidose ou la cétose métabolique, l'hypoglycémie réactive, l'hyperinsulinémie, les troubles du métabolisme du glucose, l'insulino-résistance, le syndrome métabolique, les dys-lipidémies d'origines les plus diverses, l'athérosclérose et maladies apparentées, les adiposités, l'hypertension, l'insuffisance cardiaque chronique, les oedèmes, l'hyperuricémie.

25. Utilisation selon la revendication 24, **caractérisée en ce que** la maladie métabolique est choisie dans le groupe comprenant le diabète sucré de type 1 et de type 2 et les complica-tions diabétiques.

26. Utilisation d'au moins un composé selon au moins l'une des revendications 1 à 21, ou d'un sel physiologiquement acceptable selon la revendication 22, pour la production d'un médicament pour l'inhibition du cotransporteur du glucose sodium-dépendant SGLT2.

27. Utilisation d'au moins un composé selon au moins l'une des revendications 1 à 21, ou d'un sel physiologiquement acceptable selon la revendication 22, pour la production d'un médicament pour empêcher la dégénérescence des cellules bêta pancréatiques et/ou pour améliorer et/ou restaurer la fonctionnalité des cellules bêta pancréatiques.

28. Utilisation d'au moins un composé selon au moins l'une des revendications 1 à 21, ou d'un sel physiologiquement acceptable selon la revendication 22 pour la production de diurétiques et/ou d'antihypertenseurs.

29. Procédé de production des composés selon la formule générale I selon les revendications 1 à 21, **caractérisé en ce qu'**on fait réagir un composé de formule générale II 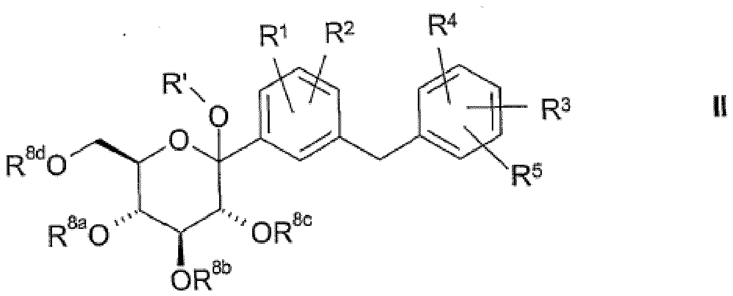 dans laquelle
R' désigne H, un groupe alkyle en C₁ à C₄, (alkyle en C₁ à C₁₈) carbonyle, (alkyle en C₁ à C₁₈)oxycarbonyle, arylcarbonyle ou aryle-(alkyle en C₁ à C₃)-carbonyle, les groupes alkyle ou aryle pouvant être mono- ou multi-substitués par un atome d'halogène ;
R^{8a}, R^{8b},
R^{8c}, R^{8d} sont choisis indépendamment les uns des autres dans le groupe comprenant un atome d'hydrogène, un groupe (alkyle en C₁ à C₁₈)-carbonyle, (alkyle en C₁ à C₁₈)oxycarbonyle, arylcarbonyle et aryle-(alkyle en C₁ à C₃)-carbonyle ou un groupe benzyle ou un groupe R^{a}R^{b}R^{c}Si ou un groupe cétal ou acétal, respectivement deux radicaux voisins R^{8a}, R^{8b}, R^{8c}, R^{8d} pouvant former un groupe cétal ou acétal cyclique ou un pont 1,2-di(alcoxy en C₁ à C₃)-1,2-di(alkyle en C₁ à C₃)-éthylène, le pont éthylène cité formant conjointement avec deux atomes d'oxygène et les deux atomes de carbone dépendants du cycle pyranose, un cycle dioxane substitué et les groupes alkyle, aryle et/ou benzyle pouvant être mono- ou multisubstitués par un atome d'halogène ou un groupe alcoxy en C₁ à C₃ et les groupes benzyle pouvant être substitués par un groupe di-(alkyle en C₁ à C₃)-amino; et
R^{a}, R^{b}, R^{c} désignent indépendamment les uns des autres, un groupe alkyle en C₁ à C₄, aryle ou aryle-alkyle en C₁ à C₃, les groupes alkyle ou aryle pouvant être mono- ou multisubstitués par un atome d'halogène;
sachant que les groupes aryle mentionnés dans la définition des radicaux précédemment cités désignent des groupes phényle ou naphtyle, de préférence des groupes phényle ;
et R¹ à R⁵ ont les significations indiquées dans les revendications 1 à 21,
avec un milieu de réduction en présence d'un acide de Lewis ou de Bronsted, les groupes protecteurs éventuellement présents étant clivés de façon concomitante ou ultérieurement ;
éventuellement un composé de formule générale I ainsi obtenu, dans laquelle R⁶ représente un atome d'hydrogène, est converti par acylation en un composé acyle correspondant de formule générale I et/ou
si besoin, un radical protecteur utilisé dans les réactions décrites précédemment est à nouveau clivé et/ou
si on le souhaite, un composé de formule générale I ainsi obtenu est séparé en ses stéréo-isomères et/ou
si on le souhaite, un composé de formule générale I ainsi obtenu est converti en ses sels physiologiquement acceptables.

30. Procédé selon la revendication 29, **caractérisé en ce que** le composé de formule générale II est obtenu selon le procédé décrit dans la revendication 31 ou 32.

31. Procédé de production des composés de formule générale II 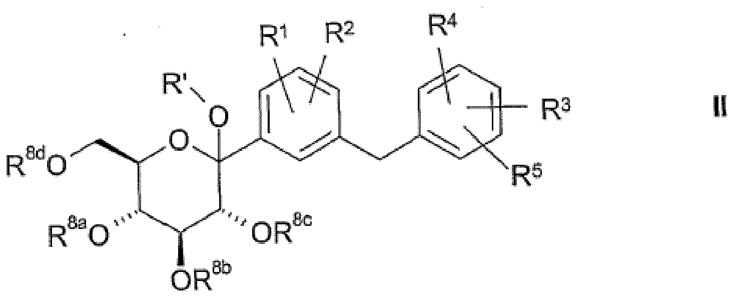 dans laquelle
R' désigne H, un groupe alkyle en C₁ à C₄, (alkyle en C₁ à C₁₈) carbonyle, (alkyle en C₁ à C₁₈)oxycarbonyle, arylcarbonyle ou aryle-(alkyle en C₁ à C₃)-carbonyle, les groupes alkyle ou aryle pouvant être mono- ou multi-substitués par un atome d'halogène ;
R^{8a}, R^{8b},
R^{8c}, R^{8d} sont choisis indépendamment les uns des autres dans le groupe comprenant un atome d'hydrogène, un groupe (alkyle en C₁ à C₁₈)-carbonyle, (alkyle en C₁ à C₁₈)oxycarbonyle, arylcarbonyle et aryle-(alkyle en C₁ à C₃)-carbonyle ou un groupe benzyle ou un groupe R^{a}R^{b}R^{c}Si ou un groupe cétal ou acétal, respectivement deux radicaux voisins R^{8a}, R^{8b}, R^{8c}, R^{8d} pouvant former un groupe cétal ou acétal cyclique ou un pont 1,2-di(alcoxy en C₁ à C₃)-1,2-di(alkyle en C₁ à C₃)-éthylène, le pont éthylène cité formant conjointement avec deux atomes d'oxygène et les deux atomes de carbone dépendants du cycle pyranose, un cycle dioxane substitué et les groupes alkyle, aryle et/ou benzyle pouvant être mono- ou multisubstitués par un atome d'halogène ou un groupe alcoxy en C₁ à C₃ et les groupes benzyle pouvant être substitués également par un groupe di-(alkyle en C₁ à C₃)amino ; et
R^{a}, R^{b}, R^{c} désignent indépendamment les uns des autres, un groupe alkyle en C₁ à C₄, aryle ou aryle-alkyle en C₁ à C₃, les groupes alkyle ou aryle pouvant être mono- ou multisubstitués par un atome d'halogène ;
sachant que les groupes aryle mentionnés dans la définition des radicaux précédemment cités désignent des groupes phényle ou naphtyle, de préférence des groupes phényle ;
et R¹ à R⁵ ont les significations indiquées dans les revendications 1 à 21,
dans laquelle un composé organométallique (V) qui peut être obtenu par échange d'halogène-métal ou par insertion d'un métal dans le composé de carbone-halogène d'un composé d'halo-gène-benzylbenzène de formule générale IV 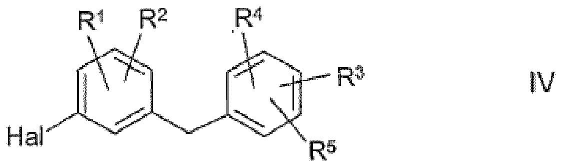 dans laquelle Hal désigne Cl, Br et I et les R¹ à R⁵ sont tels que définis précédemment et éventuellement, une remétallisation consécutive, sur un atome de gluconolactone de formule générale VI 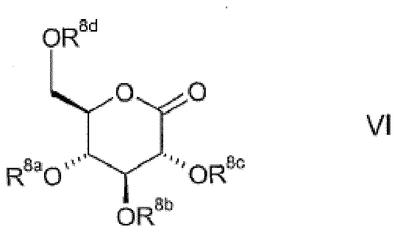 dans laquelle R^{8a}, R^{8b}, R^{8c}, R^{8d} sont tels que définis précédemment,
est ajouté, et
ensuite, on fait réagir l'adduit obtenu avec de l'eau ou un alcool R'-OH, où R' désigne un groupe alkyle en C₁ à C₄ éventuellement substitué, en présence d'un acide et éventuellement, le produit obtenu dans la réaction avec de l'eau dans lequel R' désigne H, est converti dans une réaction consécutive avec un agent acylant en produit de formule II où R' désigne un groupe (alkyle en C₁ à C₁₈)carbonyle, (alkyle en C₁ à C₁₈) oxycarbonyle, arylcarbonyle ou aryl(alkyle en C₁ à C₃)-carbonyle qui peut être substitué comme indiqué.

32. Procédé selon la revendication 31, **caractérisé en ce que** le composé organométallique (V) est un composé de lithium ou de magnésium.

33. Procédé de production des composés de formule générale I selon les revendications 1 à 21, dans lequel R⁶ désigne un atome d'hydrogène, **caractérisé en ce qu'**un composé de formule générale III 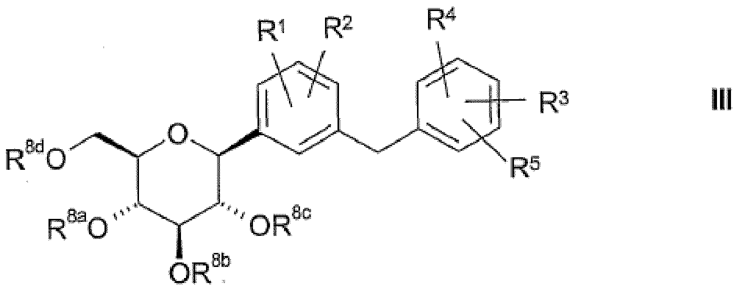 dans laquelle
R^{8a}, R^{8b},
R^{8c}, R^{8d} sont choisis indépendamment les uns des autres dans le groupe comprenant un atome d'hydrogène, un groupe (alkyle en C₁ à C₁₈)-carbonyle, (alkyle en C₁ à C₁₈)oxycarbonyle, arylcarbonyle et aryle-(alkyle en C₁ à C₃)-carbonyle, toutefois au moins l'un des radicaux R^{8a}, R^{8b}, R^{8c}, R^{8d} ne désigne pas un atome d'hydrogène ou un groupe benzyle ou un groupe R^{a}R^{b}R^{c} ou un groupe cétal ou acétal, respective-ment deux radicaux R^{8a}, R^{8b}, R^{8c}, R^{8d} voisins pouvant former un groupe cétal ou acétal cyclique ou un pont 1,2-di(alcoxy en C₁ à C₃) -1,2-di (alkyle en C₁ à C₃)-éthylène, le pont éthylène cité formant conjointement avec deux atomes d'oxygène et les deux atomes de carbone dépendants du cycle pyranose un cycle dioxane substitué et les groupes alkyle, aryle et/ou benzyle pouvant être mono- ou multisubstitués par un atome d'halogène ou un groupe alcoxy en C₁ à C₃ et les groupes benzyle pouvant être substitués également par un groupe di-(alkyle en C₁ à C₃) amino ; et
R^{a}, R^{b}, R^{c} désignent indépendamment les uns des autres, un groupe alkyle en C₁ à C₄, aryle ou aryle-alkyle en C₁ à C₃, les groupes alkyle ou aryle pouvant être mono- ou multisubstitués par un atome d'halogène ;
sachant que les groupes aryle mentionnés dans la définition des radicaux précédemment cités désignent des groupes phényle ou naphtyle, de préférence des groupes phényle ;
et R¹ à R⁵ ont les significations indiquées dans les revendications 1 à 21,
est hydrolysé, et
si on le souhaite, un composé de formule I ainsi obtenu, dans laquelle R⁶ désigne un atome d'hydrogène, est converti par acylation en un composé acyle correspondant de formule générale I, et/ou
si besoin, un radical protecteur utilisé dans les réactions décrites précédemment est à nou-veau clivé et/ou
si on le souhaite, un composé de formule géné-rale I ainsi obtenu est séparé en ses stéréo-isomères et/ou
si on le souhaite, un composé de formule générale I ainsi obtenu est converti en ses sels physiologiquement acceptables, en particulier pour l'utilisation pharmaceutique, en leurs sels physiologiquement acceptables.

34. Procédé selon la revendication 33, **caractérisé en ce que** le composé de formule III est obtenu par un procédé selon la revendication 29 ou 30.

35. Composé de formule générale IV 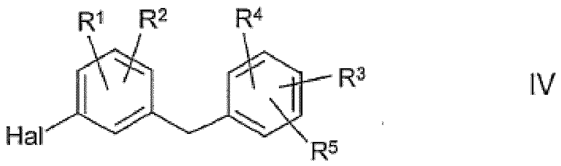 dans laquelle Hal désigne un atome de chlore, de brome ou d'iode et
R¹ désigne un atome d'hydrogène, de fluor, de chlore, de brome, un groupe cyano, méthyle, éthyle, isopropyle, difluorométhyle, trifluoro-méthyle, éthinyle, prop-1-in-1-yle, but-1-in-1-yle, hydroxy, méthoxy, éthoxy, difluorométhoxy, cyclopropyloxy, cyclobutyloxy, cyclopentyloxy ou cyclohexyloxy ; et
R² désigne un atome d'hydrogène ou un groupe méthyle ; et
R³ est choisi parmi les groupes triméthylsilyléthyle, éthinyle, 2-hydroxyprop-2-yléthinyle, 2-méthoxyprop-2-yléthinyle, 3-hydroxy-1-propin-1-yle, 3-méthoxy-1-propin-1-yle, cyclopropyloxy, cyclobutyloxy, cyclopentyloxy, cyclohexyloxy, tétrahydrofuran-3-yloxy, tétrahydropyran-4-yloxy, pipéridin-4-yloxy, N-méthylpipéridin-4-yloxy et N-acétylpipéridin-4-yloxy ; et
R⁴ désigne un atome d'hydrogène ou de fluor ; et
R⁵ désigne un atome d'hydrogène ou de fluor.

36. Composé de formule IV selon la revendication 35, **caractérisé par** la formule 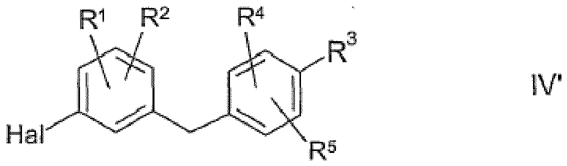 dans laquelle Hal désigne un atome de chlore, de brome ou d'iode et les radicaux R¹, R², R³, R⁴ et R⁵ sont tels que définis dans la revendication 35.

37. Composé de formule générale II 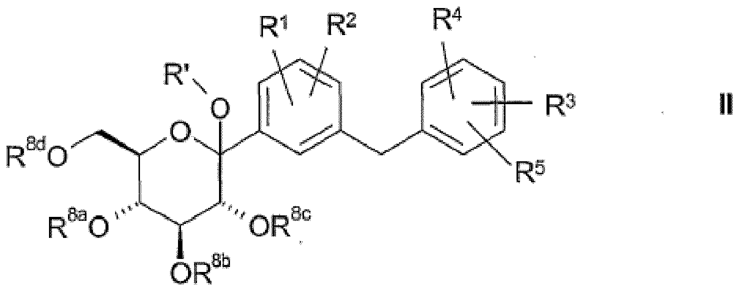 dans laquelle
R' désigne H, un groupe alkyle en C₁ à C₄, (alkyle en C₁ à C₁₈) carbonyle, (alkyle en C₁ à C₁₈)oxycarbonyle, arylcarbonyle ou aryle-(alkyle en C₁ à C₃)-carbonyle, les groupes alkyle ou aryle pouvant être mono- ou multi-substitués par un atome d'halogène ;
R^{8a}, R^{8b},
R^{8c}, R^{8d} sont choisis indépendamment les uns des autres dans le groupe comprenant un atome d'hydrogène, un groupe (alkyle en C₁ à C₁₈)-carbonyle, (alkyle en C₁ à C₁₈)oxycarbonyle, arylcarbonyle et aryle-(alkyle en C₁ à C₃)-carbonyle ou un groupe benzyle ou un groupe R^{a}R^{b}R^{c}Si ou un groupe cétal ou acétal, respectivement deux radicaux voisins R^{8a}, R^{8b}, R^{8c}, R^{8d} pouvant former un groupe cétal ou acétal cyclique ou un pont 1,2-di(alcoxy en C₁ à C₃)-1,2-di(alkyle en C₁ à C₃)-éthylène, le pont éthylène cité formant conjointement avec deux atomes d'oxygène et les deux atomes de carbone dépendants du cycle pyranose, un cycle dioxane substitué et les groupes alkyle, aryle et/ou benzyle pouvant être mono- ou multisubstitués par un atome d'halogène ou un groupe alcoxy en C₁ à C₃ et les groupes benzyle pouvant être substitués également par un groupe di-(alkyle en C₁ à C₃)amino ; et
R^{a}, R^{b}, R^{c} désignent indépendamment les uns des autres, un groupe alkyle en C₁ à C₄, aryle ou aryle-alkyle en C₁ à C₃, les groupes alkyle ou aryle pouvant être mono- ou multisubstitués par un atome d'halogène ;
sachant que les groupes aryle mentionnés dans la définition des radicaux précédemment cités désignent des groupes phényle ou naphtyle, de préférence des groupes phényle ;
et R¹ à R⁵ ont les significations indiquées dans les revendications 1 et 4 à 14.

38. Dérivés de benzène substitués par un glucopyranosyle de formule générale I 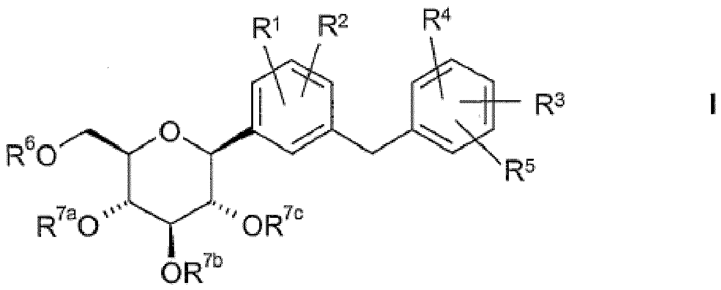 dans laquelle R¹ à R⁵ sont tels que définis dans une ou plusieurs des revendications 1 à 21 et
R⁶, R^{7a},
R^{7b}, R^{7c} ont indépendamment les uns des autres, une signification choisie dans le groupe (alkyle en C₁ à C₁₈)-carbonyle, (alkyle en C₁ à C₁₈)oxycarbonyle, arylcarbonyle et aryl-(alkyle en C₁ à C₃)-carbonyle, les L2 sont choisis indépendamment les uns des autres dans le groupe comprenant un atome de fluor, de chlore, de brome, d'iode, un groupe alkyle en C₁ à C₃, difluorométhyle, trifluoro-méthyle, alcoxy en C₁ à C₃, difluorométhoxy, trifluorométhoxy et cyano ; et
les groupes aryle mentionnés dans la définition des radicaux mentionnés précédemment désignent des groupes phényle ou naphtyle qui peuvent être mono-ou disubstitués indépendamment les uns des autres par des radicaux L2 identiques ou différents ; et sauf indication contraire, les groupes alkyle mentionnés précédemment peuvent être à chaîne linéaire ou ramifiée,
leurs tautomères, leurs stéréomères, leurs mélanges et leurs sels.

39. Dérivés de benzène substitués par un glucopyranosyle selon la revendication 38, **caractérisés par** la formule 1.2 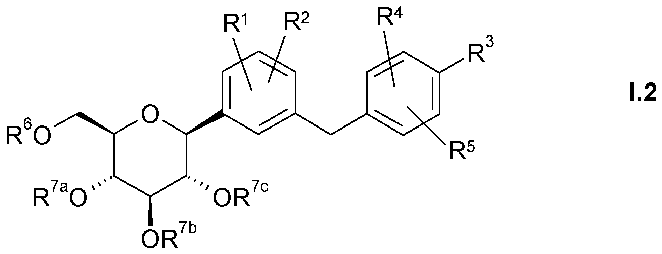 dans laquelle les radicaux R¹ à R⁵ et R⁶, R^{7a}, R^{7b} et R^{7c} sont tels que définis à la revendication 38.

40. Dérivés de benzène substitués par un glucopyranosyle selon la revendication 38, **caractérisés par** la formule I.2c 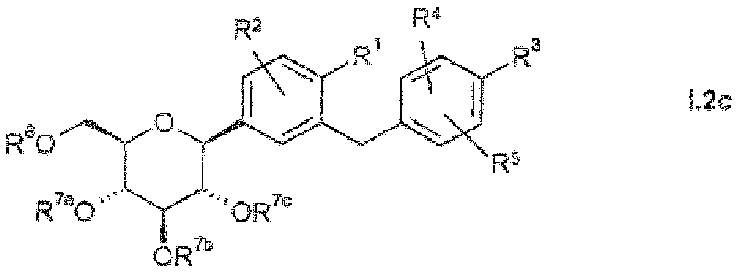 dans laquelle les radicaux R¹ à R⁵ et R⁶, R^{7a}, R^{7b}, R^{7c} sont tels que définis à la revendication 38.

41. Médicament qui présente un composé selon une ou plusieurs des revendications 1 à 21 ou sel physiologiquement acceptable de ce composé et au moins une substance active choisie dans le groupe des anti-diabétiques parallèlement à, éventuellement, un ou plusieurs véhicules et/ou diluants inertes.

42. Médicament qui présente un composé selon une ou plusieurs des revendications 1 à 21 ou sel physiologiquement acceptable de ce composé et au moins une substance active choisie dans le groupe des inhibiteurs de la protéine tyrosinephosphatase 1, des substances qui influent sur une production de glucose dérégulée dans le foie comme par exemple, des inhibiteurs de la glucose-6-phosphatase ou de la fructose-1,6-bisphosphatase, de la glycogène-phosphorylase, des antagonistes du récepteur du glucagon et des inhibiteurs de la phosphoénolpyruvatecarbo-xykinase, de la glycogène-synthase-kinase ou de la pyruvatedéshydrokinase, des hypolipidémiants comme par exemple les inhibiteurs de la HMG-CoA-réductase, des fibrates, l'acide nicotinique et leurs dérivés, les agonistes du PPAR-alpha, les agonistes du PPAR-delta, les inhibiteurs de l'ACAT ou des inhibiteurs de la résorption du cholestérol, des substances se liant à l'acide biliaire, des inhibiteurs du transport de l'acide biliaire iléal, des composés augmentant le HDL tels que par exemple, des inhibiteurs du CETP ou des régulateurs de l'ABC1 ou des substances actives pour le traitement de l'obésité telles que la sibutramine ou la tétra-hydrolipstatine, la dexfenfluramine, l'axokine, les antagonistes du récepteur du cannabinoïde 1, les antagonistes du récepteur de MCH-1, les agonistes du récepteur de MC4, les antagonistes de NPY5 ou NPY2 ou les β3-agonistes et les agonistes du récepteur de 5HT2c, parallèlement à, éventuellement, un ou plusieurs véhicules et/ou diluants inertes.

43. Médicament qui présente un composé selon une ou plusieurs des revendications 1 à 21, ou sel physiologiquement acceptable de ce composé et au moins une substance active choisie dans le groupe des médicaments destinés à influer sur l'hypertension artérielle, l'insuffisance cardiaque chronique ou l'athérosclérose comme par exemple des antagonistes A-II ou des inhibiteurs de l'ECA, des inhibiteurs de l'ECE, des diurétiques, des bêtabloquants, des anta-gonistes du Ca, des antihypertenseurs actifs au niveau central, des antagonistes du récepteur alpha-2-adrénergique, des inhibiteurs de l'endo-peptidase neutre et des inhibiteurs de l'agrégation plaquettaire ou des combinaisons de ceux-ci, parallèlement à, éventuellement un ou plusieurs véhicules et/ou diluants inertes.

44. Médicament selon la revendication 43, **caractérisé en ce que** la substance active est choisie dans le groupe des antagonistes du récepteur de l'angiotensine II, dans le groupe comprenant le candesartan cilexetil, le losartan potassium, le mésylate d'éprosartan, le valsartan, le telmisartan, l'irbesartan, l'EXP-3174, le L-158809, l'EXP-3312, l'olmesartan, le medoxomil, le tasosartan, le KT-3-671, le GA-0113, le RU-64276, l'EMD-90423, le BR-9701.

45. Médicament qui présente un composé selon une ou plusieurs des revendications 1 à 21, ou sel physiologiquement acceptable de ce composé et au moins une substance active choisie dans le groupe comprenant les inhibiteurs de la synthèse de l'acide urique et les uricosuriques parallèlement à, éventuellement un ou plusieurs véhicules et/ou diluants inertes.

46. Médicament qui présente un composé selon une ou plusieurs des revendications 1 à 21, ou sel physiologiquement acceptable de ce composé et au moins une substance active choisie dans le groupe comprenant des antagonistes du récepteur GABA, des inhibiteurs du canal sodique, le topi-ramate, des inhibiteurs de la protéine kinase C, des inhibiteurs des produits terminaux de la glycation avancée et des inhibiteurs de l'aldose réductase, parallèlement à, éventuellement un ou plusieurs véhicules et/ou diluants inertes.

47. Médicament selon l'une des revendications 41 à 46, **caractérisé en ce que** le composé selon l'invention ou son sel physiologiquement acceptable et l'autre substance active à combiner se présentent conjointement dans une forme galénique ou séparés en deux formes galéniques identiques ou différentes.
